# EUROPEAN PATENT APPLICATION

(11) **EP 4 710 845 A2**
(43) Date of publication of application: **18.03.2026**
(21) Application number: 25212078.7
(22) Date of filing: 02.02.2022
(51) Int. Cl.: A61B 5/00

(54) **METHODS FOR TREATING AUTISM SPECTRUM DISORDER**

(30) Priority: 03.02.2021 US 202163145250 P
(62) Divisional of application: 22750292.9
(71) Applicant: The General Hospital Corporation, Boston, MA 02114 (US)
(72) Inventor: JOSHI, Gagan, Weston, 02493 (US)
(74) Representative: Forresters IP LLP

(57) **Abstract**

Described herein are methods for predicting treatment response to glutamate modulating agents, selecting treatment comprising glutamate modulating agents, for, and treating subjects with glutamate modulating agents, in subjects with autism spectrum disorder (ASD).

## Description

### CLAIM OF PRIORITY

This application claims the benefit of U.S. Provisional Patent Application Serial No. 63/145,250, filed on February 3, 2021. The entire contents of the foregoing are hereby incorporated by reference.

### FEDERALLY SPONSORED RESEARCH OR DEVELOPMENT

This invention was made with Government support under Grant No. MH100450 awarded by the National Institutes of Health. The Government has certain rights in the invention.

### TECHNICAL FIELD

Described herein are methods for predicting treatment response to glutamate modulating agents, selecting treatment comprising glutamate modulating agents, for, and treating subjects with glutamate modulating agents, in subjects with autism spectrum disorder (ASD).

### BACKGROUND

Autism spectrum disorder (ASD) is a highly disabling neurodevelopmental disorder characterized by deficits in social communication and interaction along with restricted, repetitive behaviors that is estimated to affect up to 2% of youth in the general population (Blumberg et al., 2013).

Although there are psychopharmacological options available for the treatment of irritability and hyperactivity in ASD, to date, no medications have consistently been shown to reliably improve social impairments in ASDs (Hollander et al., 2005; Hollander et al., 2012; McCracken et al., 2002; McDougle et al., 1998; McDougle et al., 1996; "Risperidone treatment of autistic disorder: longer-term benefits and blinded discontinuation after 6 months," 2005). Earlier studies of fenfluramine, secretin, and naltrexone have largely been disappointing (Campbell, 1988; Campbell et al., 1993; Willemsen-Swinkels et al., 1996; Williams et al., 2012).

### SUMMARY

The present invention is based, at least in part, on the discovery that subjects with autism spectrum disorder (ASD) have differing levels of brain glutamate in specific brain regions, e.g., in the pregenual region of the anterior cingulate cortex (PgACC). As shown herein, non-invasive imaging methods can be used to determine these levels, and these levels can be used in turn to identify a subset of subjects with ASD for whom treatment with glutamate modulating agents can be efficacious. The methods can also be used predict whether a subject with ASD will respond to glutamate modulating agents; to select treatment comprising glutamate modulating agents for subjects with ASD; and to identify and treat subjects with ASD with glutamate modulating agents.

Thus provided herein are methods for predicting response to treatment with a glutamate modulating (lowering) agent in a subject with autism spectrum disorder (ASD). The methods include: (i) identifying a subject who has ASD; (ii) determining a level of glutamate in one or more selected brain regions of interest (ROIs) in the brain of the subject; (iii) comparing the level of glutamate in the ROI to a reference level; and (iv) on the basis of step (iii), identifying a subject who has a level of glutamate in the ROI above the reference level as likely to respond to treatment with a glutamate modulating agent.

Also provided herein are methods for selecting a treatment comprising administration of a glutamate modulating (lowering) agent for a subject with autism spectrum disorder (ASD). The methods include (i) identifying a subject who has ASD; (ii) determining a level of glutamate in one or more selected brain regions of interest (ROIs) in the brain of the subject; (iii) comparing the level of glutamate in the ROI to a reference level; and (iv) on the basis of step (iii), selecting a treatment with a glutamate modulating agent for a subject who has a level of glutamate in the ROI above the reference level.

Additionally provided herein are methods for treating a subject with autism spectrum disorder (ASD). The methods include (i) identifying a subject who has ASD; (ii) determining a level of glutamate in one or more selected brain regions of interest (ROIs) in the brain of the subject; (iii) comparing the level of glutamate in the ROI to a reference level; and (iv) on the basis of step (iii), administering a treatment comprising a therapeutically effective amount of a glutamate modulating agent to a subject who has a level of glutamate in the ROI above the reference level.

Further, provided herein are methods for treating a subject with autism spectrum disorder (ASD). The methods include: (i) providing a subject identified as having ASD characterized by increased glutamate activity in one or more selected brain regions of interest (ROIs) in the brain of the subject; and (ii) administering to the subject a therapeutically effective amount of a glutamate modulating agent.

Also provided herein are methods for monitoring response to treatment with a glutamate modulating (lowering) agent in a subject with autism spectrum disorder (ASD). The methods include (i) identifying a subject who has ASD; (ii) determining a baseline level of glutamate in one or more selected brain regions of interest (ROIs) in the brain of the subject; (iii) administering two or more doses of the glutamate modulating agent to the subject; (iv) determining a subsequent level of glutamate in the ROI; (v) comparing the baseline level of glutamate in the ROI to the subsequent level; and identifying a subject who has a subsequent level of glutamate in the ROI below the baseline level as responding to treatment with a glutamate modulating agent.

In some embodiments, at least one ROI comprises all or part of the pregenual anterior cingulate cortex (PgACC).

In some embodiments, determining a level of glutamate in one or more selected ROIs comprises using magnetic resonance imaging (MRI), positron emission tomography (PET), or magnetic resonance spectroscopy (MRS), preferably proton Magnetic Resonance Spectroscopy (¹H MRS).

In some embodiments, determining a level of glutamate in the ROI comprises acquiring and/or analyzing a proton spectra using ¹H MRS. In some embodiments, the proton spectra is acquired at 3 or 4 Tesla (3T or 4T) using a two-dimensional J-resolved (2D-JPRESS) ¹H MRS.

In some embodiments, determining a level of glutamate in the ROI comprises using a glutamate chemical exchange saturation transfer (GluCEST) imaging.

In some embodiments, the glutamate modulating agent is an NMDAR modulating (inhibiting) agent.

In some embodiments, the glutamate modulating agent is memantine, a nitro-aminoadamantane compound, lamotrigine, amantadine, D-cycloserine, N-Acetylcysteine, an amino-adamantane derivative, or a pharmaceutically acceptable salt thereof.

In some embodiments, the reference level represents a level in a cohort of subjects who have a level of glutamate in the ROI that is above the level of glutamate in the ROI of a cohort of healthy control subjects.

In some embodiments, the reference level represents a level of glutamate that is at least one standard deviation above the level of glutamate in the cohort of healthy subjects.

In some embodiments, the treatment comprises administration of the glutamate modulating agent at least once a day for at least a week, a month, three months, six months, or a year.

In some embodiments, the therapeutically effective amount is sufficient to result in an improvement in one or more impairments measured in the Social Responsiveness Scale-Second Edition (SRS-2) and/or the Clinical Global Impression (CGI) severity scale.

In some embodiments, the subject has high-functioning ASD (HF-ASD) and/or is intellectually capable.

In some embodiments, determining a level of glutamate in the ROI comprises acquiring and/or analyzing a proton spectra, e.g., using ¹H MRS. In some embodiments, the proton spectra is acquired at 3 or 4 Tesla (3T or 4T) using a two-dimensional J-resolved (2D-JPRESS) ¹H MRS.

In some embodiments, the subject is younger than 21 years old, 18 years old, or 15 years old.

In some embodiments, the glutamate modulating agent is memantine, or a pharmaceutically acceptable salt thereof.

In some embodiments, the glutamate modulating agent is a nitro-aminoadamantane compound, or a pharmaceutically acceptable salt thereof.

In some embodiments, the nitro-aminoadamantane compound is a compound of any one of formulas (I)-(V).

In some embodiments, the nitro-aminoadamantane compound is selected from: 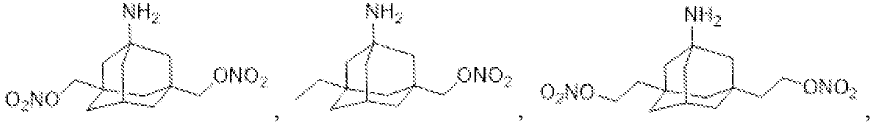 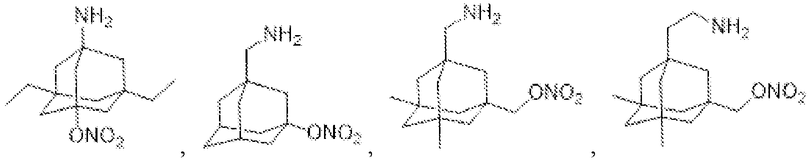 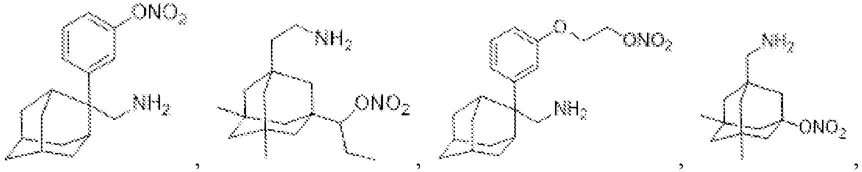 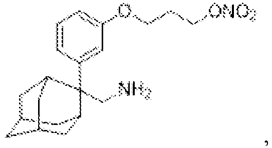 and pharmaceutically acceptable salts thereof.

Thus described herein is a method of predicting response to treatment with a glutamate modulating (lowering) agent in a subject with autism spectrum disorder (ASD), the method including: (i) identifying a subject who has ASD; (ii) determining a level of glutamate in one or more selected brain regions of interest (ROIs) in the brain of the subject; (iii) comparing the level of glutamate in the ROI to a reference level; and (iv) on the basis of step (iii), identifying a subject who has a level of glutamate in the ROI above the reference level as likely to respond to treatment with a glutamate modulating agent.

In a related aspect, described herein is a method of selecting a treatment including administration of a glutamate modulating (lowering) agent for a subject with autism spectrum disorder (ASD), the method including: (i) identifying a subject who has ASD; (ii) determining a level of glutamate in one or more selected brain regions of interest (ROIs) in the brain of the subject; (iii) comparing the level of glutamate in the ROI to a reference level; and (iv) on the basis of step (iii), selecting a treatment with a glutamate modulating agent for a subject who has a level of glutamate in the ROI above the reference level.

In another related aspect, described herein is a method of treating a subject with autism spectrum disorder (ASD), the method including: (i) identifying a subject who has ASD; (ii) determining a level of glutamate in one or more selected brain regions of interest (ROIs) in the brain of the subject; (iii) comparing the level of glutamate in the ROI to a reference level; and (iv) on the basis of step (iii), administering a treatment including a therapeutically effective amount of a glutamate modulating agent to a subject who has a level of glutamate in the ROI above the reference level.

In a related aspect, described herein is a method of treating a subject with autism spectrum disorder (ASD), the method including: (i) providing a subject identified as having ASD characterized by increased glutamate activity in one or more selected brain regions of interest (ROIs) in the brain of the subject; and (ii) administering to the subject a therapeutically effective amount of a glutamate modulating agent.

In an embodiment of any of the above methods, at least one ROI includes all or part of the pregenual anterior cingulate cortex (PgACC). In another embodiment of any of the above methods, determining a level of glutamate in one or more selected ROIs includes using magnetic resonance imaging (MRI), positron emission tomography (PET), or magnetic resonance spectroscopy (MRS), preferably proton Magnetic Resonance Spectroscopy (¹H MRS). For example, determining a level of glutamate in the ROI can include acquiring and/or analyzing a proton spectra using ¹H MRS. The proton spectra can be acquired at 3 or 4 Tesla (3T or 4T) using a two-dimensional J-resolved (2D-JPRESS) ¹H MRS. In another embodiment, determining a level of glutamate in the ROI can include using a glutamate chemical exchange saturation transfer (GluCEST) imaging.

In another embodiment of any of the above methods, the glutamate modulating agent is memantine, a nitro-aminoadamantane compound, lamotrigine, amantadine, D-cycloserine, or N-Acetylcysteine, or a pharmaceutically acceptable salt thereof.

In particular embodiments of any of the above methods, the reference level represents a level in a cohort of subjects who have a level of glutamate in the ROI that is above the level of glutamate in the ROI of a cohort of healthy control subjects. For example, the reference level can represent a level of glutamate that is at least one standard deviation above the level of glutamate in the cohort of healthy subjects.

In certain embodiments of any of the above methods, the treatment includes administration of the glutamate modulating agent at least once a day for at least a week, a month, three months, six months, or a year.

In one embodiment of any of the above methods, the subject has high-functioning ASD (HF-ASD) and/or is intellectually capable.

In a related aspect, described herein is a method of monitoring response to treatment with a glutamate modulating (lowering) agent in a subject with autism spectrum disorder (ASD), the method including: (i) identifying a subject who has ASD; (ii) determining a baseline level of glutamate in one or more selected regions of interest (ROIs) in the brain of the subject; (iii) administering two or more doses of the glutamate modulating agent to the subject; (iv) determining a subsequent level of glutamate in the ROI; (v) comparing the baseline level of glutamate in the ROI to the subsequent level; and identifying a subject who has a subsequent level of glutamate in the ROI below the baseline level as responding to treatment with a glutamate modulating agent. The at least one ROI can include all or part of the pregenual anterior cingulate cortex (PgACC). In certain embodiments, determining a level of glutamate in one or more selected ROIs includes using magnetic resonance imaging (MRI), positron emission tomography (PET), or magnetic resonance spectroscopy (MRS), preferably proton Magnetic Resonance Spectroscopy (¹H MRS). In another embodiment of any of the above methods, determining a level of glutamate in one or more selected ROIs includes using magnetic resonance imaging (MRI), positron emission tomography (PET), or magnetic resonance spectroscopy (MRS), preferably proton Magnetic Resonance Spectroscopy (¹H MRS). For example, determining a level of glutamate in the ROI can include acquiring and/or analyzing a proton spectra using ¹H MRS. The proton spectra can be acquired at 3 or 4 Tesla (3T or 4T) using a two-dimensional J-resolved (2D-JPRESS) ¹H MRS. In another embodiment, determining a level of glutamate in the ROI can include using a glutamate chemical exchange saturation transfer (GluCEST) imaging. In another embodiment, the glutamate modulating agent is memantine, a nitro-aminoadamantane compound, lamotrigine, amantadine, D-cycloserine, or N-Acetylcysteine, or a pharmaceutically acceptable salt thereof.

In another embodiment of any of the above methods, the subject is younger than 21 years old, 18 years old, or 15 years old. In other embodiments, the subject is 21 years or older.

In particular embodiments of any of the above methods, the glutamate modulating agent is memantine, or a pharmaceutically acceptable salt thereof.

In certain embodiments of any of the above methods, the glutamate modulating agent is a nitro-aminoadamantane compound, or a pharmaceutically acceptable salt thereof. For example, the nitro-aminoadamantane compound can be a compound of any one of formulas (I)-(V) (described herein). In some embodiments, the nitro-aminoadamantane compound is selected from: 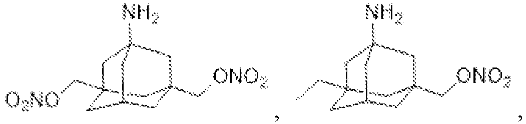 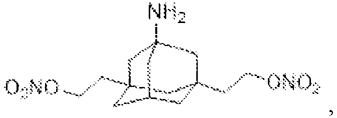 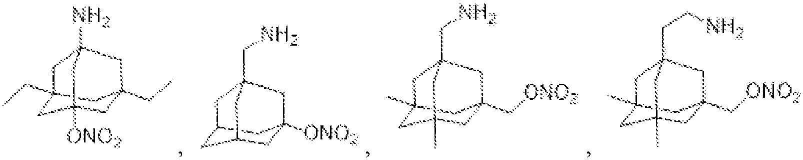 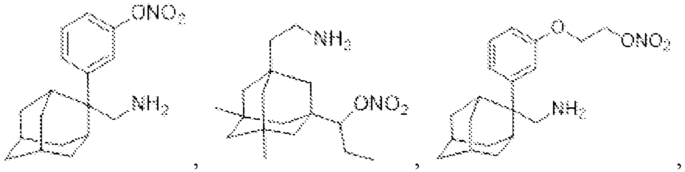 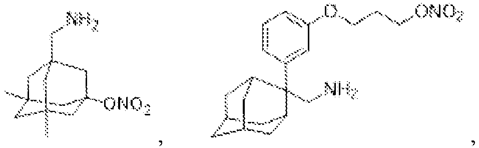 and
and pharmaceutically acceptable salts thereof.

As used herein, a "therapeutically effective amount" refers to an amount of a glutamate modulating agent required to ameliorate one or more symptoms of ASD. The effective amount of a glutamate modulating agent used to practice the present methods for the treatment of ASD can vary depending upon the glutamate modulating agent used, the manner of administration, the age, body weight, and general health of the subject. Ultimately, the attending physician will decide the appropriate amount and dosage regimen. Such amount is referred to as a "therapeutically effective amount."

By "pharmaceutical composition" is meant any composition that contains a glutamate modulating agent combined with a pharmaceutically acceptable carrier that together is suitable for administration to a subject. Pharmaceutical compositions useful in the present methods can take the form of tablets, gelcaps, capsules, pills, powders, granulates, suspensions, and/or emulsions.

As used herein, the term "pharmaceutically acceptable carrier" refers to an excipient or diluent in a pharmaceutical composition. For example, a pharmaceutically acceptable carrier may be a vehicle capable of suspending or dissolving the active ingredients (e.g., a glutamate modulating agent). The pharmaceutically acceptable carrier can be compatible with the other ingredients of the formulation and not deleterious to the recipient. For oral administration, a solid carrier may be preferred.

As used herein, the term "treat" or "treating" includes administration of a glutamate modulating agent to a subject by any route, e.g., orally. The subject, e.g., a patient, can be one having ASD. Treatment includes alleviating, relieving, altering, partially remedying, ameliorating, improving or affecting the one or more symptoms of ASD.

As used herein, the term "pharmaceutically acceptable salt" refers to salt forms (e.g., acid addition salts or metal salts) of the glutamate modulating agent suitable for therapeutic use according to the methods described herein.

As used herein, the term "nitro-aminoadamantane compound" refers to compounds including an adamantane moiety substituted by at least one amino group and at least one terminal nitrate group. The nitro-aminoadamantane compound used in the methods described herein can be any nitro-aminoadamantane compound of formulas (I)-(V), or subgenera thereof.

The terms "halogen", "halide" and "halo" refer to fluorine/fluoride, chlorine/chloride, bromine/bromide and iodine/iodide.

The term "alkyl" refers to a linear or branched, saturated monovalent hydrocarbon radical, wherein the alkyl group can optionally be substituted with one or more substituents as described herein. In certain embodiments, an alkyl group is a linear saturated monovalent hydrocarbon radical that has 1 to 10 (C₁₋₁₀) or 1 to 6 (C₁₋₆) carbon atoms, or is a branched saturated monovalent hydrocarbon radical that has 3 to 10 (C₃₋₁₀) or 3 to 6 (C₃₋₆) carbon atoms. As an example, the term "C₁₋₆ alkyl" refers to a linear saturated monovalent hydrocarbon radical of 1 to 6 carbon atoms or a branched saturated monovalent hydrocarbon radical of 3 to 6 carbon atoms. Linear C₁₋₆ and branched C₃₋₆ alkyl groups may also be referred to as "lower alkyl". Non-limiting examples of alkyl groups include methyl, ethyl, propyl (including n-propyl and isopropyl), butyl (including all isomeric forms, such as n-butyl, isobutyl, sec-butyl and tert-butyl), pentyl (including all isomeric forms, such as n-pentyl), and hexyl (including all isomeric forms, such as n-hexyl).

The terms "alkylene" and "-alkyl-" refer to a divalent alkyl group, which can optionally be substituted with one or more substituents as described herein.

The term "heteroalkyl" refers to a linear or branched, saturated monovalent hydrocarbon group containing one or more heteroatoms independently selected from O, N and S. In some embodiments, one or more heteroatoms are in the main chain of the linear or branched hydrocarbon group. The terms "heteroalkylene" and "-heteroalkyl-" refer to a divalent heteroalkyl group. A heteroalkyl group and a -heteroalkyl- group can optionally be substituted with one or more substituents as described herein. Examples of heteroalkyl and -heteroalkyl- groups include without limitation -(CH₂)ₘ-(O or S)-(CH₂)ₙCH₃ and -(CH₂)ₘ-(O or S)-(CH₂)ₚ-, wherein m is 1, 2 or 3, n is 0, 1 or 2, and p is 1, 2 or 3.

The term "alkoxy" refers to an -O-alkyl group, which can optionally be substituted with one or more substituents as described herein.

Examples of -O-heteroalkyl and -O-heteroalkyl- groups include without limitation ethylene glycol groups and polyethylene glycol (PEG) groups, including but not limited to -(OCH₂CH₂)ₙ-OR and -(OCH₂CH₂)ₙ-O-, wherein R is hydrogen or alkyl and n is 1, 2 or 3. It is understood that for a -O-heteroalkyl-ONO₂ group, when the -O-heteroalkyl- group is an ethylene glycol or PEG group, the terminal oxygen atom of the ethylene glycol or PEG group is part of the nitrate (-ONO₂) group. An -O-heteroalkyl group and an -O-heteroalkyl- group can optionally be substituted with one or more substituents as described herein.

The term "haloalkyl" refers to an alkyl group that is substituted with one or more halogen/halide atoms. A haloalkyl group can optionally be substituted with one or more additional substituents as described herein. Examples of haloalkyl groups include without limitation fluoroalkyl groups such as -CH₂F, -CHF₂ and -(CH₂)ₙCF₃, and perfluoroalkyl groups such as -CF₃ and -(CF₂)ₙCF₃, wherein n is 1, 2, 3, 4 or 5.

The term "-alkylaryl" refers to an alkyl group that is substituted with one or more aryl groups. An -alkylaryl group can optionally be substituted with one or more additional substituents as described herein.

The term "cycloalkyl" refers to a cyclic saturated, bridged or non-bridged monovalent hydrocarbon radical, which can optionally be substituted with one or more substituents as described herein. In certain embodiments, a cycloalkyl group has from 3 to 10 (C₃₋₁₀), or from 3 to 8 (C₃₋₈), or from 3 to 6 (C₃₋₆) carbon atoms. Non-limiting examples of cycloalkyl groups include cyclopropyl, cyclobutyl, cyclopentyl, cyclohexyl, cycloheptyl, cyclooctyl, norbornyl, decalinyl and adamantyl. The term "-cycloalkyl-" refers to a divalent cycloalkyl group, which can optionally be substituted with one or more substituents as described herein.

The terms "heterocyclyl" and "heterocyclic" refer to a monocyclic non-aromatic group or a multicyclic group that contains at least one non-aromatic ring, wherein at least one non-aromatic ring contains one or more heteroatoms independently selected from O, N and S. The non-aromatic ring containing one or more heteroatoms may be attached or fused to one or more saturated, partially unsaturated or aromatic rings. In certain embodiments, a heterocyclyl or heterocyclic group has from 3 to 10, or 3 to 8, or 3 to 6 ring atoms. In some embodiments, a heterocyclyl or heterocyclic group is a monocyclic, bicyclic or tricyclic ring system, which may include a fused or bridged ring system, and in which nitrogen or sulfur atoms can optionally be oxidized, nitrogen atoms can optionally be quaternized, and one or more rings may be fully or partially saturated, or aromatic. A heterocyclyl or heterocyclic group may be attached to the main structure at any heteroatom or carbon atom which results in the creation of a stable compound. Examples of heterocyclyl or heterocyclic groups include without limitation azepinyl, azetidinyl, aziridinyl, benzodioxanyl, benzodioxolyl, benzofuranonyl, benzopyranonyl, benzopyranyl, benzotetrahydrofuranyl, benzotetrahydrothienyl, benzothiopyranyl, β-carbolinyl, chromanyl, decahydroisoquinolinyl, dihydrobenzisothiazinyl, dihydrobenzisoxazinyl, dihydrofuryl, dihydropyranyl, dihydropyrazinyl, dihydropyridinyl, dihydropyrazolyl, dihydropyrimidinyl, dihydropyrrolyl, dioxolanyl, dithianyl, furanonyl, imidazolidinyl, imidazolinyl, indolinyl, indolizinyl, isobenzotetrahydrofuranyl, isobenzotetrahydrothienyl, isochromanyl, isoindolinyl, isothiazolidinyl, isoxazolidinyl, morpholinyl, octahydroindolyl, octahydroisoindolyl, oxazolidinonyl, oxazolidinyl, oxiranyl, piperazinyl, piperidinyl, 4-piperidonyl, pyrrolidinyl, pyrrolinyl, quinuclidinyl, tetrahydrofuryl, tetrahydrofuranyl (oxolanyl), tetrahydroisoquinolinyl, tetrahydropyranyl, tetrahydrothienyl (tetrahydrothiophenyl, thiolanyl), thiamorpholinyl (thiomorpholinyl), thiazolidinyl and 1,3,5-trithianyl. The term "-heterocyclyl-" refers to a divalent heterocyclyl group. A heterocyclyl or heterocyclic group, and a - heterocyclyl- group, can optionally be substituted with one or more substituents as described herein.

The term "aryl" refers to a monocyclic aromatic hydrocarbon group or a multicyclic group that contains at least one aromatic hydrocarbon ring. In certain embodiments, an aryl group has from 6 to 10 ring atoms. Non-limiting examples of aryl groups include phenyl, naphthyl, fluorenyl, azulenyl, anthryl, phenanthryl, biphenyl and terphenyl. The aromatic hydrocarbon ring of an aryl group may be attached or fused to one or more saturated, partially unsaturated or aromatic rings - e.g., dihydronaphthyl, indenyl, indanyl and tetrahydronaphthyl (tetralinyl). The term "-aryl-" refers to a divalent aryl group. An aryl group and an -aryl- group can optionally be substituted with one or more substituents as described herein.

The term "heteroaryl" refers to a monocyclic aromatic group or a multicyclic group that contains at least one aromatic ring, wherein at least one aromatic ring contains one or more heteroatoms independently selected from O, N and S. The heteroaromatic ring may be attached or fused to one or more saturated, partially unsaturated or aromatic rings that may contain only carbon atoms or that may contain one or more heteroatoms. A heteroaryl group may be attached to the main structure at any heteroatom or carbon atom which results in the creation of a stable compound. In certain embodiments, a heteroaryl group has from 5 to 10 ring atoms. Examples of monocyclic heteroaryl groups include without limitation pyrrolyl, pyrazolyl, pyrazolinyl, imidazolyl, oxazolyl, isoxazolyl, thiazolyl, thiadiazolyl, isothiazolyl, furanyl, thienyl (thiophenyl), oxadiazolyl, triazolyl, tetrazolyl, pyridyl, pyridonyl, pyrazinyl, pyrimidinyl, pyridazinyl, pyridazinonyl and triazinyl. Non-limiting examples of bicyclic heteroaryl groups include indolyl, benzothiazolyl, benzothiadiazolyl, benzoxazolyl, benzisoxazolyl, benzothienyl (benzothiophenyl), quinolinyl, tetrahydroisoquinolinyl, isoquinolinyl, benzimidazolyl, benzotriazolyl, indolizinyl, benzofuranyl, isobenzofuranyl, chromonyl, coumarinyl, cinnolinyl, quinazolinyl, quinoxalinyl, indazolyl, naphthyridinyl, phthalazinyl, quinazolinyl, purinyl, pyrrolopyridinyl, furopyridinyl, thienopyridinyl, dihydroisoindolyl and tetrahydroquinolinyl. Examples of tricyclic heteroaryl groups include without limitation carbazolyl, benzindolyl, dibenzofuranyl, phenanthrollinyl, acridinyl, phenanthridinyl, xanthenyl and phenothiazinyl. The term "-heteroaryl-" refers to a divalent heteroaryl group. A heteroaryl group and a -heteroaryl- group can optionally be substituted with one or more substituents as described herein.

Each group described herein (including without limitation monovalent and divalent alkyl, heteroalkyl, -O-alkyl, -O-heteroalkyl, alkylaryl, cycloalkyl, heterocyclyl, aryl and heteroaryl), whether as a primary group or as a substituent group, can optionally be substituted with one or more substituents. In certain embodiments, each group described herein can optionally be substituted with 1, 2, 3, 4, 5 or 6 substituents independently selected from halide, cyano, nitro, nitrate, hydroxyl, sulfhydryl (-SH), - NH₂, -OR¹¹, -SR¹¹, -NR¹²R¹³, -C(=O)R¹¹, -C(=O)OR¹¹, -OC(=O)R¹¹, -C(=O)NR¹²R¹³, - NR¹²C(=O)R¹¹, -OC(=O)OR¹¹, -OC(=O)NR¹²R¹³, -NR¹²C(=O)OR¹¹, - NR¹¹C(=O)NR¹²R¹³, alkyl, haloalkyl, alkoxy, cycloalkyl, heterocyclyl, aryl and heteroaryl, wherein:
R¹¹ in each occurrence independently is hydrogen, alkyl, cycloalkyl, heterocyclyl, aryl or heteroaryl; and
R¹² and R¹³ in each occurrence independently are hydrogen, alkyl, cycloalkyl, heterocyclyl, aryl or heteroaryl, or R¹² and R¹³ and the nitrogen atom to which they are connected form a heterocyclic or heteroaryl ring.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

FIG. 1 is an exemplary RCT CONSORT Flow Diagram. Trial Profile, including eligibility, randomization, and participation follow-up through the study of memantine for autism related behaviors in youth is shown.
FIGs. 2A-D. Treatment Response Based on Baseline PgACC glutamate Activity. A. Baseline *Pg*ACC glutamate activity in ASD. B. Treatment response based on baseline *Pg*ACC glutamate activity. C. 3-way interaction between treatment arm, wave, and baseline glutamate activity. D. AUC response to treatment comparisons between memantine and placebo based on baseline glutamate activity.
FIG. 3. Schematic showing exemplary voxel placement in preguenal anterior cingulate cortex (ACC).

### DETAILED DESCRIPTION

Described herein are methods for identifying a subpopulation of subjects with ASD for whom treatment with glutamate modulating agents can be efficacious. The methods described herein include treatment methods wherein ASD subjects are first screened before commencing therapy with a glutamate modulating agent to improve patient outcomes.

Emerging evidence on the role of glutamatergic agents for the treatment of social deficits in ASDs is encouraging. Dysregulation in glutamatergic activity has been hypothesized to be associated with the pathophysiology of ASD (Carlsson, 1998; McDougle, 2002). Glutamate is the primary excitatory amino acid neurotransmitter in the brain and, through its activity on N-methyl-D-aspartate (NMDA) receptors, is involved in neuronal development and synaptic plasticity (Cotman & Anderson, 1995). Over-activation of glutamate is associated with excitotoxicity and apoptosis. Serological, postmortem brain, genetic, and proton magnetic resonance spectroscopy (¹HMRS) research suggests increased glutamate activity in ASD (Blue et al., 1999; Fatemi et al., 2002; Jamain et al., 2002; Lappalainen & Riikonen, 1996; Purcell & Sham, 2002; Ramoz et al., 2004; Serajee et al., 2003; Shimmura et al., 2011; Shuang et al., 2004). A recent ¹HMRS study from our lab documented significantly increased brain glutamate activity in the pregenual region of the anterior cingulate cortex (PgACC) with no such abnormality in the bilateral MTL regions in adolescent males with high-functioning ASD (HF-ASD) (Joshi et al., 2012). ACC is critical for social and emotional processing and thus, a region of interest in autism. Converging evidence from various investigative modalities suggests abnormalities in the ACC region in individuals with ASDs including histopathological changes of increased cell packing density and decreased cell size, smaller in volume, decrease in regional cerebral blood flow, and metabolically less active (Haznedar, Buchsbaum, Metyger, et al., 1997) with abnormal functional activity (theory of mind task related and resting state) (Bauman & Kemper, 1985; Di Martino et al., 2008; Di Martino et al., 2009; Haznedar, Buchsbaum, Metzger, et al., 1997; Kemper & Bauman, 1998; Ohnishi et al., 2000; Silk et al., 2006; Simms et al., 2009).

The glutamate modulating agents memantine, lamotrigine, amantadine, D-cycloserine, and N-Acetylcysteine have been studied as potential treatments for the core symptoms of ASD. However, the empirical evidence to date for their efficacy from the limited number of controlled trials conducted in predominantly intellectually disabled populations of youth with ASD is modest at best (Belsito et al., 2001; D'Souza et al., 1995; Posey et al., 2004).

Lamotrigine is an antiepileptic drug of the phenyltriazine class that stabilizes neural membranes by interfering with sodium channels, decreasing glutamate release. While lamotrigine has been shown to be an effective mood stabilizer for bipolar disorders, it does not appear to have an impact on ASD symptoms. In an RCT, investigators found no significant differences in improvements between lamotrigine or placebo groups on the Autism Behavior Checklist, the Aberrant Behavior Checklist, the Vineland Adaptive Behavior scales, the PL-ADOS, or the CARS (Belsito et al., 2001)

Like memantine, amantadine is a noncompetitive NMDA antagonist. Only one RCT has been conducted on the effects on amantadine on ASD. The study showed an improvement in ASD symptoms in 5-19 year-olds following four weeks on amantadine hydrochloride vs placebo. This improvement was only reflected in the clinical rated scale CGI, but not in the parent-rated behavior scale ABC-CV (King et al., 2001).

D-Cycloserine is a partial glycine agonist at the N-methyl-D-aspartate (NMDA) receptor. It has been shown to improved stereotypic symptoms in older adolescents and young adults with ASDs measured by the Aberrant Behavior Checklist subscale 3 (Urbano et al., 2014). The same group of investigators also demonstrated the application of D-cycloserine in statistically and clinically significant improvement on the Social Responsiveness Scale and the Aberrant Behavior Checklist (Urbano et al., 2015).

NAC is an antioxidant that is commonly used for restoring hepatic concentrations of cysteine following liver damage. The cysteine group is also oxidized to cystine, which interacts with glutamate-cystine antiporters to theoretically reduce glutamatergic neurotransmission. One RCT of NAC shows significant improvement in the ABC irritability subscale score in ASD children compared to the placebo group (Hardan et al., 2012). However, a separate study suggests that NAC has no significant impact on social impairment in youth with ASD (Wink et al., 2016).

Memantine is a moderate-affinity, non-competitive, NMDA receptor antagonist that is approved by the U.S. Food and Drug Administration (FDA) for the treatment of moderate to severe Alzheimer's disease (U.S. FDA, 2003). Preliminary evidence from limited trials of memantine in youth unequivocally suggest acceptable safety and tolerability profile (Aman et al., 2017; Findling et al., 2007). Available data from retrospective and prospective treatment studies of memantine in individuals with ASDs reported significant improvement in a range of behavioral impairments including attention, hyperactivity, language, eye contact, social interaction and withdrawal, and repetitive behaviors (Aman et al., 2017; Findling et al., 2007). The only controlled trial of memantine in children with and without intellectual disability who received 12 week of memantine at a substantially lower dose by study design (mean dose: 5 mg/day) reported no improvement in ASD behaviors relative to the observed placebo response (Aman et al., 2017). Preliminary results from an uncontrolled trial of memantine reported significant improvement in social behaviors upon 12-week treatment with 20 mg/day of memantine in adults with HF-ASD (Joshi et al., 2016).

One common limitation of the above-mentioned trials of glutamate modulating agents in ASD is that many were conducted in intellectually compromised populations that do not allow for the study of treatment response of higher order social deficits observed in intellectually capable populations with ASD.

None of the studies described above utilize a biomarker characteristic of a subpopulation of ASD subjects responsive to therapy with glutamate modulating agents. The present invention arises out of the discovery of such a biomarker, and provides a strategy for the treatment of the subpopulation as part of a precision medicine therapeutic intervention. The present study evaluated short-term behavioral and neural effects of memantine therapy in ASD. Clinical and neural response to short-term memantine therapy was examined in youth with ASD by conducting a 12-week randomized controlled trial (RCT). The study demonstrated clinical efficacy and tolerability of glutamate modulating agents for the treatment of social impairment in youth with ASD who have elevated levels of glutamate in the ACC.

### Subjects

The methods described herein can be used to identify, select, and treat subjects who have ASD, e.g., who have been diagnosed with ASD. These subjects can include those who meet DSM-IV-TR/5 diagnostic criteria for autism, as established by clinical diagnostic evaluation. In some embodiments, the subjects have at least moderate severity of ASD, as determined by a score on the informant-rated Social Responsiveness Scale-Second Edition (SRS-2; Constantino et al, 2003) and/or a score on the clinician-rated National Institute of Mental Health (NIMH) Clinical Global Impression (CGI) severity scale (CGI-S; NIMH, 1985). In some embodiments, the subjects do not have a formal diagnosis of ASD. In some embodiments, the subjects are considered high-functioning, or are without intellectual disability (IQ > 70).

### Social Responsiveness Scale

The Social Responsiveness Scale - Second Edition (SRS-2) School-Age Form assesses severity of ASD symptoms in children ages 4-18 years. This affordable, paper- and-pencil questionnaire is completed by the child's caregiver and contains a total of 65 items. The rating scale measures the severity of ASD symptoms including elements of reciprocal social behaviors (39 items), social use of language (6 items), and behaviors characteristic of children with ASD (20 items). Each item on the scale is rated on a Likert scale from "0" (never true) to "3" (almost always true). There are five treatment subscales that can be computed: social awareness, social cognition, social communication, social motivation, and autistic mannerisms, which can provide additional information for the clinician. The psychometric properties of the SRS have been well established as a valid measure to assess for autistic traits and can distinguish autism from other neuropsychiatric conditions (Constantino et al., 2003; Constantino & Todd, 2003). The assessment takes approximately 15-20 minutes to complete.

### Social Responsiveness Scale (SRS 2) Scoring Algorithm

| | **Total Raw Score** | | | **T- Score** |
|---|---|---|---|---|
| | **School- Age*** | | **Adult**** | |
| | **Male** | **Female** | | |
| Severity Levels | | | | |
| Normal | ≤ 57 | ≤ 51 | ≤ 67 | ≤59 |
| Mild | 58 - 72 | 52 - 65 | 68 - 84 | 60 - 65 |
| Moderate | 73 - 97 | 66 - 89 | 85 - 112 | 66 - 75 |
| Severe | ≥98 | ≥90 | ≥113 | ≥76 |
| Screening Threshold | | | | |
| Non-referred | ≥70 | ≥70 | ≥70 | |
| Referred | ≥85 | ≥85 | ≥85 | |

| | | | | |
|---|---|---|---|---|
| *Informant-rated; **Self & Informant-rated | | | | |

### Social Responsiveness Scale - Subscale Items

| **SOCIAL AWARENESS** | **SOCIAL COGNITION** | **SOCIAL MOTIVATION** | **SOCIAL COMMUNICATION** | **AUTISTIC MANNERISM** |
|---|---|---|---|---|
| Expressions on his or her face don't match what he or she is saying | Doesn't recognize when others are trying to take advantage of him or her | Seems self-confident when interacting with others | Is awkward in turn-taking interactions with others | Rigid or inflexible patterns of odd behavior under stress |
| Walks in between people who are talking | Recognizes when something is unfair | Has good self-confidence | Is able to communicate feelings to others | Difficulty with changes in routine |
| Focuses his or her attention to where others are looking and listening | Is able to understand the meaning of other people's tone of voice & facial expression | Would rather be alone than with others | Has trouble keeping up with the flow of a normal conversation | Can't get mind off thinking about something |
| Doesn't mind being out of step with or "not on the same wavelength" as others | Seams overly sensitive to sounds, textures, or smells | Does not join group activities unless told to do so | Is socially awkward, even when trying to be polite | Has repetitive, odd behaviors such as hand flapping or rocking |
| Knows when he or she is talking too loud or making too much noise | Becomes upset in a situation with lots of things going on | Avoids staring social interactions with peers or adults | Gets frustrated trying to get ideas across in conversations | Unusual sensory interests or strange ways of playing with toys |
| Seems to reach to people as if they are objects | Concentrates too much on parts of things rather than seeing the whole picture | Seems much more fidgety in social situations than when alone | Wanders aimlessly from one activity to another | Thinks or talks about the same thing over and over |
| Is aware of what others are thinking or feeling | Doesn't understand how events relate to one another (cause and effect) | Clings to adults, seems too dependent on them | Has difficulty making friends, even when trying his or her best | Does extremely well at a few tasks, but does not do as well at most other tasks |
| Has good personal hygiene | Has a sense of humor, understands jokes | Stares or gazes off into space | Has difficulty relating to adults | Has an unusually narrow range of interests |
| | Is overly suspicious | Is too tense in social settings | Plays appropriately with peers | Is not well coordinated |
| | Is imaginative, good at pretending | Separates easily from caregivers | is able to imitate others' actions | Touches others in an unusual way |
| | Takes things too literally and doesn't get the real meaning of a conversation | Avoids people who want to be emotionally close to him or her | Is inflexible, has a hard time changing his or her mind | Behaves in ways that seem strange or bizarre |
| | Gives unusual or illogical reasons for doing things | | Knows when he or she is too close to **someone or is invading someone's space** | Is regarded by other children as odd or weird |
| | | | Talks to people with an unusual tone of voice | |
| | | | Avoids eye contact or has unusual eye **contact** | |
| | | | **Has overly serious facial expressions** | |
| | | | Is too silly or laughs inappropriately | |
| | | | Responds appropriately to mood changes in others | |
| | | | Is emotionally distant, doesn't show feelings | |
| | | | Has difficulty relating to peers | |
| | | | Gets teased a lot | |
| | | | Offers comfort to others when they are sad | |
| | | | Has difficulty answering questions directly and ends up talking around the subject | |

### Aberrant Behavior Checklist - Subscale Items

| **IRRITABILITY** | **HYPERACTIVITY** | **SOCIAL WITHDRAWAL** | **INAPPROPRIATE SPEECH** | **STEREOTYPY** |
|---|---|---|---|---|
| Injures self on purpose | Excessively active at home, school, work or elsewhere | Seeks isolation from others | Talks excessively | Stereotyped behavior; abnormal, repetitive movements |
| Deliberately hurts himself/herself | Tends to be excessively active | Prefers to be alone | Repetitive speech | Meaningless, recurring body movements |
| Does physical violence to self | Constantly runs or jumps around the room | Withdrawn; prefers solitary activities | Repeats a word or phrase over and over | Moves or rolls head back and forth repetitively |
| Aggressive to other children or adults (verbally or physically) | Restless, unable to sit still | Isolates from other children or adults | Talks to self loudly | Repetitive hand, body, or head movements |
| Cries and screams inappropriately | Does not stay in seat (during lesson, meals, etc.) | Listless, sluggish, inactive | | Waves or shakes the extremities repeatedly |
| Screams inappropriately | Boisterous (inappropriately noisy and rough) | Sits or stands in one position for a long times | | Odd, bizarre in behavior |
| Yells at inappropriate times | Impulsive (acts without thinking) | Does nothing but sit and watch others | | |
| Irritable and whiny | Disobedient; difficult to control | Preoccupied; stares into space | | |
| Depressed mood | Uncooperative | Unresponsive to structured activities (does not react) | | |
| Mood changes quickly | Disrupts group activities | Is difficult to reach, contact, or get through to | | |
| Stamps feet or bangs object or slams doors | Disturbs others | Fixed facial expression; lack of emotional responsiveness | | |
| Temper tantrums/outbursts | Easily distracted | Does not try to communicate by words or gestures | | |
| Has temper outbursts or tantrums when does not get own way | Does not pay attention to instructions | Resists any form of physical contact | | |
| Demands must be met immediately | Pays no attention when spoken to | Responds negatively to affection | | |
| | Deliberately ignores directions | Shows few social reactions to others | | |

### Clinical Global Impression

The Clinical Global Impression is a clinician-rated scale measuring overall mental health with scores ranging from 1 to 7. It includes subscales for global severity (CGI-S: 1 indicates not at all ill; 2, borderline mentally ill; 3, mildly ill; 4, moderately ill; 5, markedly ill; 6, severely ill; and 7, extremely ill) and global improvement (CGI-I: 1 indicates very much improved; 2, much improved; 3, minimally improved; 4, no change; 5, minimally worse; 6, much worse; and 7, very much worse). The CGI-I measures illness improvement as well as efficacy of treatment, with the effectiveness index indicating the extent to which therapeutic effects in conjunction with the level of adverse events (AEs) are experienced.

In some embodiments, the subjects have no, or have no recent (e.g., within 7, 14, 21, 28, 45, or 60 days or longer) history of treatment with memantine or other glutamate-modulating agents, such as lamotrigine, amantadine, and D-cycloserine.

In some embodiments, the subjects are at least 6, 7, or 8, years old, up to 12, 13, 14, 15, 16, 17, or 18 years old. In some embodiments, the subjects are between 4, 5, 6, 7, 8 and up to 12, 13, 14, 15, 16, 17, 18, 19, 20, or 21 years of age. Ranges with any of these specific ages as endpoints are also provided.

### Subject Selection

The present methods include identifying and selecting subjects who have increased levels of glutamate in selected regions of interest (ROIs) in the brain, including the pregenual anterior cingulate cortex (PgACC). The methods can include obtaining and/or analyzing glutamate activity in the ROI, e.g., in the *Pg*ACC, using magnetic resonance imaging (MRI), positron emission tomography (PET), and magnetic resonance spectroscopy (MRS), e.g., proton Magnetic Resonance Spectroscopy (¹H MRS). In some embodiments, the methods are based on acquisition and/or analysis of proton spectra, e.g., acquired at 3 or 4 Tesla (3T or 4T), e.g., using a two-dimensional J-resolved (2D-JPRESS) ¹H MRS protocol. See, e.g., Öngür et al., Biol Psychiatry 2008;64:718-726; Henry et al., Journal of Magnetic Resonance 208 (2011) 210-218; Jensen et al., NMR Biomed. 2009 Aug;22(7):762-9; Prescot et al., J Magn Reson Imaging. 2013 Mar;37(3):642-5; Dhamala et al., NMR in Biomedicine. 2019;32:e4058; Ding et al., Magnetic Resonance in Medicine 73:921-928 (2015); Gottschalk et al., NMR Biomed. 2016; 29: 943-951; Wiebenga et al., NMR Biomed. 2014; 27: 304-311; and Bogner et al., NMR Biomed. 2012; 25: 873-882; Wozniak et al., Isr J Psychiatry Relat Sci. 2012;49(1):62-9; Joshi et al., Eur Arch Psychiatry Clin Neurosci. 2013 Aug; 263(5):379-84. Alternatively, a glutamate chemical exchange saturation transfer (CEST) imaging technique (GluCEST) can be used (Mao et al., Quant Imaging Med Surg. 2019 Oct; 9(10): 1652-1663). In some embodiments, glutamate concentrations in combination with glutamine and gamma-aminobutyric acid (together designated as Glx) are measured. In some embodiments, only glutamate concentrations are measured, and/or either or both of glutamine and/or gamma-aminobutyric acid are not measured.

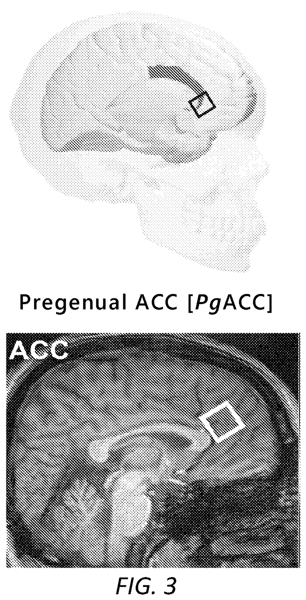

In some embodiments, the methods can include imaging a region of interest (ROI), e.g., an ROI comprising aan 8 cc (2 cm x 2 cm x 2 cm) single voxel, placed in the PgACC along the midline such that the inferior edge of the voxel is parallel to the descending surface of the corpus callosum, as determined by longitudinal relaxation time (T1) images used also for tissue segmentation, e.g., as shown in Fig. 3. Other voxel sizes can be used, e.g., 1mm x 1mm x 1mm (van Elst et al., 2014); 20mm x 20mm x 20mm (Cochran et al., 2015); 3cm x 3cm x 3cm (Ito et al., 2017); or 2.5cm x 4cm x 2cm (Montag et al., 2008), or variations thereof.

These methods can be used to determine a level of glutamate in the ROI in the subject. This level can then be compared to a reference level.

Suitable reference values can be determined using methods known in the art, e.g., using standard clinical trial methodology and statistical analysis. The reference values can have any relevant form. In some cases, the reference comprises a predetermined value for a meaningful level of glutamate, e.g., a control reference level that represents a normal or near-normal level of glutamate, e.g., a level in a control subject who has ASD, but who would not be likely to respond to treatment with a glutamate modulating agent, and/or a responder reference that represents an elevated level of glutamate, e.g., a level in a subject who is likely to respond to treatment with a glutamate modulating agent.

The predetermined level can be a single cut-off (threshold) value, such as a median or mean, or a level that defines the boundaries of an upper or lower quartile, tertile, or other segment of a clinical trial population that is determined to be statistically different from the other segments. It can be a range of cut-off (or threshold) values, such as a confidence interval. It can be established based upon comparative groups, such as where association with likelihood of response in one defined group is a fold higher, or lower, (e.g., approximately 2-fold, 4-fold, 8-fold, 16-fold or more) than the likelihood of response in another defined group. It can be a range, for example, where a population of subjects (e.g., control and ASD subjects) is divided equally (or unequally) into groups, such as a low glutamate group, a medium glutamate group, and a high glutamate group, wherein those in the high glutamate group are most likely to respond to treatment with a glutamate modulating agent; or into quartiles, the lowest quartile being subjects with the lowest glutamate and the highest quartile being subjects with the highest glutamate, or into n-quantiles (i.e., n regularly spaced intervals) the lowest of the n-quantiles being subjects with the lowest glutamate and the highest of the n-quantiles being subjects with the highest glutamate, again, with those in the high glutamate group most likely to respond to treatment with a glutamate modulating agent. In some embodiments, the threshold is one standard deviation from the level of glutamate in a healthy control.

Subjects (or cohorts of subjects) associated with predetermined values are typically referred to as reference subjects. For example, in some embodiments, a control or reference subject does not have a disorder described herein (e.g., ASD). In some embodiments the control or reference subject does have ASD, but is not likely to respond, or is likely to respond to treatment with a glutamate modulating agent. In some cases it may be desirable that the control subject (or cohort of subjects) is age- (e.g., range of ages) or sex-matched.

Thus, in some cases the level of glutamate in a subject being less than or equal to a reference level of glutamate is indicative of a low likelihood of response to treatment with a glutamate modulating agent. In other cases the level of glutamate in a subject being greater than or equal to the reference level of glutamate is indicative of a high likelihood of respond to treatment with a glutamate modulating agent. In some embodiments, the amount by which the level of glutamate in the subject is the less or more than the reference level is sufficient to distinguish a subject from a control subject, and optionally is a statistically significantly less or more than the level in a control subject. In cases where the level of glutamate in a subject being equal to the reference level of glutamate, the "being equal" refers to being approximately equal (e.g., not statistically different, or within one standard deviation).

The predetermined value can depend upon the particular population of subjects (e.g., human subjects) selected. For example, an apparently healthy population will have a different 'normal' range of levels of glutamate than will a population of subjects who have ASD. Accordingly, the predetermined values selected may take into account the category (e.g., sex, age, health, risk, presence of other diseases) in which a subject (e.g., human subject) falls. Appropriate ranges and categories can be selected with no more than routine experimentation by those of ordinary skill in the art.

In some embodiments, the level of glutamate is determined to be comparable to or above a threshold, or reference level in a cohort of subjects who respond to treatment with a glutamate modulating (lowering) agent, then the subject is identified as in the high glutamate group, as likely to respond to treatment with a glutamate modulating agent, and/or selected for and optionally treated with a glutamate modulating agent.

### Glutamate Modulating Agents

Glutamate modulating agents that can be used in the methods described herein include, without limitation, memantine, a nitro-aminoadamantane compound, lamotrigine, amantadine, D-cycloserine, N-Acetylcysteine, other amino-adamantane derivatives, or a pharmaceutically acceptable salt thereof.

The nitro-aminoadamantane compound used in the methods described herein can be a compound of any of formulas (I)-(V): 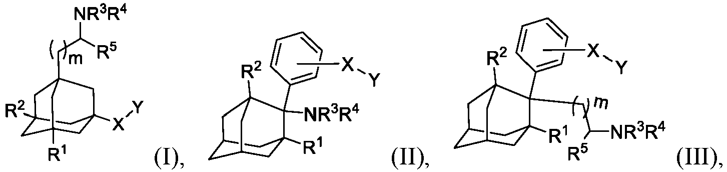 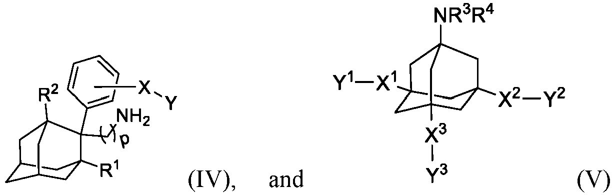 wherein in formulas (I)-(IV), Y is a nitrate-containing group and R1, R2, R3, R4, R5, X, p, and m are as defined elsewhere herein. In formula (V) each of Y¹, Y², and Y³, is optionally a nitrate-containing group and Y¹, Y², Y³, X¹, X², X³, R³, and R⁴ m are as defined elsewhere herein

### Nitro-aminoadamantane compounds

The nitro-aminoadamantane compounds used in the methods described herein can be synthesized using methods analogous to those described in U.S. Patent No. 7,326.730 and PCT Publication No. WO2019104020, each of which is incorporated herein in its entirety.

The nitro-aminoadamantane compound used in the methods described herein can be a compound of formula (I): 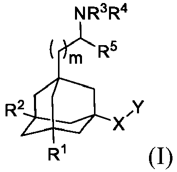 or a pharmaceutically acceptable salt thereof, wherein:
R¹ and R² independently are hydrogen, halide, linear or branched alkyl, linear or branched heteroalkyl, linear or branched alkoxy, linear or branched -O-heteroalkyl, cycloalkyl, heterocyclyl, aryl or heteroaryl, each of which can optionally be substituted;
R³ and R⁴ independently are hydrogen or linear or branched C₁-C₆ alkyl, or R³, R⁴ and the nitrogen atom to which they are attached form a 3-8-membered heterocyclic ring;
R⁵ is hydrogen or linear or branched C₁-C₆ alkyl;
X is bond, linear or branched -alkyl-, linear or branched -heteroalkyl-, linear or branched -O-alkyl-, linear or branched -O-heteroalkyl-, -(CH₂)ⱼ-cycloalkyl-(CH₂)ₖ-, - (CH₂)ⱼ-heterocyclyl-(CH₂)ₖ-, -(CH₂)ⱼ-aryl-(O)ₕ-(CH₂)ₖ- or -(CH₂)ⱼ-heteroaryl-(O)ₕ-(CH₂)ₖ-, each of which can optionally be substituted;
Y is -ONO₂ or 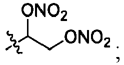
m is 0, 1, 2, 3, 4 or 5; j is 0, 1, 2 or 3; k is 0, 1, 2 or 3; and h is 0 or 1.

In certain embodiments, the nitro-aminoadamantane compound used in the methods described herein is further described by formula (Ia): 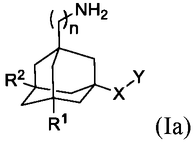 or a pharmaceutically acceptable salt thereof, wherein R¹, R², X and Y are as defined for formula (I); and n is 1, 2, 3, 4, 5 or 6.

In certain embodiments, the nitro-aminoadamantane compound used in the methods described herein is further described by formula (IA): 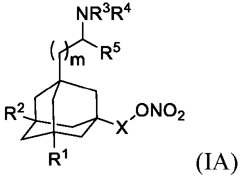 or a pharmaceutically acceptable salt thereof, wherein R¹, R², R³, R⁴, R⁵, X and m are as defined for formula (I).

In certain embodiments, the nitro-aminoadamantane compound used in the methods described herein is further described by formula (IA-a): 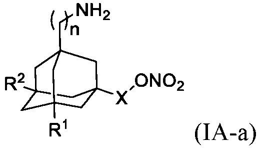 or a pharmaceutically acceptable salt thereof, wherein R¹, R² and X are as defined for formula (I); and n is 1, 2, 3, 4, 5 or 6.

In certain embodiments, the nitro-aminoadamantane compound used in the methods described herein is further described by formula (IB): 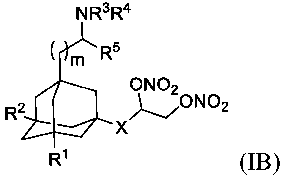 or a pharmaceutically acceptable salt thereof, wherein R¹, R², R³, R⁴, R⁵, X and m are as defined for formula (I).

In certain embodiments, the nitro-aminoadamantane compound used in the methods described herein is further described by formula (IB-a): 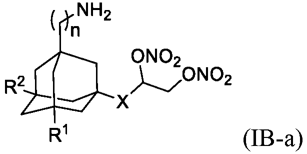 or a pharmaceutically acceptable salt thereof, wherein R¹, R² and X are as defined for formula (I); and n is 1, 2, 3, 4, 5 or 6.

In some embodiments, X of the compounds of formula (I) and subgenera thereof is bond, linear or branched C₁-C₆ or C₁-C₃ -alkyl-, or linear or branched C₁-C₆ or C₁-C₃ - O-alkyl-. In certain embodiments, X of the compounds of Formula I and subgenuses thereof is bond or linear or branched C₁-C₃ -alkyl- [e.g., -CH₂-, -(CH₂)₂-, -CHCH₃, - (CH₂)₃-, -ĆHCH₂CH₃, -CH₂CHCH₃ or -CH(CH₃)CH₂-].

The nitro-aminoadamantane compound used in the methods described herein can be a compound of formula (II) or formula (III): 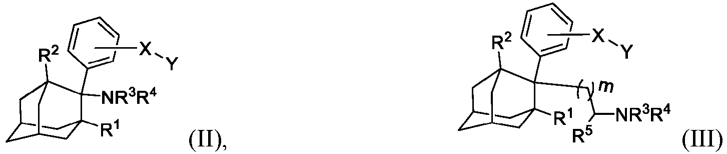 or a pharmaceutically acceptable salt thereof, wherein:
R¹ and R² independently are hydrogen, halide, linear or branched alkyl, linear or branched heteroalkyl, linear or branched alkoxy, linear or branched -O-heteroalkyl, cycloalkyl, heterocyclyl, aryl or heteroaryl, each of which can optionally be substituted;
R³ and R⁴ independently are hydrogen or linear or branched C₁-C₆ alkyl, or R³, R⁴ and the nitrogen atom to which they are attached form a 3-8-membered heterocyclic ring;
R⁵ is hydrogen or linear or branched C₁-C₆ alkyl;
X is bond, linear or branched -alkyl-, linear or branched -heteroalkyl-, linear or branched -O-alkyl-, linear or branched -O-heteroalkyl-, -(CH₂)ⱼ-cycloalkyl-(CH₂)ₖ-, - (CH₂)ⱼ-heterocyclyl-(CH₂)ₖ-, -(CH₂)ⱼ-aryl-(O)ₕ-(CH₂)ₖ- or -(CH₂)ⱼ-heteroaryl-(O)ₕ-(CH₂)ₖ-, each of which can optionally be substituted;
Y is -ONO₂ or 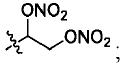
m is 0, 1, 2, 3, 4 or 5; j is 0, 1, 2 or 3; k is 0, 1, 2 or 3; and h is 0 or 1.

The nitro-aminoadamantane compound used in the methods described herein can be a compound of formula (IV): 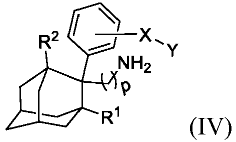 or a pharmaceutically acceptable salt thereof, wherein R¹, R², X and Y are as defined for formulas (II) and (III); and p is 0, 1, 2, 3, 4, 5 or 6.

In certain embodiments, the nitro-aminoadamantane compound used in the methods described herein is further described by formula (IV-a): 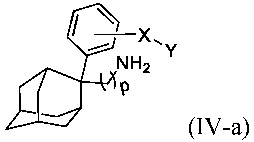 or a pharmaceutically acceptable salt thereof, wherein X and Y are as defined for formulas (II) and (III); and p is 0, 1, 2, 3, 4, 5 or 6.

In certain embodiments, the nitro-aminoadamantane compound used in the methods described herein is further described by formula (II-a) or formula (III-a): 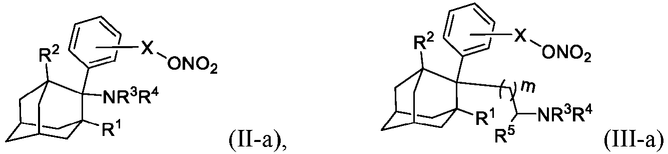 or a pharmaceutically acceptable salt thereof, wherein R¹, R², R³, R⁴, R⁵, X and m are as defined for formulas (II) and (III).

In certain embodiments, the nitro-aminoadamantane compound used in the methods described herein is further described by formula (IVA): 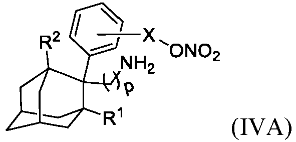 or a pharmaceutically acceptable salt thereof, wherein R¹, R² and X are as defined for formulas (II) and (III); and p is 0, 1, 2, 3, 4, 5 or 6.

In certain embodiments, the nitro-aminoadamantane compound used in the methods described herein is further described by formula (IVA-a): 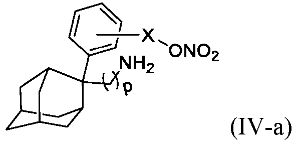 or a pharmaceutically acceptable salt thereof, wherein X is as defined for formulas (II) and (III); and p is 0, 1, 2, 3, 4, 5 or 6.

In certain embodiments, the nitro-aminoadamantane compound used in the methods described herein is further described by formula (IIB) or formula (IIIB): 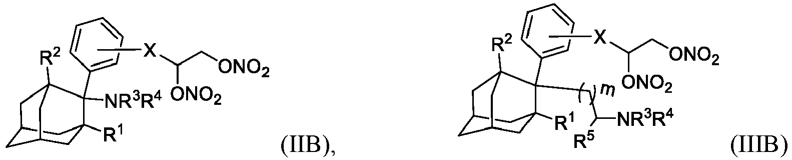 or a pharmaceutically acceptable salt thereof, wherein R¹, R², R³, R⁴, R⁵, X and m are as defined for formulas (II) and (III).

In certain embodiments, the nitro-aminoadamantane compound used in the methods described herein is further described by formula (IVB): 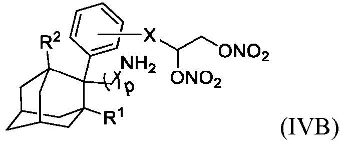 or a pharmaceutically acceptable salt thereof, wherein R¹, R² and X are as defined for formulas (II) and (III); and p is 0, 1, 2, 3, 4, 5 or 6.

In certain embodiments, the nitro-aminoadamantane compound used in the methods described herein is further described by formula (IVB-a): 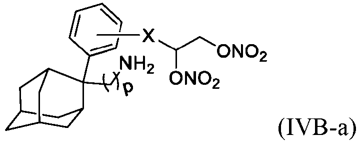 or a pharmaceutically acceptable salt thereof, wherein X is as defined for formulas (II) and (III); and p is 0, 1, 2, 3, 4, 5 or 6.

For the compounds of formulas (II) and (III), and subgenera thereof, the compounds of Formula **III** and subgenuses thereof, and the compounds of Formula IV and subgenuses thereof, the -X-Y, -X-ONO₂ or -X-CH(ONO₂)CH₂-ONO₂ moiety can be attached to an ortho position, a meta position or the para position of the phenyl ring. In certain embodiments, the -X-Y, -X-ONO₂ or -X-CH(ONO₂)CH₂-ONO₂ moiety is attached to a meta position of the phenyl ring.

For the compounds of formulas (II), (III), (IV), and subgenera thereof, in some embodiments X is bond, linear or branched C₁-C₆ or C₁-C₃ -alkyl-, or linear or branched C₁-C₆ or C₁-C₃ -O-alkyl-. In certain embodiments, X is bond or linear or branched C₁-C₃ -O-alkyl- [e.g., -O-CH₂-, -O-(CH₂)₂-, -O-CHCH₃, -O-(CH₂)₃-, -O-CHCH_{Z}CH₃, -O-CH₂CHCH₃ or -O-CH(CH₃)CH₂-].

For the compounds of formulas (I), (II) and (III), and subgenera thereof, examples of 3-8-membered, nitrogen-containing heterocyclic rings include without limitation aziridinyl, azetidinyl, pyrrolidinyl, piperidinyl, morpholinyl, piperazinyl, azepanyl and azocanyl, where the second ring nitrogen atom of a piperazinyl group can optionally be substituted with a linear C₁-C₃ alkyl group or a
-(C=O)R group, wherein R is hydrogen or linear C₁-C₃ alkyl. In certain embodiments, R³, R⁴ and the nitrogen atom to which they are attached form a 3-6-membered heterocyclic ring.

For the compounds of formulas (I), (III) and (IV), and subgenera thereof, in certain embodiments m is 0, 1 or 2; n is 1, 2 or 3; and p is 0, 1, 2 or 3.

For the compounds of formulas (I), (II) and (III), and subgenera thereof, in some embodiments both R³ and R⁴ are hydrogen. In other embodiments, one of R³ and R⁴ is hydrogen, and the other is linear or branched C₁-C₃ alkyl. In certain embodiments, one of R³ and R⁴ is hydrogen, and the other is methyl or ethyl. In yet other embodiments, R³ and R⁴ independently are linear C₁-C₃ alkyl (e.g., methyl or ethyl), optionally the same alkyl group.

For the compounds of formulas (I) and (III), and subgenera thereof, in some embodiments R⁵ is hydrogen. In other embodiments, R⁵ is linear or branched C₁-C₃ alkyl. In certain embodiments, R⁵ is methyl or ethyl.

For the compounds of formulas (I), (II), (III), and (IV), and subgenera thereof, in some embodiments R¹ and R² independently are hydrogen or linear or branched C₁-C₆ or C₁-C₃ alkyl. In certain embodiments, both R¹ and R² are hydrogen. In other embodiments, R¹ is hydrogen and R² is linear or branched C₁-C₆ or C₁-C₃ alkyl, or R² is hydrogen and R¹ is linear or branched C₁-C₆ or C₁-C₃ alkyl. In certain embodiments, R¹ is hydrogen and R² is methyl, ethyl or n-propyl, or R² is hydrogen and R¹ is methyl, ethyl or n-propyl. In yet other embodiments, R¹ and R² independently are linear or branched C₁-C₆ or C₁-C₃ alkyl, optionally the same alkyl group. In certain embodiments, R¹ and R² independently are methyl, ethyl or n-propyl, optionally the same alkyl group. In some embodiments, R¹ is hydrogen and R² is methyl or ethyl, or R² is hydrogen and R¹ is methyl or ethyl. In other embodiments, both R¹ and R² are methyl or ethyl.

For the compounds of formulas (I), (II), (III), and (IV), and subgenera thereof, in some embodiments the R¹ group, the R² group or the X group, or any combination or all thereof, independently are substituted with 1, 2 or 3 substituents selected from linear or branched C₁-C₆ or C₁-C₃ alkyl, haloalkyl, -OR⁶, -NR⁷R⁸, -ONO₂, -CN, -C(=O)R⁶, - C(=O)OR⁶, -OC(=O)R⁶, -C(=O)NR⁷R⁸, -NR⁷C(=O)R⁶, -OC(=O)OR⁶, -OC(=O)NR⁷R⁸, - NR⁷C(=O)OR⁶, -NR⁶C(=O)NR⁷R³, aryl and heteroaryl, or/and are substituted with 1 to 6 halogen (e.g., fluorine) or have all available hydrogen atoms replaced with halogen (e.g., fluorine), wherein R⁶ in each occurrence independently is hydrogen or linear or branched C₁-C₆ or C₁-C₃ alkyl; and R⁷ and R⁸ in each occurrence independently are hydrogen or linear or branched C₁-C₆ or C₁-C₃ alkyl, or R⁷, R⁸ and the nitrogen atom to which they are attached form a 3-6-membered ring. In certain embodiments, the R¹ group, the R² group or the X group, or any combination or all thereof, independently are monovalent or divalent fluoroalkyl or alkyl-ONO₂.

For the compounds of formulas (I), (II), (III), and (IV), and subgenera thereof, non-limiting examples of linear or branched C₁-C₆ alkyl groups include methyl, ethyl, n-propyl, isopropyl, n-butyl, isobutyl, sec-butyl, *tert-butyl,* n-pentyl and n-hexyl. Examples of linear or branched C₁-C₃ alkyl groups include methyl, ethyl, n-propyl and isopropyl.

For the compounds of formulas (I), (II), (III), and (IV), and subgenera thereof, in some embodiments X has 0, 1, 2, 3, 4, 5 or 6 carbon atoms. In certain embodiments, X has 0, 1, 2 or 3 carbon atoms.

Table A1 depicts representative compounds of formula (IA-a-i) to (IA-a-xx):

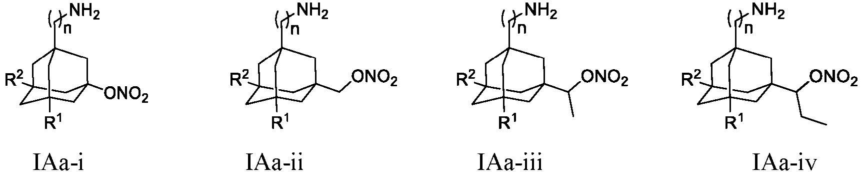

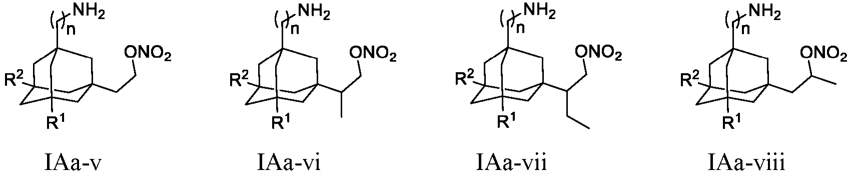

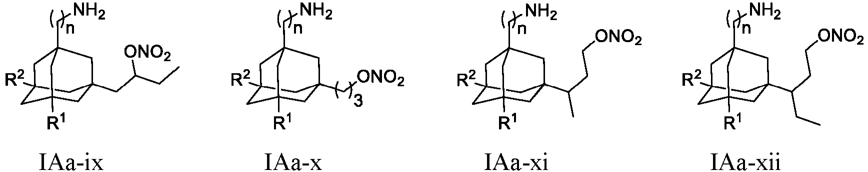

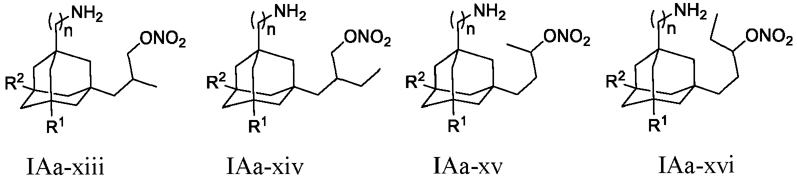

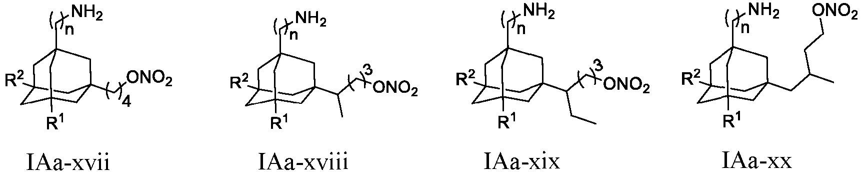

**Table A1**

| For each subgenus of formula (IA-a-i) to (IA-a-xx): | | |
|---|---|---|
| **n** | **R¹** | **R²** |
| 1, 2 and 3 | methyl | methyl |
| 1, 2 and 3 | hydrogen | methyl |
| 1, 2 and 3 | methyl | hydrogen |
| 1, 2 and 3 | ethyl | ethyl |
| 1, 2 and 3 | hydrogen | ethyl |
| 1, 2 and 3 | ethyl | hydrogen |
| 1, 2 and 3 | n-propyl | n-propyl |
| 1, 2 and 3 | hydrogen | n-propyl |
| 1, 2 and 3 | n-propyl | hydrogen |
| 1, 2 and 3 | isopropyl | isopropyl |
| 1, 2 and 3 | hydrogen | isopropyl |
| 1, 2 and 3 | isopropyl | hydrogen |
| 1, 2 and 3 | n-butyl | n-butyl |
| 1, 2 and 3 | hydrogen | n-butyl |
| 1, 2 and 3 | n-butyl | hydrogen |
| 1, 2 and 3 | isobutyl | isobutyl |
| 1, 2 and 3 | hydrogen | isobutyl |
| 1, 2 and 3 | isobutyl | hydrogen |
| 1, 2 and 3 | *sec*-butyl | *sec*-butyl |
| 1, 2 and 3 | hydrogen | *sec*-butyl |
| 1, 2 and 3 | *sec*-butyl | hydrogen |
| 1, 2 and 3 | -CH₂-ONO₂ | -CH₂-ONO₂ |
| 1, 2 and 3 | hydrogen | -CH₂-ONO₂ |
| 1, 2 and 3 | -CH₂-ONO₂ | hydrogen |
| 1, 2 and 3 | -(CH₂)₂-ONO₂ | -(CH₂)₂-ONO₂ |
| 1, 2 and 3 | hydrogen | -(CH₂)₂-ONO₂ |
| 1, 2 and 3 | -(CH₂)₂-ONO₂ | hydrogen |
| 1, 2 and 3 | -(CH₂)₃-ONO₂ | -(CH₂)₃-ONO₂ |
| 1, 2 and 3 | hydrogen | -(CH₂)₃-ONO₂ |
| 1, 2 and 3 | -(CH₂)₃-ONO₂ | hydrogen |
| 1, 2 and 3 | -CH₂CH(ONO₂)CH₃ | -CH₂CH(ONO₂)CH₃ |
| 1, 2 and 3 | hydrogen | -CH₂CH(ONO₂)CH₃ |
| 1, 2 and 3 | -CH₂CH(ONO₂)CH₃ | hydrogen |
| 1, 2 and 3 | -CH₂CH(CH₃)CH₂-ONO₂ | -CH₂CH(CH₃)CH₂-ONO₂ |
| 1, 2 and 3 | hydrogen | -CH₂CH(CH₃)CH₂-ONO₂ |
| 1, 2 and 3 | -CH₂CH(CH₃)CH₂-ONO₂ | hydrogen |

In certain embodiments, the nitro-aminoadamantane compound used in the methods described herein is further described by a subgenus of formula (IA-a-i) to (IA-a-xx), or a pharmaceutically acceptable salt thereof.

In certain embodiments, the nitro-aminoadamantane compound used in the methods described herein is selected from: 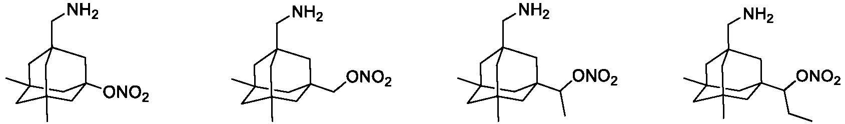 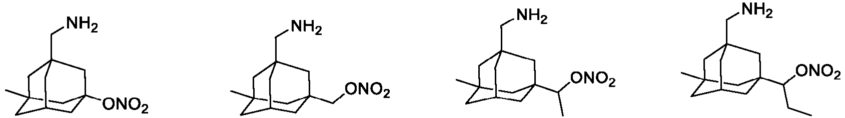 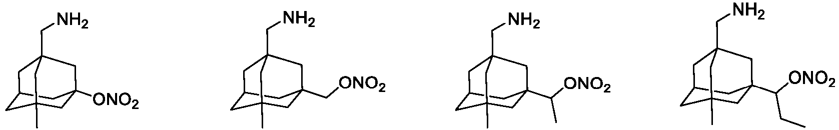 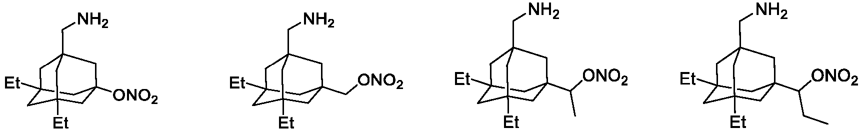 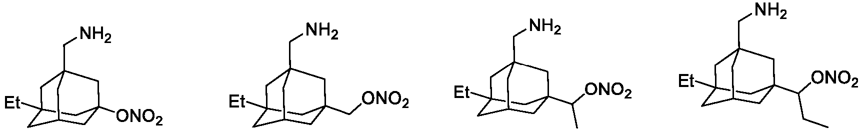 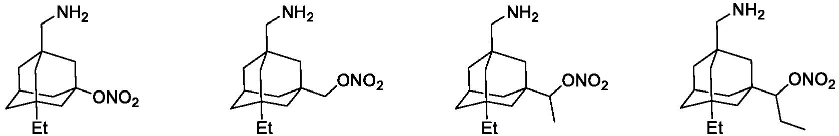 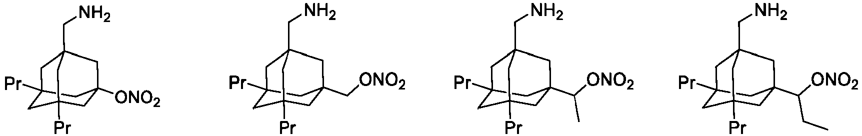 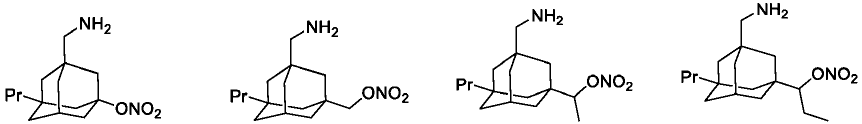 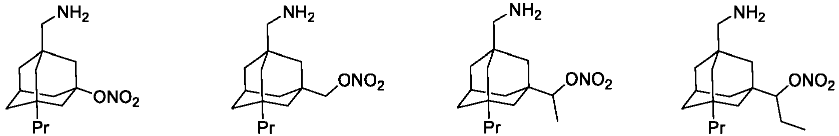 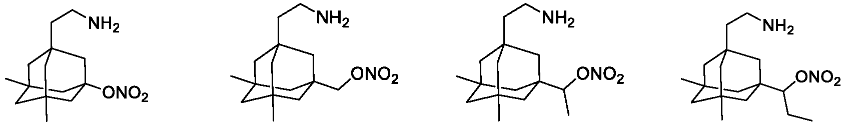 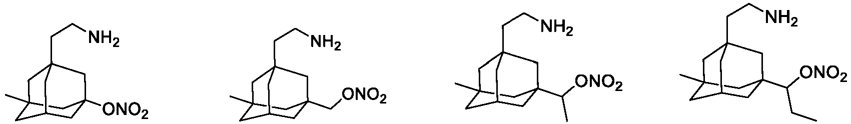 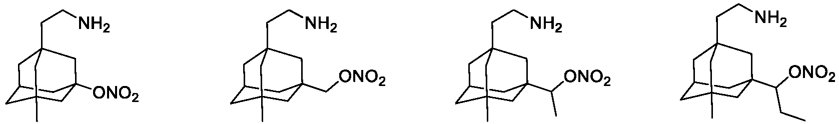 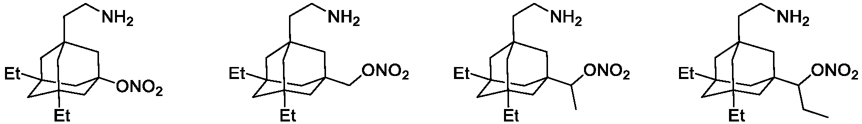 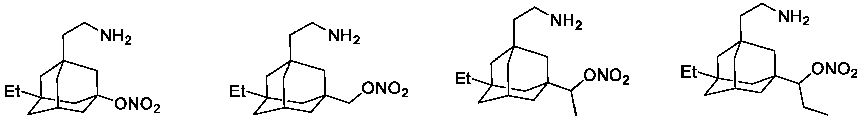 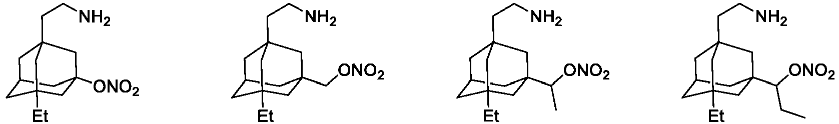 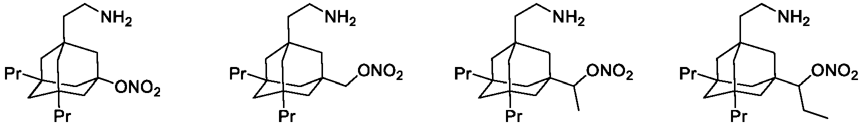 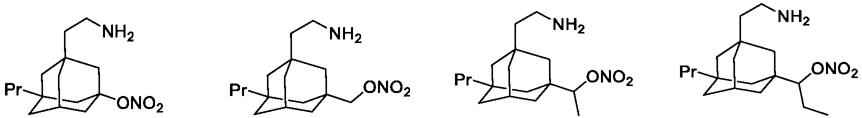 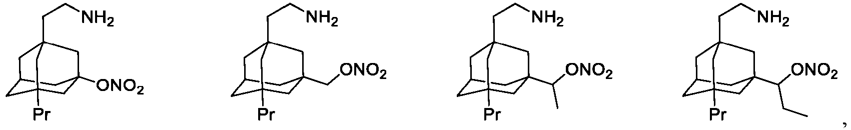 and pharmaceutically acceptable salts thereof.

Table A2 depicts representative compounds of formula (IB-a-i) to (IB-a-vii):

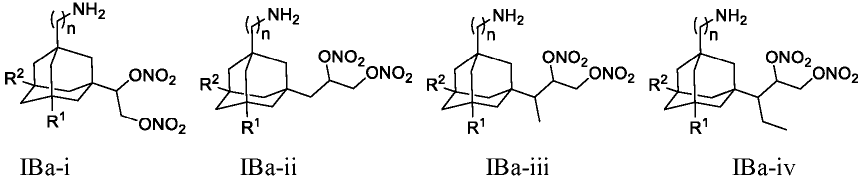

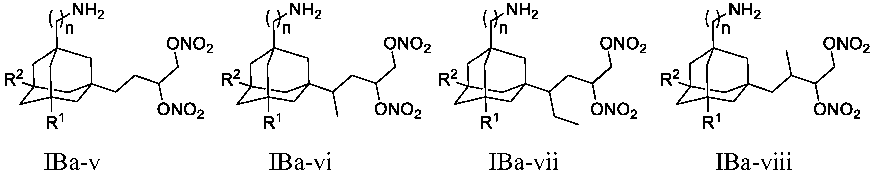

**Table A2**

| For each subgenus of formula (IB-a-i) to (IB-a-vii): | | |
|---|---|---|
| **n** | **R¹** | **R²** |
| 1, 2 and 3 | methyl | methyl |
| 1, 2 and 3 | hydrogen | methyl |
| 1, 2 and 3 | methyl | hydrogen |
| 1, 2 and 3 | ethyl | ethyl |
| 1, 2 and 3 | hydrogen | ethyl |
| 1, 2 and 3 | ethyl | hydrogen |
| 1, 2 and 3 | n-propyl | n-propyl |
| 1, 2 and 3 | hydrogen | n-propyl |
| 1, 2 and 3 | n-propyl | hydrogen |
| 1, 2 and 3 | isopropyl | isopropyl |
| 1, 2 and 3 | hydrogen | isopropyl |
| 1, 2 and 3 | isopropyl | hydrogen |
| 1, 2 and 3 | n-butyl | n-butyl |
| 1, 2 and 3 | hydrogen | n-butyl |
| 1, 2 and 3 | n-butyl | hydrogen |
| 1, 2 and 3 | isobutyl | isobutyl |
| 1, 2 and 3 | hydrogen | isobutyl |
| 1, 2 and 3 | isobutyl | hydrogen |
| 1, 2 and 3 | sec-butyl | sec-butyl |
| 1, 2 and 3 | hydrogen | *sec*-butyl |
| 1,2 and 3 | sec-butyl | hydrogen |
| 1, 2 and 3 | -CH₂-ONO₂ | -CH₂-ONO₂ |
| 1, 2 and 3 | hydrogen | -CH₂-ONO₂ |
| 1, 2 and 3 | -CH₂-ONO₂ | hydrogen |
| 1, 2 and 3 | -(CH₂)₂-ONO₂ | -(CH₂)₂-ONO₂ |
| 1, 2 and 3 | hydrogen | -(CH₂)₂-ONO₂ |
| 1, 2 and 3 | -(CH₂)₂-ONO₂ | hydrogen |
| 1, 2 and 3 | -(CH₂)₃-ONO₂ | -(CH₂)₃-ONO₂ |
| 1, 2 and 3 | hydrogen | -(CH₂)₃-ONO₂ |
| 1, 2 and 3 | -(CH₂)₃-ONO₂ | hydrogen |
| 1, 2 and 3 | -CH₂CH(ONO₂)CH₃ | -CH₂CH(ONO₂)CH₃ |
| 1, 2 and 3 | hydrogen | -CH₂CH(ONO₂)CH₃ |
| 1, 2 and 3 | -CH₂CH(ONO₂)CH₃ | hydrogen |
| 1, 2 and 3 | -CH₂CH(CH₃)CH₂-ONO₂ | -CH₂CH(CH₃)CH₂-ONO₂ |
| 1, 2 and 3 | hydrogen | -CH₂CH(CH₃)CH₂-ONO₂ |
| 1, 2 and 3 | -CH₂CH(CH₃)CH₂-ONO₂ | hydrogen |

In certain embodiments, the nitro-aminoadamantane compound used in the methods described herein is further described by a subgenus of formula (IB-a-i) to (IB-a-vii), or a pharmaceutically acceptable salt thereof.

In certain embodiments, the nitro-aminoadamantane compound used in the methods described herein is selected from: 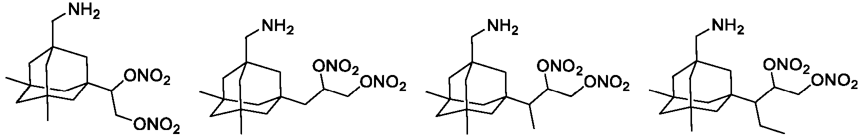 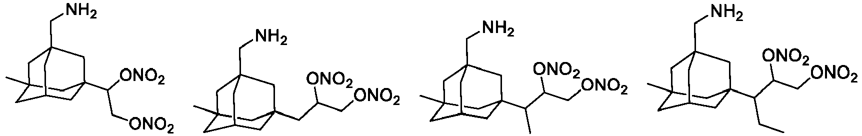 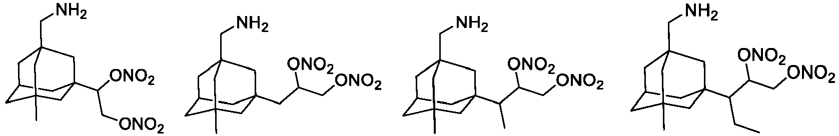 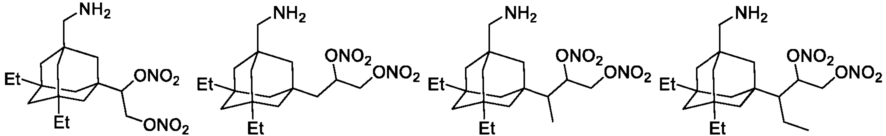 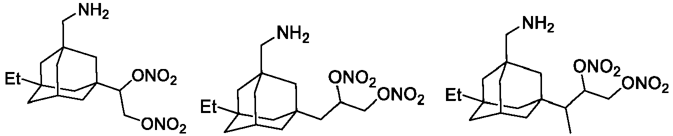 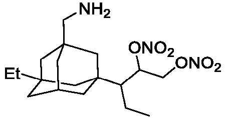 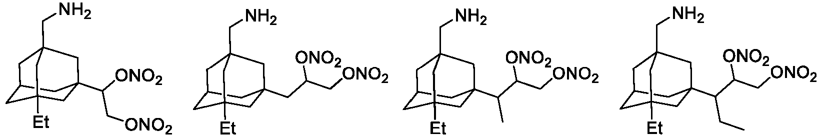 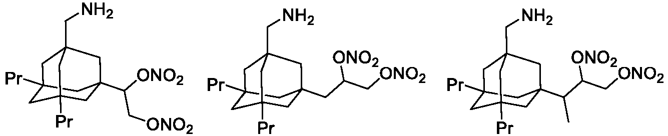 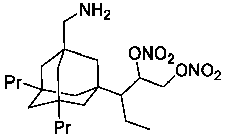 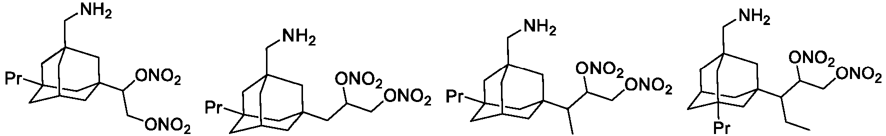 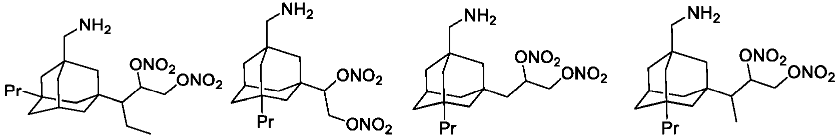 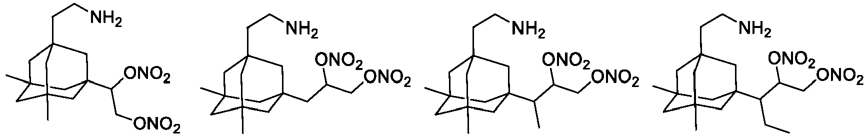 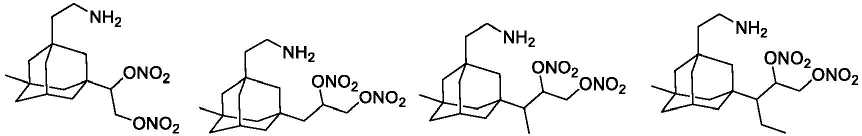 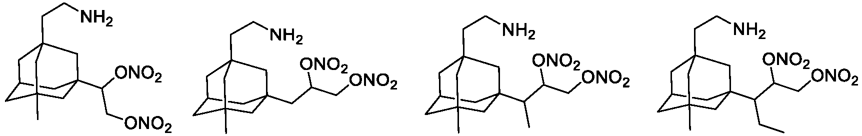 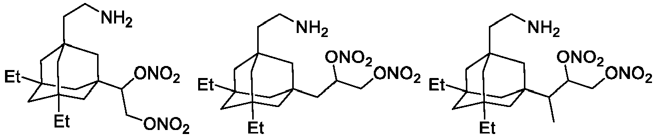 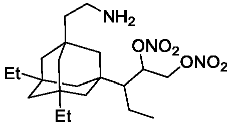 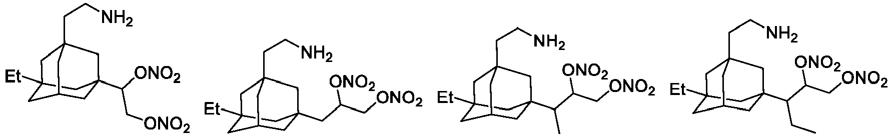 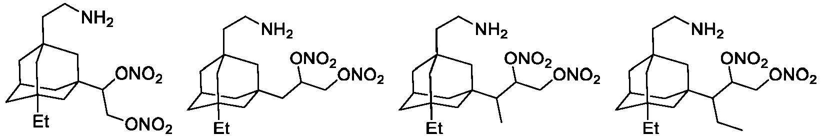 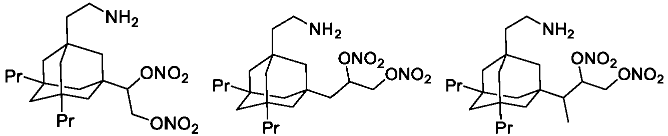 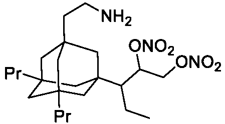 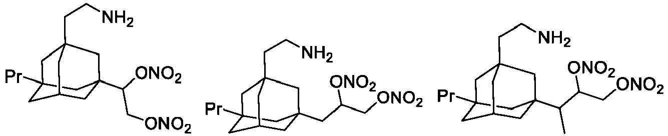 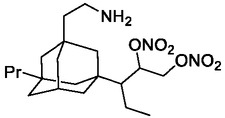 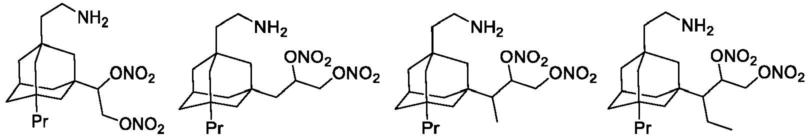 and pharmaceutically acceptable salts thereof.

Table A3 depicts representative compounds of formula (IVA-i) to (IVA-vii):

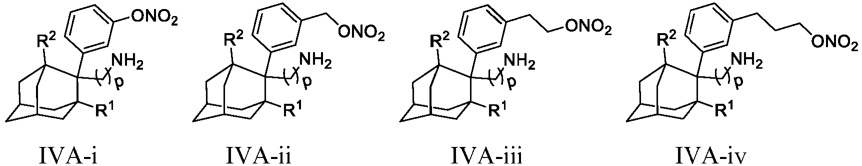

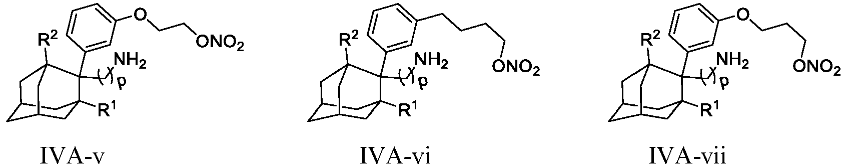

**Table A3**

| For each subgenus of formula (IVA-i) to (IVA-vii) | | |
|---|---|---|
| **p** | **R¹** | **R²** |
| 0, 1, 2 and 3 | hydrogen | hydrogen |
| 0, 1, 2 and 3 | methyl | methyl |
| 0, 1, 2 and 3 | hydrogen | methyl |
| 0, 1, 2 and 3 | methyl | hydrogen |
| 0, 1, 2 and 3 | ethyl | ethyl |
| 0, 1, 2 and 3 | hydrogen | ethyl |
| 0, 1, 2 and 3 | ethyl | hydrogen |
| 0, 1, 2 and 3 | n-propyl | n-propyl |
| 0, 1, 2 and 3 | hydrogen | n-propyl |
| 0, 1, 2 and 3 | n-propyl | hydrogen |
| 0, 1, 2 and 3 | isopropyl | isopropyl |
| 0, 1, 2 and 3 | hydrogen | isopropyl |
| 0, 1, 2 and 3 | isopropyl | hydrogen |
| 0, 1, 2 and 3 | n-butyl | n-butyl |
| 0, 1, 2 and 3 | hydrogen | n-butyl |
| 0, 1, 2 and 3 | n-butyl | hydrogen |
| 0, 1, 2 and 3 | isobutyl | isobutyl |
| 0, 1, 2 and 3 | hydrogen | isobutyl |
| 0, 1, 2 and 3 | isobutyl | hydrogen |
| 0, 1, 2 and 3 | *sec*-butyl | *sec*-butyl |
| 0, 1, 2 and 3 | hydrogen | *sec*-butyl |
| 0, 1, 2 and 3 | *sec*-butyl | hydrogen |
| 0, 1, 2 and 3 | -CH₂-ONO₂ | -CH₂-ONO₂ |
| 0, 1, 2 and 3 | hydrogen | -CH₂-ONO₂ |
| 0, 1, 2 and 3 | -CH₂-ONO₂ | hydrogen |
| 0, 1, 2 and 3 | -(CH₂)₂-ONO₂ | -(CH₂)₂-ONO₂ |
| 0, 1, 2 and 3 | hydrogen | -(CH₂)₂-ONO₂ |
| 0, 1, 2 and 3 | -(CH₂)₂-ONO₂ | hydrogen |
| 0, 1, 2 and 3 | -(CH₂)₃-ONO₂ | -(CH₂)₃-ONO₂ |
| 0, 1, 2 and 3 | hydrogen | -(CH₂)₃-ONO₂ |
| 0, 1, 2 and 3 | -(CH₂)₃-ONO₂ | hydrogen |
| 0, 1, 2 and 3 | -CH₂CH(ONO₂)CH₃ | -CH₂CH(ONO₂)CH₃ |
| 0, 1, 2 and 3 | hydrogen | -CH₂CH(ONO₂)CH₃ |
| 0, 1, 2 and 3 | -CH₂CH(ONO₂)CH₃ | hydrogen |
| 0, 1, 2 and 3 | -CH₂CH(CH₃)CH₂-ONO₂ | -CH₂CH(CH₃)CH₂-ONO₂ |
| 0, 1, 2 and 3 | hydrogen | -CH₂CH(CH₃)CH₂-ONO₂ |
| 0, 1, 2 and 3 | -CH₂CH(CH₃)CH₂-ONO₂ | hydrogen |

In certain embodiments, the nitro-aminoadamantane compound used in the methods described herein is further described by a subgenus of formula (IVA-i) to (IVA-vii), or a pharmaceutically acceptable salt thereof.

In certain embodiments, the nitro-aminoadamantane compound used in the methods described herein is selected from: 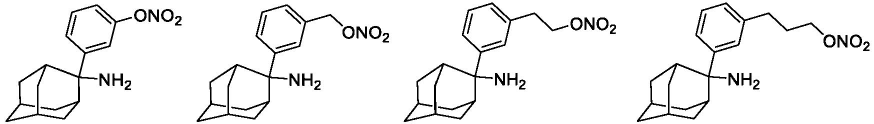 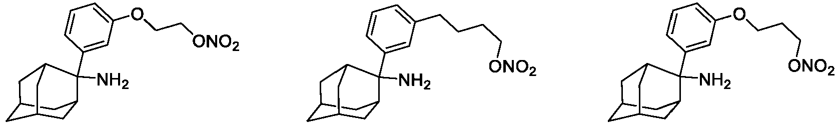 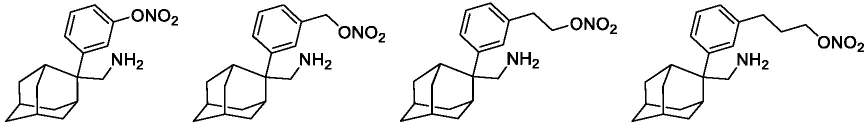 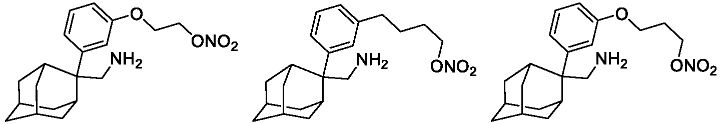 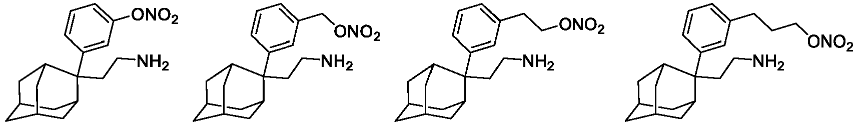 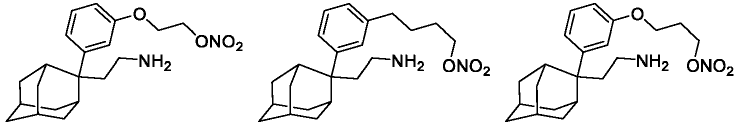 and pharmaceutically acceptable salts thereof.

Table A4 depicts representative compounds of formula (IVB-i) to (IVB-vi):

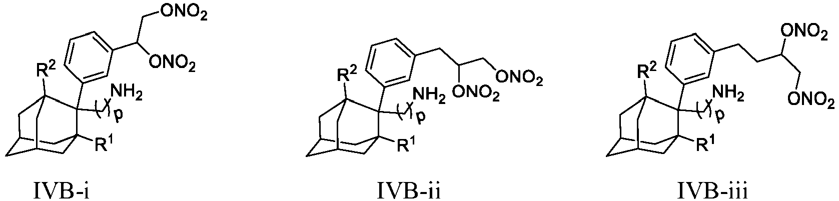

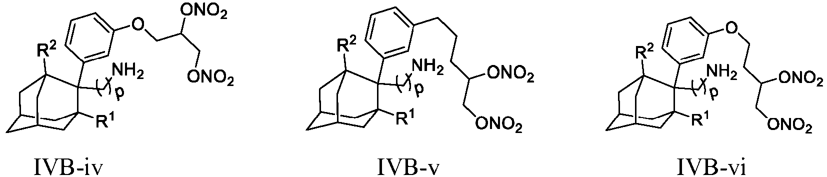

**Table A4**

| For each subgenus of formula (IVB-i) to (IVB-vi) | | |
|---|---|---|
| **p** | **R¹** | **R²** |
| 0, 1, 2 and 3 | hydrogen | hydrogen |
| 0, 1, 2 and 3 | methyl | methyl |
| 0, 1, 2 and 3 | hydrogen | methyl |
| 0, 1, 2 and 3 | methyl | hydrogen |
| 0, 1, 2 and 3 | ethyl | ethyl |
| 0, 1, 2 and 3 | hydrogen | ethyl |
| 0, 1, 2 and 3 | ethyl | hydrogen |
| 0, 1, 2 and 3 | n-propyl | n-propyl |
| 0, 1, 2 and 3 | hydrogen | n-propyl |
| 0, 1, 2 and 3 | n-propyl | hydrogen |
| 0, 1, 2 and 3 | isopropyl | isopropyl |
| 0, 1, 2 and 3 | hydrogen | isopropyl |
| 0, 1, 2 and 3 | isopropyl | hydrogen |
| 0, 1, 2 and 3 | n-butyl | n-butyl |
| 0, 1, 2 and 3 | hydrogen | n-butyl |
| 0, 1, 2 and 3 | n-butyl | hydrogen |
| 0, 1, 2 and 3 | isobutyl | isobutyl |
| 0, 1, 2 and 3 | hydrogen | isobutyl |
| 0, 1, 2 and 3 | isobutyl | hydrogen |
| 0, 1, 2 and 3 | sec-butyl | *sec*-butyl |
| 0, 1, 2 and 3 | hydrogen | *sec*-butyl |
| 0, 1, 2 and 3 | sec-butyl | hydrogen |
| 0, 1, 2 and 3 | -CH₂-ONO₂ | -CH₂-ONO₂ |
| 0, 1, 2 and 3 | hydrogen | -CH₂-ONO₂ |
| 0, 1, 2 and 3 | -CH₂-ONO₂ | hydrogen |
| 0, 1, 2 and 3 | -(CH₂)₂-ONO₂ | -(CH₂)₂-ONO₂ |
| 0, 1, 2 and 3 | hydrogen | -(CH₂)₂-ONO₂ |
| 0, 1, 2 and 3 | -(CH₂)₂-ONO₂ | hydrogen |
| 0, 1, 2 and 3 | -(CH₂)₃-ONO₂ | -(CH₂)₃-ONO₂ |
| 0, 1, 2 and 3 | hydrogen | -(CH₂)₃-ONO₂ |
| 0, 1, 2 and 3 | -(CH₂)₃-ONO₂ | hydrogen |
| 0, 1, 2 and 3 | -CH₂CH(ONO₂)CH₃ | -CH₂CH(ONO₂)CH₃ |
| 0, 1, 2 and 3 | hydrogen | -CH₂CH(ONO₂)CH₃ |
| 0, 1, 2 and 3 | -CH₂CH(ONO₂)CH₃ | hydrogen |
| 0, 1, 2 and 3 | -CH₂CH(CH₃)CH₂-ONO₂ | -CH₂CH(CH₃)CH₂-ONO₂ |
| 0, 1, 2 and 3 | hydrogen | -CH₂CH(CH₃)CH₂-ONO₂ |
| 0, 1, 2 and 3 | -CH₂CH(CH₃)CH₂-ONO₂ | hydrogen |

In certain embodiments, the nitro-aminoadamantane compound used in the methods described herein is further described by a subgenus of formula (IVB-i) to (IVB-vi), or a pharmaceutically acceptable salt thereof.

In certain embodiments, the nitro-aminoadamantane compound used in the methods described herein is selected from: 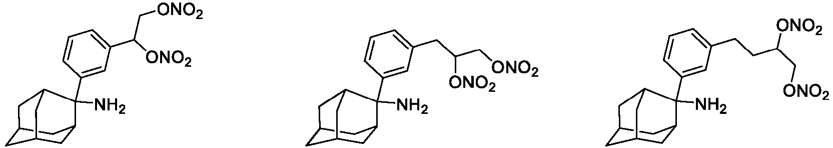 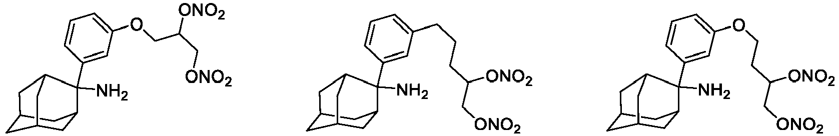 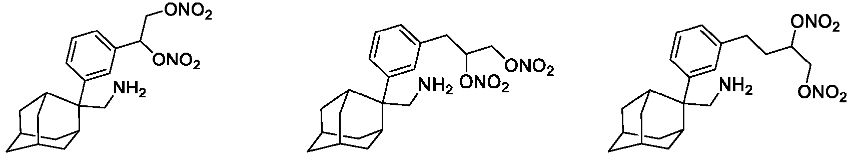 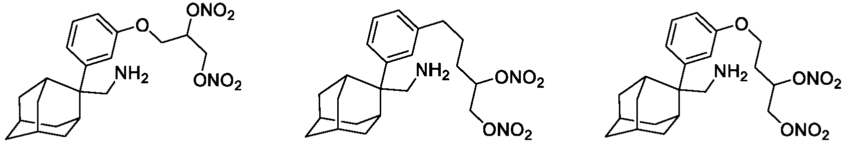 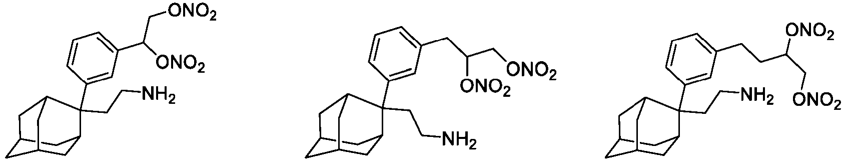 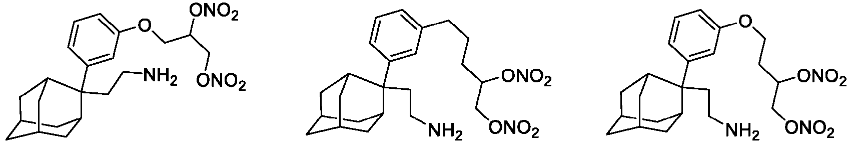 and pharmaceutically acceptable salts thereof.

In particular embodiments, instead of being an amine group, the amine group indirectly or directly connected to the C-1 or C-2 position of the nitro-aminoadamantane compounds described herein can be an amide, carbamate or urea (e.g., an -NH(C=O)R amide, -NH(C=O)OR carbamate or -NHC(=O)NR^{a}R^{b} urea group)s. In some embodiments, the -NR³R⁴ moiety of nitro-aminoadamantane compounds is - NH(C=O)R⁶, -NH(C=O)OR⁶ or -NHC(=O)NR⁷R⁸, wherein R⁶ is hydrogen (for formamide) or linear or branched C₁-C₆ alkyl, and R⁷ and R⁸ independently are hydrogen or linear or branched C₁-C₆ alkyl, or R⁷, R⁸ and the nitrogen atom to which they are attached form a 3-6-membered ring. In certain embodiments, R⁶ is hydrogen (for formamide) or linear or branched C₁-C₃ alkyl (e.g., methyl or ethyl), and R⁷ and R⁸ independently are hydrogen or linear or branched C₁-C₃ alkyl (e.g., methyl or ethyl).

The nitro-aminoadamantane compound used in the methods described herein can be a compound of formula (V): 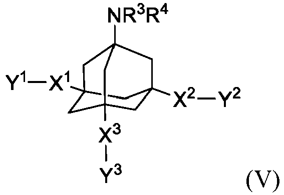 or a pharmaceutically acceptable salt thereof, wherein:
R³ and R⁴ independently are hydrogen or linear or branched C₁-C₆ alkyl, or R³, R⁴ and the nitrogen atom to which they are attached form a 3-8-membered heterocyclic ring;
R⁵ is hydrogen or linear or branched C₁-C₆ alkyl;
each of X¹, X², and X³ is, independently, selected from a bond, linear or branched -alkyl-, linear or branched -heteroalkyl-, linear or branched -O-alkyl-, linear or branched -O-heteroalkyl-, -(CH₂)ⱼ-cycloalkyl-(CH₂)ₖ-, -(CH₂)ⱼ-heterocyclyl-(CH₂)ₖ-, - (CH₂)ⱼ-aryl-(O)ₕ-(CH₂)ₖ- or -(CH₂)ⱼ-heteroaryl-(O)ₕ-(CH₂)ₖ-, each of which can optionally be substituted;
each of Y¹, Y², and Y³ is, independently, selected from -ONO₂, 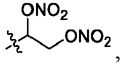 hydroxy, or a hydrogen atom, provided that at least one of Y¹, Y², and Y³ is -ONO₂ or 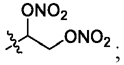
j is 0, 1, 2 or 3; k is 0, 1, 2 or 3; and h is 0 or 1.

In certain embodiments, the nitro-aminoadamantane compound used in the methods described herein is further described by formula (VA): 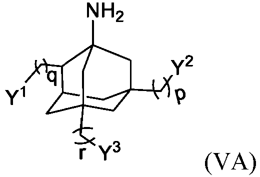 or a pharmaceutically acceptable salt thereof, wherein each of Y¹, Y², and Y³ is, independently, selected from -ONO₂, hydroxy, or a hydrogen atom; p is 0, 1, 2, 3, 4, 5 or 6; q is 0, 1, 2, 3, 4, 5 or 6; and r is 0, 1, 2, 3, 4, 5 or 6, provided that at least one of Y¹, Y², and Y³ is -ONO₂.

In certain embodiments, the nitro-aminoadamantane compound used in the methods described herein is selected from: 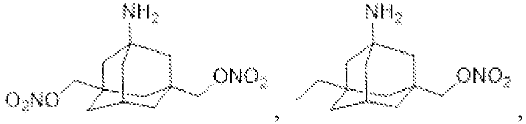 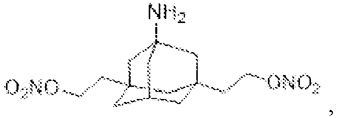 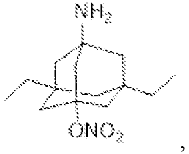 and
and pharmaceutically acceptable salts thereof.

### Pharmaceutical Compositions

The methods described herein utilize pharmaceutical compositions including a glutamate modulating agent described herein, or a pharmaceutically acceptable salt thereof, and one or more pharmaceutically acceptable excipients or carriers. The pharmaceutical compositions/formulations can be prepared in sterile form. For example, pharmaceutical compositions/formulations for parenteral administration by injection or infusion generally are sterile. Sterile pharmaceutical compositions/formulations are compounded or manufactured according to pharmaceutical-grade sterilization standards known to those of skill in the art, such as those disclosed in or required by the United States Pharmacopeia Chapters 797, 1072 and 1211, and 21 Code of Federal Regulations 211. Pharmaceutically acceptable excipients and carriers can include pharmaceutically acceptable substances, materials and vehicles. Non-limiting examples of types of excipients include liquid and solid fillers, diluents, binders, lubricants, glidants, surfactants, dispersing agents, disintegration agents, emulsifying agents, wetting agents, suspending agents, thickeners, solvents, isotonic agents, buffers, pH adjusters, absorption-delaying agents, stabilizers, antioxidants, preservatives, antimicrobial agents, antibacterial agents, antifungal agents, chelating agents, adjuvants, sweetening agents, flavoring agents, coloring agents, encapsulating materials and coating materials. The use of such excipients in pharmaceutical formulations is known in the art. For example, conventional vehicles and carriers include without limitation oils (e.g., vegetable oils, such as olive oil and sesame oil), aqueous solvents (e.g., saline, buffered saline (e.g., phosphate-buffered saline [PBS]) and isotonic solutions (e.g., Ringer's solution)), and organic solvents (e.g., dimethyl sulfoxide [DMSO] and alcohols [e.g., ethanol, glycerol and propylene glycol]). See, e.g., Remington: The Science and Practice of Pharmacy, 21st Ed., Lippincott Williams & Wilkins (Philadelphia, Pennsylvania) (2005); Handbook of Pharmaceutical Excipients, 5th Ed., Rowe et al., Eds., The Pharmaceutical Press and the American Pharmaceutical Association (2005); Handbook of Pharmaceutical Additives, 3rd Ed., Ash and Ash, Eds., Gower Publishing Co. (2007); and Pharmaceutical Pre-formulation and Formulation, Gibson, Ed., CRC Press LLC (Boca Raton, Florida) (2004). Appropriate formulation can depend on various factors, such as the route of administration chosen. Potential routes of administration of pharmaceutical compositions including glutamate modulating agents include without limitation oral, parenteral (including intradermal, subcutaneous, intramuscular, intravascular, intravenous, intraarterial, intraperitoneal, intracavitary, intramedullary, intrathecal and topical), and topical (including dermal/epicutaneous, transdermal, mucosal, transmucosal, intranasal [e.g., by nasal spray or drop], ocular/intraocular [e.g., by eye drop], pulmonary [e.g., by oral or nasal inhalation], buccal, sublingual, rectal [e.g., by suppository], and vaginal [e.g., by suppository]). Topical formulations can be designed to produce a local or systemic therapeutic effect. As an example, formulations of glutamate modulating agents suitable for oral administration can be presented as, e.g., capsules (including push-fit capsules and soft capsules), tablets, pills, cachets or lozenges; as powders or granules; as semisolids, electuaries, pastes or gels; as solutions or suspensions in an aqueous liquid or/and a non-aqueous liquid; or as oil-in-water liquid emulsions or water-in-oil liquid emulsions. Tablets can contain a glutamate modulating agent in admixture with, e.g., a filler or inert diluent (e.g., calcium carbonate, calcium phosphate, lactose, mannitol or microcrystalline cellulose), a binding agent (e.g., a starch, gelatin, acacia, alginic acid or a salt thereof, or microcrystalline cellulose), a lubricating agent (e.g., stearic acid, magnesium stearate, talc or silicon dioxide), and a disintegrating agent (e.g., crospovidone, croscarmellose sodium or colloidal silica), and optionally a surfactant (e.g., sodium lauryl sulfate). Compositions for oral administration can also be formulated as solutions or suspensions in an aqueous liquid or/and a non-aqueous liquid, or as oil-in-water liquid emulsions or water-in-oil liquid emulsions. Dispersible powder or granules of a glutamate modulating agent can be mixed with any suitable combination of an aqueous liquid, an organic solvent or/and an oil and any suitable excipients (e.g., any combination of a dispersing agent, a wetting agent, a suspending agent, an emulsifying agent or/and a preservative) to form a solution, suspension or emulsion.

### Methods of Treatment

Described herein are methods for the treatment of subjects who have ASD, to improve one or more impairments associated with ASD.

The present methods include the administration of a glutamate modulating (lowering) agent, such as, without limitation, memantine, lamotrigine, amantadine, D-cycloserine, N-Acetylcysteine, or other nitro-aminoadamantane derivatives including the agents described herein, or a pharmaceutically acceptable salt thereof. Generally, the methods include administering a therapeutically effective amount of a glutamate modulating agent as described herein, to a subject who has been identified by a method described herein as being likely to respond to such treatment, i.e., a subject who has a level of glutamate above a reference level. In some embodiments, the glutamate modulating agent is an NMDAR modulating (inhibiting) agent.

In some embodiments, the treatment comprises administration of the glutamate modulating agent at least once a day, twice a day, every other day, once a week, or once a month, and the treatment can be continued for at least a week, a month, three months, six months, or a year or more.

In some embodiments, the administration follows the FDA Guidelines for Dosage and Administration of other glutamate modulating agents.

For example, where memantine is used, doses in the range of 20-40 mg/day can be used.

In cases where lamotrigine (which has been approved for the treatment of Bipolar Disorder in adults aged 18+) is used, an escalation regimen as shown in the following table can be used (lower doses can be used as appropriate for younger subjects):

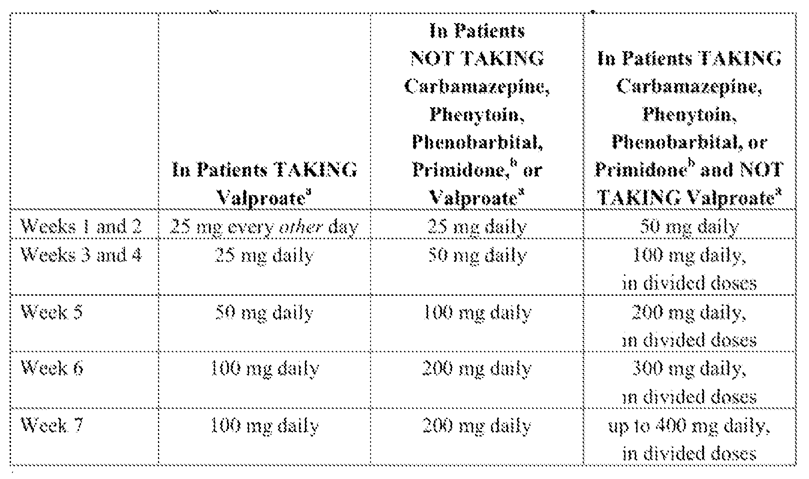

Where amantadine (which has been approved for treatment of Parkinsonism) is used, a dose of 100 mg, 2x a day (200 mg total) can be used, or 2.5mg/kg per day for one week, then increased to 5.0mg/kg per day (King, et al., 2001), or 100 to 150mg/day for patients less than 30kg or more than 30kg, respectively (administered in conjunction with risperidone (Mohammadi, et al., 2013).

Where D-cycloserine (which has been approved for treatment of renal disease) is used, an initial dosage can be up to 250 mg 2x a day, increased to 500 mg and up to 1g daily in divided doses monitored by blood levels. In some embodiments, the dose is 50 mg daily or 50mg weekly (Urbano et al., 2015); 50 mg/week (Minshawi et al., 2016).

Where N-acetylcysteine (which has been approved for treatment of acetaminophen toxicity and hepatic injury) is used, an exemplary Loading dose of 140 mg/kg can be used; other alternative regimens include 900 mg daily for four weeks, then 900mg twice or three times daily for four weeks can be used (Hardan et al., 2012), *or* 60mg/kg/day in three divided doses (Wink et al., 2016).

As used in this context, to "treat" means to ameliorate at least one symptom of the ASD. In some embodiments, the improvement is in one or more impairments measured in the Social Responsiveness Scale-Second Edition (SRS-2; Constantino et al, 2003) and/or a score on the clinician-rated National Institute of Mental Health (NIMH) Clinical Global Impression (CGI) severity scale, e.g., as shown in a Table or Figure herein.

The therapeutically effective amount and the frequency of administration of a glutamate modulating agent to treat ASD may depend on various factors, including the type of disorder, the severity of the condition, the potency of the glutamate modulating agent, the mode of administration, the age, body weight, general health, gender and diet of the subject, and the response of the subject to the treatment, and can be determined by the treating physician. In some embodiments, the effective dose of a glutamate modulating agent per day is from about 1, 5 or 10 mg to about 100 mg, or as deemed appropriate by the treating physician, which can be administered in a single dose or in divided doses. The glutamate modulating agent can be administered in any suitable frequency to treat ASD, which can be determined by the treating physician, including one, two, three or more times daily, once every two days, once every three days, twice weekly or once weekly, or as deemed appropriate by the treating physician. In certain embodiments, the glutamate modulating agent is administered once daily. The glutamate modulating agent can be administered via any suitable route for the treatment of ASD (e.g., the methods can be performed with oral or parenteral routes of administration). In certain embodiments, the glutamate modulating agent is administered orally (e.g., as a tablet or capsule). In other embodiments, the glutamate modulating agent is administered parenterally (e.g., intravenously, intramuscularly or subcutaneously, whether by injection or infusion).

In some embodiments, the subjects are not administered agents that affect memantine plasma levels, such as triamterene, hydrochlorothiazide, metformin, cimetidine, ranitidine, quinidine, nicotine, carbonic anhydrase inhibitors, or sodium bicarbonate.

### EXAMPLES

The invention is further described in the following examples, which do not limit the scope of the invention described in the claims.

### METHODS

The following methods were used in the Examples set forth below.

### Subjects

Participants were recruited from ambulatory care referrals to a general child and adolescent psychiatry clinic and a specialized ASD clinic at a university hospital from January 2015 to July 2018. This study was conducted in compliance with the principles of the Good Clinical Practice guideline and was approved by the Institutional Review Board of Partners Healthcare, which oversees human-subject research conducted by Massachusetts General Hospital (MGH) and McLean Hospital. Written informed consent was provided prior to participation by the parent or legal guardian of each participant. Participants aged <14 years provided written informed assent and ≥14 years provided written informed consent.

### Trial Design

This study was a 12-week, randomized, double-blind, placebo-controlled, parallel group clinical trial of memantine in youth with ASD. Youth with ASD who met the eligibility criteria were randomized to receive either memantine or placebo during the course of the 12-week trial. Participants underwent multimodal MRI scans prior to and upon completion of the 12-week trial. Participants were assessed weekly during the titration phase (baseline to Week 4) and at midpoint (Week 6), Week 9, and completion (Week 12) during the maintenance phase. At each study visit, safety and efficacy were assessed by administering measures of efficacy (CGI, GAF), tolerability (assessing treatment-emergent AEs), and safety (vital signs, including weight). At baseline, midpoint, and endpoint (completion/early termination) severity of autism was assessed by administering measures of autism severity (SRS-2, ABC-SW, CY-BOCS-PDD) and associated psychopathology (ADHD-RS, CDRS-R, CASI-ANX).

### Eligibility Criteria for Participants

Eligible participants were of both sexes, aged 8 to 17 years, with intact intellectual ability, as established by a full-scale intelligence quotient of 85 or greater based on the Vocabulary and Matrix Reasoning subtests of the Wechsler Abbreviated Scale of Intelligence-Second Edition (WASI-II (Wechsler, 2011)). Participants were required to meet DSM-IV-TR/5 diagnostic criteria for autism, as established by clinical diagnostic evaluation and to have at least moderate severity of ASD, as determined by a total raw score of 85 or greater on the informant-rated Social Responsiveness Scale-Second Edition (SRS-2; (Constantino et al., 2003)) and a score of 4 or greater on the clinician-rated National Institute of Mental Health (NIMH) Clinical Global Impression (CGI) severity scale (CGI-S; (National Institute of Mental Health, 1985)).

Youth were excluded from participation if they reported a history of treatment with memantine or current treatment with glutamate-modulating agents, such as lamotrigine, amantadine, N-acetylcysteine, and D-cycloserine, or agents that affect memantine plasma levels, such as triamterene, hydrochlorothiazide, metformin, cimetidine, ranitidine, quinidine, nicotine, carbonic anhydrase inhibitors, or sodium bicarbonate.

### Screening Assessments

Eligible subjects were administered a comprehensive assessment battery consisting of diagnostic, neuropsychological, and physical assessment measures. In addition to the clinical diagnostic evaluation, a systematic assessment of autism was conducted through the administration of the Autism Diagnostic Interview-Revised (ADI-R) and the Autism Diagnostic Observation Schedule-Second Edition (ADOS-2). Associated psychopathology was assessed using the Kiddie-Schedule for Affective Disorders and Schizophrenia-Epidemiologic Version (K-SADS-E). Full-scale intelligence quotient was assessed on a battery of select tasks from the WASI-II and the Wechsler Intelligence Scale for Children (WISC-IV; Wechsler, 2003) or the Wechsler Adult Intelligence Scale (WAIS-IV; Wechsler, 2008). Physical examination included height, body weight, waist circumference, and Tanner staging on the Petersen Pubertal Development Scale to establish the stage of sexual maturity. Physical assessment included standard battery of blood tests, an electrocardiogram, a urine drug test, and a urine pregnancy test for women of child-bearing potential.

### Study Intervention and Randomization

In this 12-week, randomized, double-blind, placebo-controlled, parallel group clinical, ASD participants were randomized in a 1:1 ratio to receive memantine or placebo. An independent statistician created a computer-generated randomization schedule, using stratified randomization by sex (male vs. female) and race (Caucasian vs. Non-Caucasian). The Clinical Trials Pharmacy at MGH dispensed the study medication (memantine and placebo) based on the randomization schedule. The randomization schedule remained confidential throughout the study.

Study medication was titrated to the maximum daily dose during the first 4 weeks of the trial (dose titration phase) and from there on, subjects were maintained on maximum achieved dose until the end of the trial (dose maintenance phase). Titration of the study medication was guided by a flexible titration schedule which allowed for slower titration or maintaining at a lower dose, as guided by study clinician judgment of tolerability. Study medication was administered in divided dosages. Study medication was initiated at 2.5 mg/day (raised to 5 mg/day on day 4) and was up-titrated by 5 mg/week to a maximum dose of 20 mg/day.

Medication compliance was assessed via caretaker report at each study visit. A count of the returned study medication was verified against caretaker report and those who regularly missed doses were counseled to improve the medication compliance.

### Primary Outcome Measure

The efficacy of memantine for the treatment of autism related impairments was assessed by the informant-rated Social Responsiveness Scale - Second Edition (SRS-2) as continuous outcome measure, and by the clinician-rated CGI-I subscale, as categorical outcome measure. Treatment response criteria for this trial was defined as ≥25% improvement on the SRS-2 and a score of 2 or 1 on the CGI-I subscale, i.e., "much" or "very much improved."

### Secondary Outcomes Measures

Additional treatment response of autism impairment was examined for the ASD subdomains by assessing change with treatment in the severity of the SRS-2 subscales and the Children's Yale Brown Obsessive Compulsive Scale modified for PDD (CY-BOCS-PDD) (Goodman et al., 1986 (Rev 89); Scahill et al., 2006) to assess change with treatment in the ritualistic behaviors associated with ASD. Treatment response of frequently associated psychopathologies with ASD was assessed by administering the clinician-rated Attention Deficit Hyperactivity Disorder Rating Scale (ADHD-RS) ( DuPaul, Power, Anastopoulos, & Reed, 1998), Children's Depression Rating Scale-Revised (CDRS-R)(Poznanski et al., 1985), and CASI-4 ASD Anxiety Scale (CASI-4 ASD Anxiety Scale (CASI-Anx)(Gadow et al., 2002). Change with treatment in the level of global functioning was assessed by the clinician-rated GAF.

Glutamate response to memantine therapy in the PgACC was assessed by acquiring spectroscopic MRI pre- and post-treatment.

### Safety and Tolerability Assessments

Safety and tolerability of memantine was monitored by administering a complete physical examination, battery of blood tests (complete blood count, electrolytes, liver function test, thyroid function test, and random glucose levels), Urine tests (drug screen and pregnancy screen [women of child-bearing potential only]), and EKG at baseline and endpoint of the trial, and by recording treatment-emergent AEs and obtaining vital signs (pulse, blood pressure, weight) at each study visit.

### Neuroimaging Methods

Healthy Control Participants were enrolled for spectroscopic neuro-imaging data acquisition to form a control group. Healthy controls (HC) were age-, sex-, and IQ-matched with enrolled ASD participants. HC participants were with no significant autistic traits, as screened by SRS-2 raw score of <60, and with no major psychopathology, as established by the K-SADS-E and confirmed by clinical diagnostic interview.

*Data Acquisition-Proton* spectra were acquired at 4T using a two-dimensional J-resolved (2D-JPRESS) ¹HMRS protocol. A 2D-JPRESS sequence was chosen to improve reliability for spectral fitting of the metabolites(Gonenc et al., 2010; Moore et al., 2007), allow analysis of glutamate alone versus ratios or combined measures (e.g., Glx), and confirm that measured metabolic differences truly arise from differences in metabolic levels and not from T2 relaxation time differences. An 8 cc (2 x 2 x 2 cm) single voxel was placed in the *Pg*ACC along the midline such that the inferior edge of the voxel was parallel to the descending surface of the corpus callosum, as determined by longitudinal relaxation time (T1) images used also for tissue segmentation. Data were collected in 12 TE- stepped spectra with the echo-time ranging from 30 to 250 ms in 20 ms increments, with TR = 2s, averages = 16, scan duration = 7 minutes.

*Data Processing:* Spectral analysis was conducted in a fully automated fashion using the commercially available LCModel package (version 6.2-1F). After the 2D-JPRESS dataset was resolved into a series of one-dimensional spectra where each spectrum was modeled and fitted with GAMMA simulated⁹⁶ J-resolved basis sets, glutamate activity levels were derived from the total integral across the J-series. Glutamate transverse relaxation time (T2) was obtained from the raw peak vs. TE decay curve fitted with Levenberg-Marquardt algorithm using an exponential decay function (for glutamate T2) convoluted with a polynomial function (for glutamate J-coupling) which was obtained from the GAMMA simulation run with previously measured spectral parameters (Govindaraju, Young, & Maudsley, 2000). Data quality was assessed by LCModel calculated Full Width Half Maximum (FWHM), Signal to Noise Ratio (SNR) and Cramer-Rao Lower Bounds (CRLB).

### Sample Size Determination

Based on our pilot study where four out of seven participants were responders to 15-20 mg daily of memantine (Joshi et al., 2016), we estimated that 57% of participants on memantine would respond to treatment. In the RUPP Autism Network study of risperidone (RUPP, 2002), response rates were 69.4% and 11.5% in the treatment (memantine) and placebo groups, respectively. Therefore, we based our sample size calculations on response rates of 57% in the memantine group and 11.5% in the placebo group. We determined *a priori* that we would need a sample size of 40 participants in total (20 participants per group) to detect this difference in response rate at a 2-sided significance level of .05 with 80% power. Our final sample consisted of 16 participants in the memantine group and 19 participants in the placebo group with response data. With this sample size, we had 84% power to detect the aforementioned difference in response rates.

### Statistical Methods

The primary outcome was analyzed using Pearson's chi-square test at the *a priori* defined significance level of 0.05. Secondary measures of efficacy were analyzed using mixed-effects regression models with robust standard errors to account for the repeated measures on each subject. The primary and secondary measures were compared between memantine versus placebo as well as between those with high versus normal baseline glutamate activity levels. Participants were categorized as having high versus normal glutamate activity levels at baseline using z-scores. The population mean and SD for z-score calculations were derived from healthy control glutamate activity levels (baseline and endpoint). Glutamate activity levels with z-scores ≥1 were categorized as high glutamate activity levels, those with z-scores <1 and >-1 were categorized as normal glutamate activity levels and those with z-scores ≤-1 were categorized as low glutamate activity levels. Changes in PgACC glutamate activity levels within groups were tested using paired t-tests and changes between groups were tested using two-sample t-tests. We also performed ROC curve analyses to determine the predictive utility of baseline PgACC glutamate activity levels in identifying treatment responders within the memantine and placebo groups. All analyses apart from the analysis of the primary outcome were considered exploratory. Analyses were performed using a modified intent-to-treat analysis set which included all randomized patients who were regarded exposed to study medication (completed at least two weeks of treatment) and had at least one follow-up assessment.

The mixed-effects regression models used to analyze the secondary measures of efficacy predicted the outcome measure from treatment group (memantine versus placebo), study visit (continuous), and the treatment group-by-study visit interaction, which is our test of efficacy. To examine the effect of baseline PgACC glutamate activity level on the response to treatment, we predicted outcomes measures from treatment group, study visit (continuous), PgACC glutamate activity level (continuous), treatment group-by-study visit interaction, glutamate activity level-by-study visit interaction, treatment group-by-glutamate activity level interaction, and treatment group-by-glutamate activity level-by-study visit interaction. All mixed-effects regression models had random intercepts by subject ID, used robust standard errors to account for the repeated measures on each subject, and used an independent covariance structure for the random effects. Our method of analysis did not impute data but rather used all available longitudinal data for participants in the analysis set provided they had data at baseline and at least one follow-up visit. All missing data were assumed to be missing at random.

We performed receiver operating characteristic (ROC) curve analysis to examine the ability of baseline PgACC glutamate activity levels to identify those who did and did not respond to treatment. We performed separate ROC curve analyses in the memantine and placebo groups and compared their area under the curve (AUC) statistics. ROC analysis uses each value across the entire range of baseline PgACC glutamate activity levels as the cutoff for defining a treatment responder and compares this classification to the "true" response classification, as defined by a ≥20% reduction in SRS Total raw score and CGI-I ≤2. The ROC analysis then plots the false positive rate (1-specificity) and the true positive rate (sensitivity) for each PgACC glutamate activity level on the x- and y-axis, respectively, to create the ROC curve. ROC analysis summarizes predictive utility with the area under the curve (AUC) statistic. An AUC of 0.5 means baseline PgACC glutamate activity levels do not predict treatment response in any way and an AUC of 1.0 means baseline PgACC glutamate activity levels predict the treatment response perfectly. Based on the information from the ROC curve analysis, we used the Liu approach to calculate an optimal cut-point to identify those subjects who did and did not respond to treatment (Liu, Stat Med. 2012 Oct 15;31(23):2676-86) in both the memantine and placebo groups. This approach defines the optimal cut-point as the point where the product of the sensitivity and specificity is maximized. We used conditional probabilities to examine the predictive utility of these cutoff points. For each cutoff, we calculated sensitivity, specificity, the positive predictive value (PPV), negative predictive value (NPV), and the percent correctly classified.

Effect sizes are presented as standardized mean differences (SMD) for continuous outcomes and odds ratios (OR) for dichotomous outcomes. The SMDs for the difference of the changes in scores over time between memantine and placebo and between high glutamate and normal glutamate were calculated as Cohen's *d* using the following calculations: the difference in the change scores between the two groups divided by the pooled standard deviation. The SMDs for changes in scores within groups were calculated as Cohen's d using the following calculation: difference in score from baseline to endpoint divided by the pooled standard deviation. For comparisons of memantine versus placebo and high glutamate versus normal glutamate, positive SMDs and ORs>1 indicate better improvement in the memantine group and high glutamate group, respectively. For comparisons of ASD versus healthy controls, positive SMDs indicate lower glutamate activity levels in the ASD group. As defined by Cohen (Statistical Power Analysis for the Behavioral Sciences. 2nd ed. Hillsdale, NJ: Erlbaum. 1988), a SMD=0.2 is interpreted as a small effect size, SMD=0.5 as medium, and SMD=0.8 as large. We considered ORs>2 to be clinically meaningful.

Safety results were summarized descriptively by treatment group and baseline glutamate activity level (high versus normal) and compared at the end of the study using Fisher's exact or Pearson's chi-square tests. All analyses were performed using Stata (Version 16) (StataCorp, Stata Statistical Software: Release 16. College Station, TX: StataCorp LLC. 2019).

### Example 1. Study Population

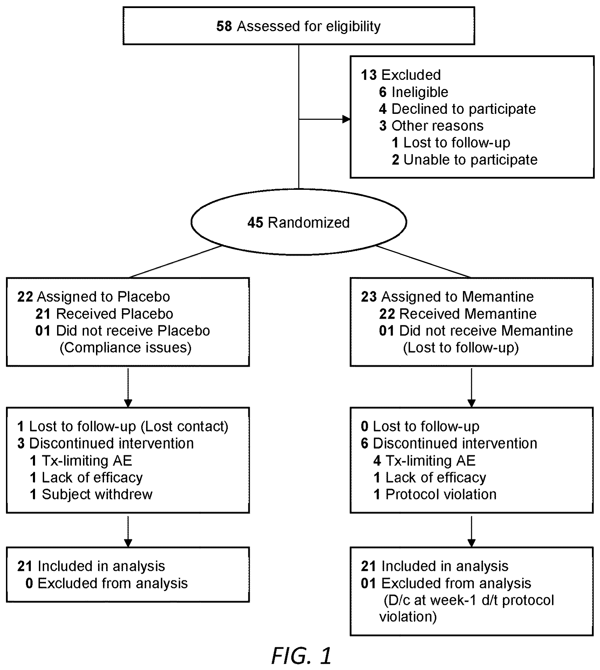

As shown in Fig. 1, 58 participants were assessed for eligibility and 45 were randomized to treatment with either memantine (23 participants) or placebo (22 participants). The major reasons for exclusion prior to randomization were ineligibility (7 [12.1%]) and refusal to participate (4 [6.9%]). Twenty-two participants received memantine and 21 participants received placebo as one was lost to follow-up and one had compliance issues, respectively. One participant in the memantine treatment group was removed in the first week of the trial due to protocol violation (started exclusionary medication). Thus, 21 participants per group were included in analysis. Of those who received treatment, 16 memantine and 17 placebo participants completed the trial.

### Participant Characterization

Participant characteristics are given in Table B. The participants receiving memantine and placebo showed similar age distribution (mean [SD], 13.1 [2.8] vs. 13.3 [2.5] years), sex distribution (males, 16 [76%] vs. 16 [76%]; females, 5 [24%] vs. 5 [24%]), race/ethnicity (Caucasian, 19 [90%] vs. 20 [95%]), and other characteristics pertaining to neurocognitive, autism, psychological, functional, and psychopharmacotherapy profiles.

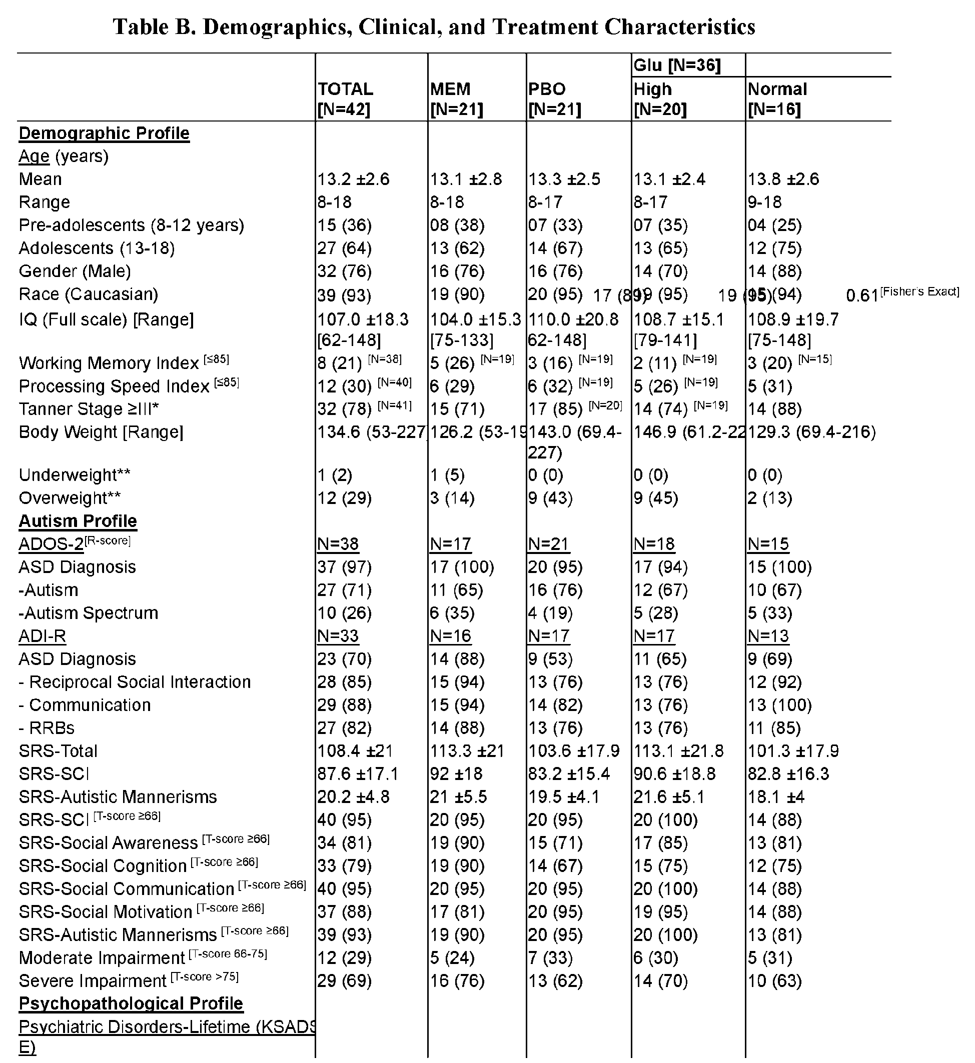

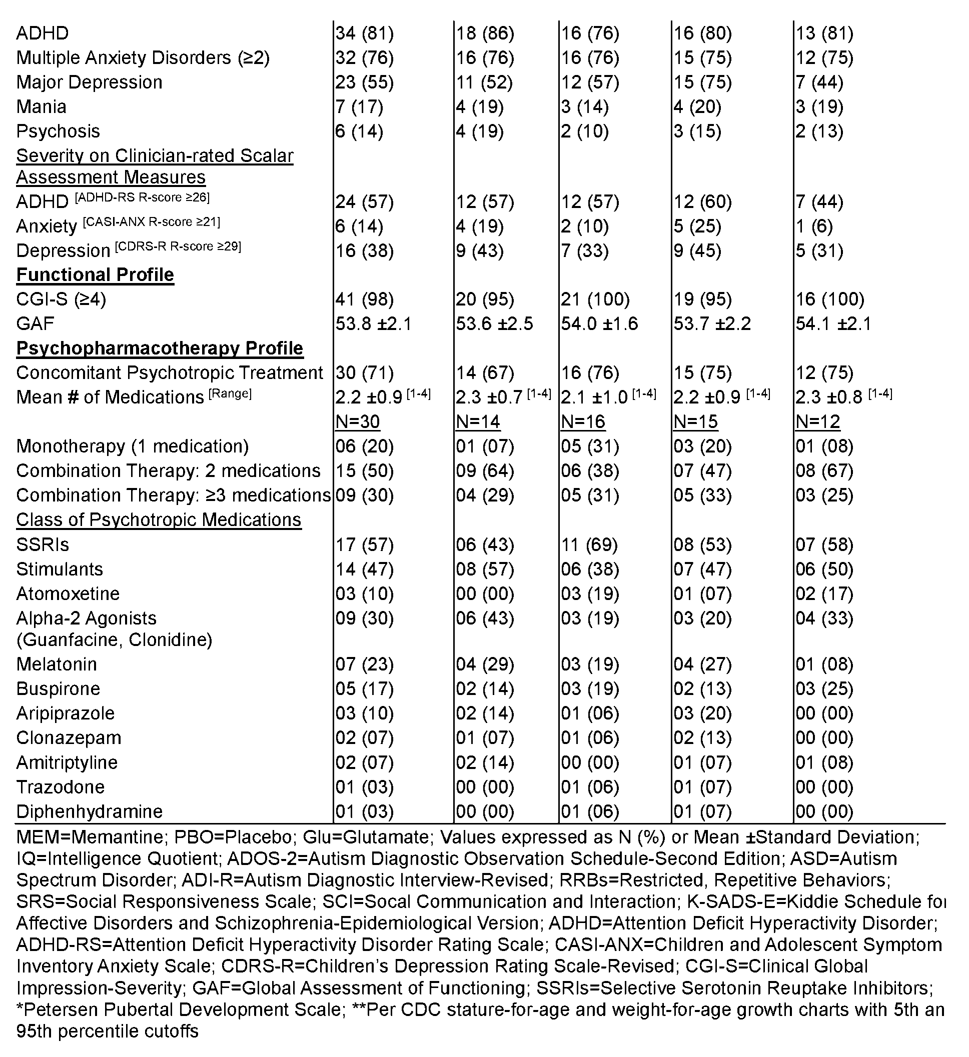

### Treatment Fidelity

Excluding participants who were lost to follow-up (N=1), the study visit fidelity of ASD participants was excellent with mean (SD) ratings of 100.0 (0.0). Study medication treatment compliance of participants who reached midpoint was excellent with mean (SD) ratings of 98.5 (4.9). Study medication treatment compliance was calculated based on return of study medication.

### Example 2. Efficacy

### Primary Outcomes:

Analysis of our primary outcome, the percent of participants who responded to treatment as defined by ≥25% decrease in SRS total score and CGI-I score ≤2, included 16 memantine and 19 placebo participants. Seven participants were excluded because they only had baseline SRS data. The rate of treatment response was significantly higher in the memantine group compared to the placebo group (9 [56%] vs. 4 [21%]; OR=4.82, 95% CI 1.10 to 21.19; P=.03) (Table 1).

### Secondary Outcomes:

As with the primary outcome, secondary outcomes were analyzed for 16 memantine and 19 placebo participants due to seven participants only having baseline data available. Of the seven SRS scales, ABC Social Withdrawal scale, four scalar measures for psychopathology, the only significant differences between memantine and placebo were on the SRS Social Cognition and SRS Social Awareness scales (Table 1). Those in the memantine group showed greater improvement from baseline to endpoint compared to placebo group on the SRS Social Cognition (SMD=0.83; 95% CI 0.13 to 1.52; P=.02) and SRS Social Awareness (SMD=0.74 95% CI 0.05 to 1.42; P=.04) sub-scales.

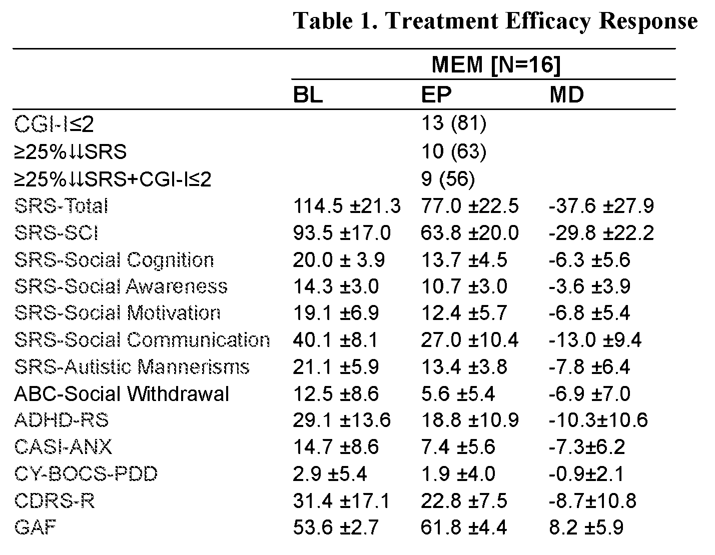

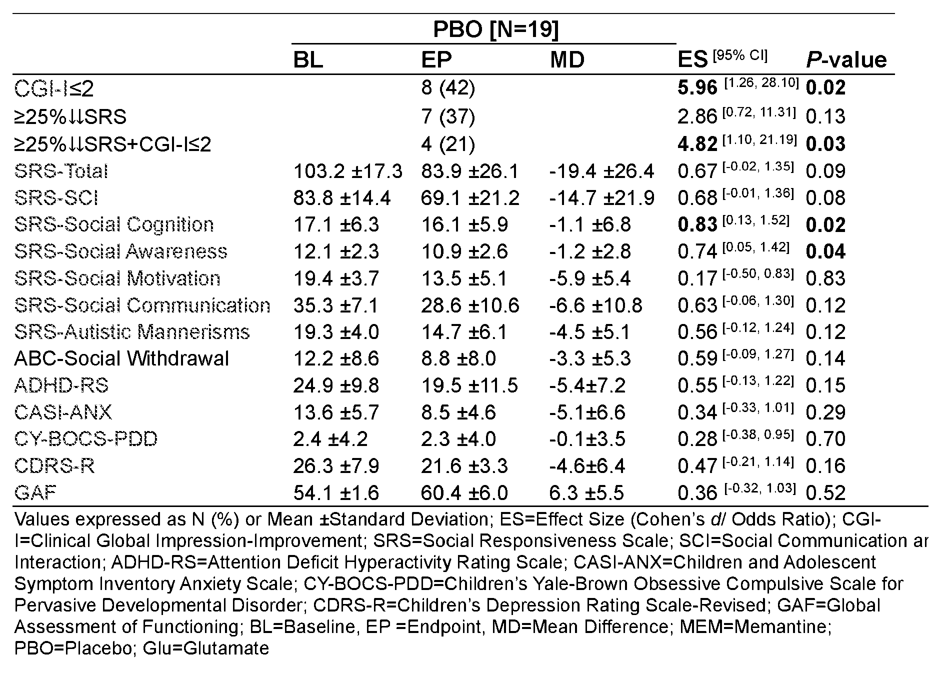

### Example 3. Safety and Tolerability

As shown in Table 2, those in the memantine and placebo groups had a similar dose distribution (mean [SD] 19.7 [1.1] vs. 19.0 [3.0], P = .35) and the majority of participants reached 20 mg/day (18 [95%] vs. 19 [90%], P >0.99). There were no significant differences between memantine and placebo in the number of adverse events (AEs) reported (2.2 [1.2] vs. 2.1 [1.5], P = .87) or the percent of participants reporting AEs (14 [67%] vs. 9 [43%], P = 0.12). The most commonly reported AEs were headaches (5 [24%] vs. 2 [10%], P = 0.41) and insomnia (3 [14%] vs. 2 [10%], P>.99) (Table 2). Two participants in the memantine group reported severe AEs (headache [5%] and worsening irritability and agitation [5%]). There were no differences between memantine and placebo in the percent of participants requiring rescue medication (4 [19%] vs. 4 [19%], P>0.99). Four participants terminated memantine treatment due AE of worsening anxiety (2 in the 1^{st} week and 2 in the 4^{th} week) and one participant terminated placebo treatment in the second week d/t AE of worsening irritability.

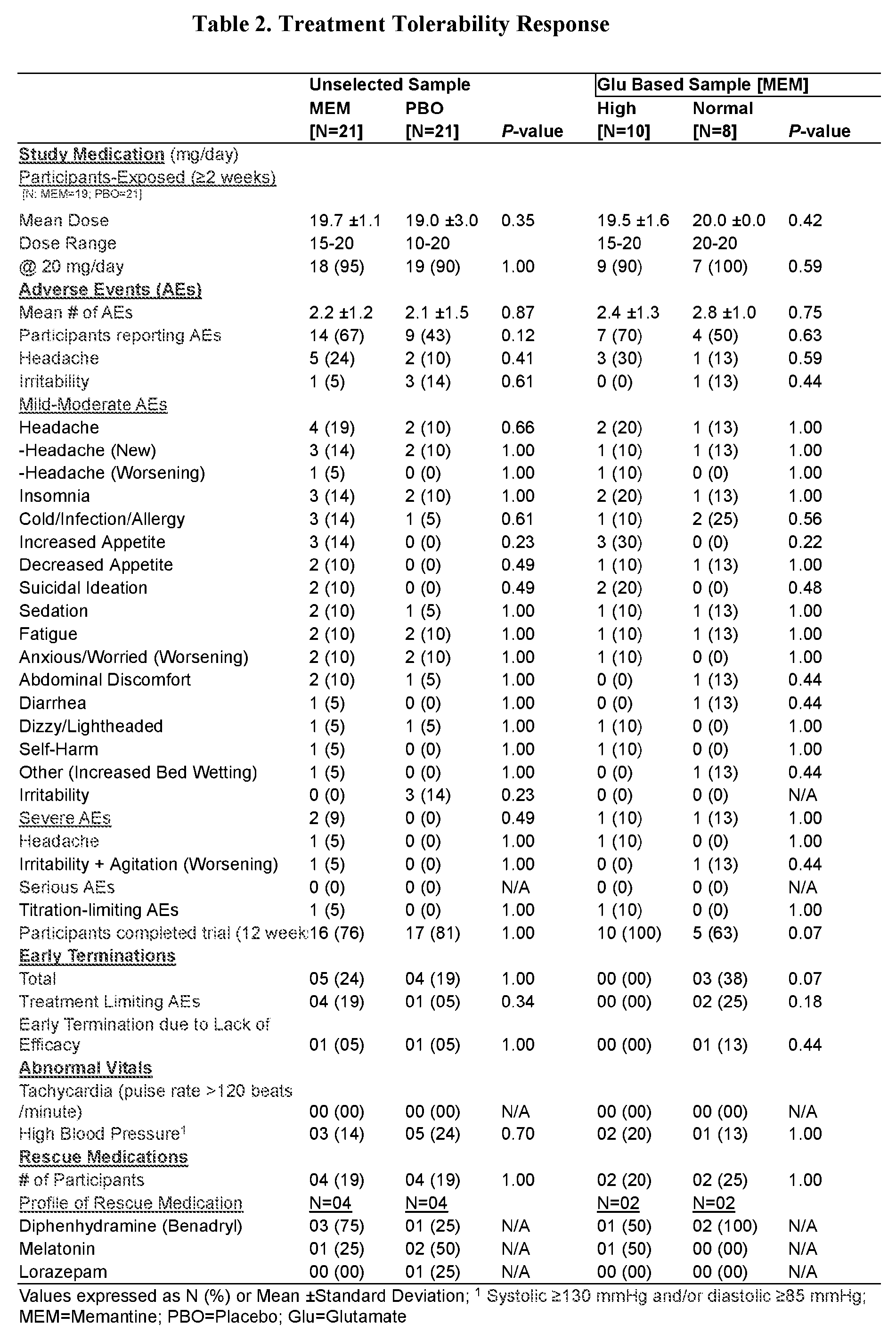

### Example 4. MRSpectroscopic PgACC Glutamate Activity based Response

Baseline spectroscopic *Pg*ACC glutamate data was collected for 37 ASD and 16 HC participants. Of those, endpoint spectroscopic data was collected for 31 ASD and 13 HC participants.

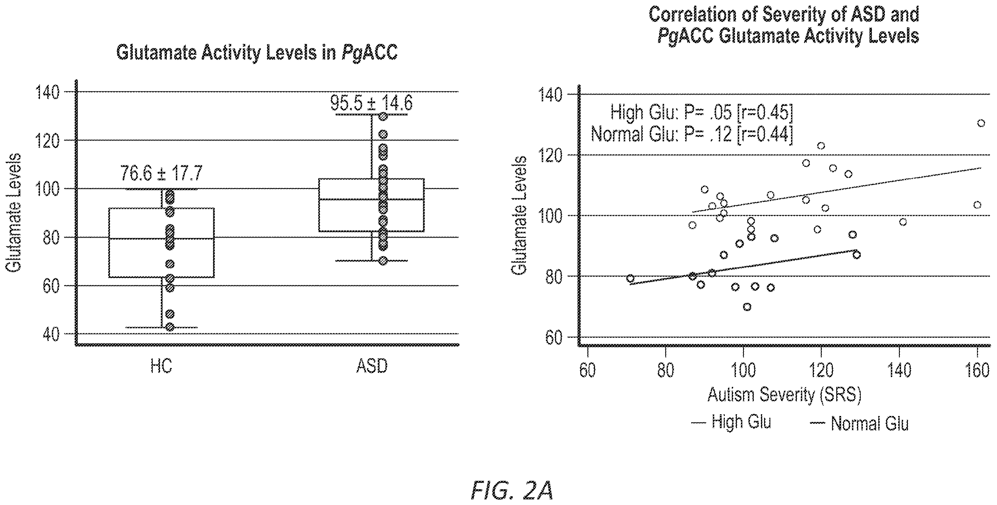

Baseline *Pg*ACC Glutamate Activity in ASD: The PgACC glutamate activity at baseline was significantly higher in ASD as compared to the HCs (95.5 ±14.6 vs. 76.6 ±17.7; MD=-18.8 ±33.9; SMD=-1.21, 95% CI -1.84 to -0.57; *P*<.001). In addition, there was significantly strong positive correlation between the levels of pgACC Glu activity and the severity of autism in the ASD participants. *See* Fig. 2A-2B.

Glutamate Dysregulation Subtype of ASD: For z-score calculations the mean ±SD value of 76.5 ±18.0 were derived from HCs baseline (N=16) and endpoint (N=13) glutamate activity levels. Based on the z-scores high *Pg*ACC glutamate activity was observed in 54% of the ASD participants and none of the ASD participant had low *Pg*ACC glutamate activity.

Stability of *Pg*ACC Glutamate Activity: The mean glutamate activity levels in the HC remained relatively unchanged in 12 weeks period (baseline=75.7 ±18.3, endpoint=76.2 ±19.2; MD=0.5 ±1.7; SMD=0.02, 95% CI -0.71 to 0.75; *P*=.28).

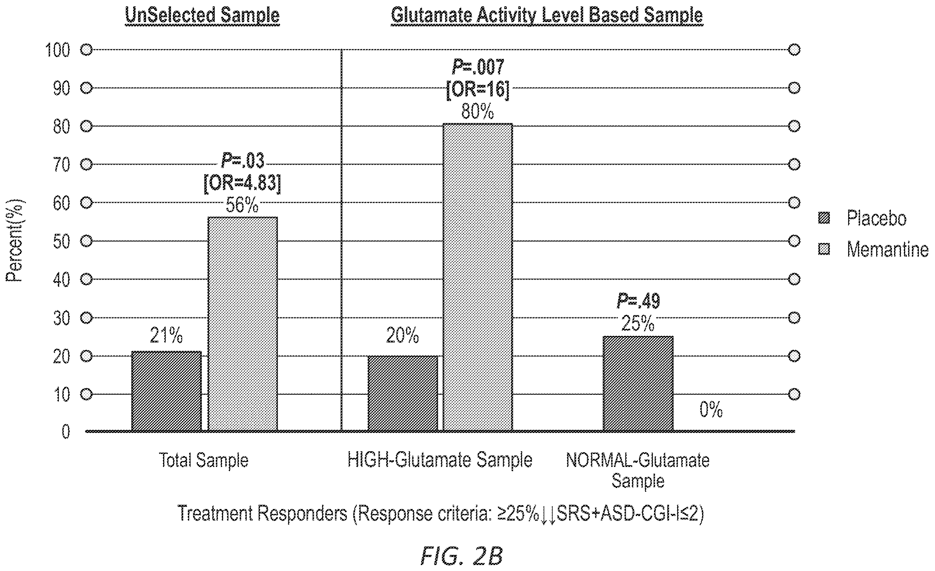

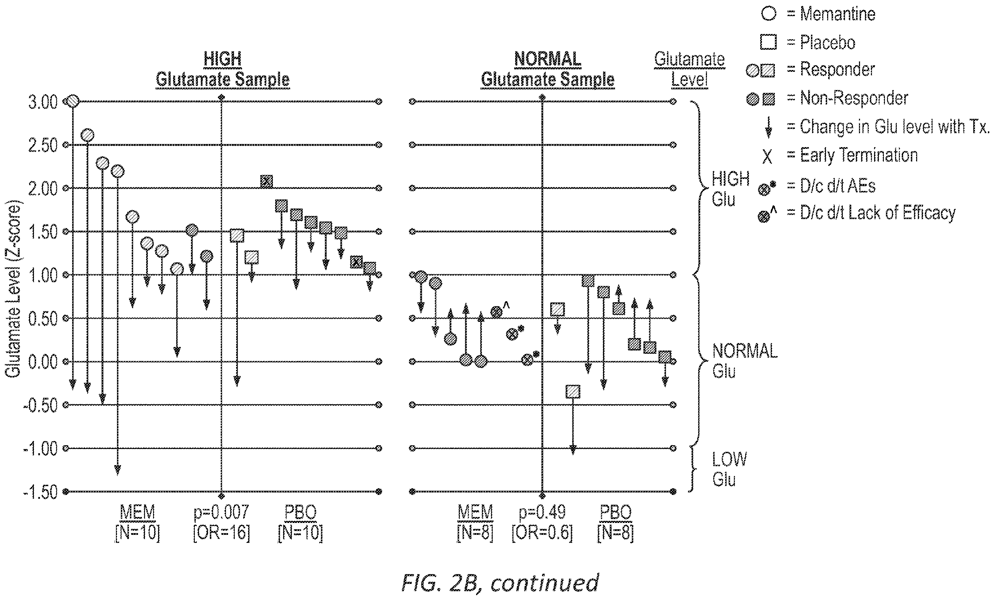

*Pg*ACC Glutamate Response to Tx: Table 3A presents within group changes in *Pg*ACC glutamate activity levels from baseline to endpoint in the ASD group as a whole and stratified by treatment group (memantine vs. placebo), treatment response (responder vs. non-responder), and baseline *Pg*ACC glutamate activity (high vs. normal). In unselected sample of ASD significant reduction with treatment in the *Pg*ACC glutamate activity was observed in the memantine and placebo treated groups, treatment responders group, and with memantine responder groups. In high glutamate sample there was significant reduction with treatment in the *Pg*ACC glutamate activity in both memantine and placebo treated groups, thought the magnitude of glutamate reduction was significantly higher with memantine versus placebo treated groups. In the normal-glutamate sample the *Pg*ACC glutamate activity did not change significantly with treatments (memantine, placebo, memantine and placebo non-responders). See Fig. 2B. Table 3A further compares baseline PgACC glutamate activity levels at baseline and at endpoint between the HC group and different ASD groups. In the unselected ASD sample, the baseline *Pg*ACC glutamate activity was significantly higher than HCs in all ASD groups by treatment exposure or responder status except for memantine non-responder group were glutamate activity was not higher than HCs. However, posttreatment *Pg*ACC glutamate activity in the unselected sample of ASD was not significantly different from HCs apart from the non-responder group with significantly higher glutamate activity than HCs. In the high glutamate sample, the PgACC glutamate activity with treatment was not significantly different from HCs for the memantine group and memantine responder group whereas the glutamate activity in the placebo group and placebo non-responder group was significantly higher than HCs. In the normal-glutamate sample there was no significant difference in the glutamate activity between different groups of ASD and HCs.

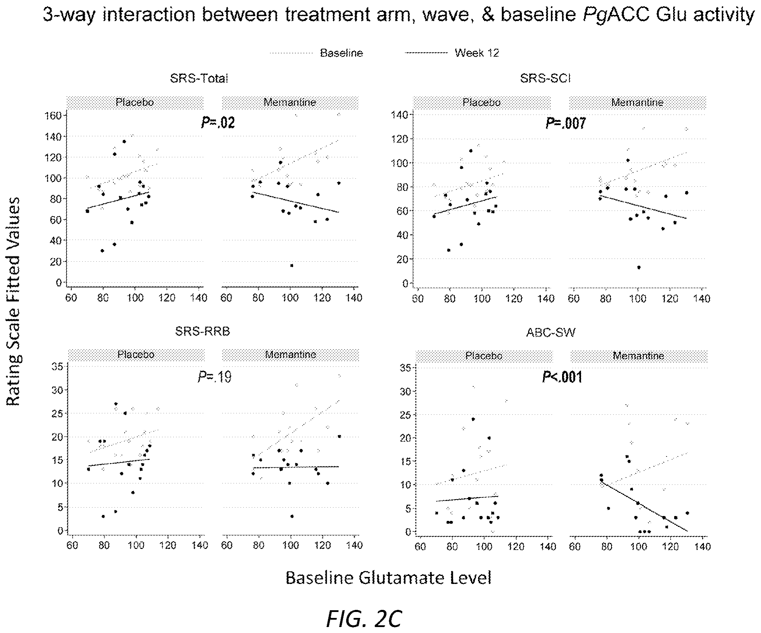

Tx. Response Based on Glutamate Dysregulation in ASD: *Moderating Effect of PgACC Glutamate activity levels:* As shown in Figure 2C, there were significant three-way interactions between *Pg*ACC glutamate activity level, treatment group, and study visit when predicting SRS Total (P = .02), SRS-SCI (P=.007), and ABC-Social Withdrawal (P<.001) scores but not SRS-Autistic Mannerisms scores (P=.19) (Fig. 2C, Table 3B). Higher *Pg*ACC glutamate activity levels were associated with greater change scores in the memantine group but not in the placebo group.

### Example 5. Predicative Utility of PgACC Glutamate activity levels

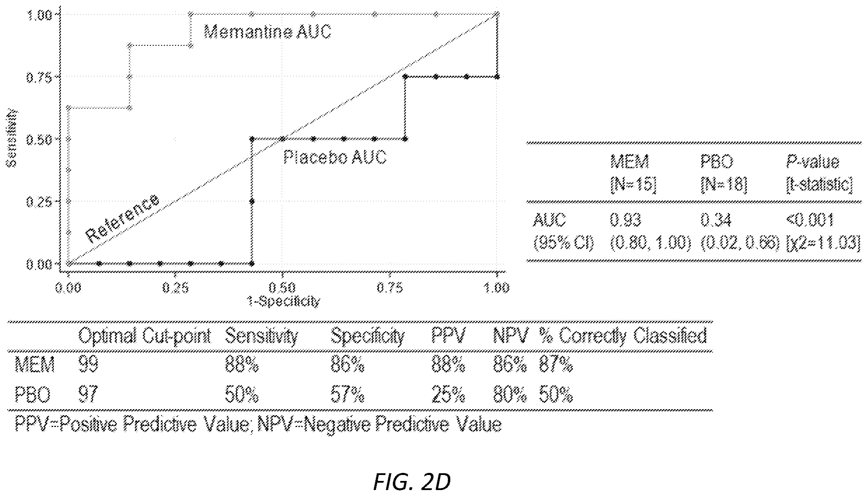

As shown in Figure 2D, there was a significant difference in the area under the curve when comparing the predictive utility of baseline glutamate between the memantine and placebo groups (AUC statistic [95% CI]: 0.93 [0.80, 1.00] vs. 0.34 [0.02, 0.66], *P*<.001). Baseline *Pg*ACC glutamate activity levels had excellent predictive utility in the memantine group and extremely poor predictive utility in the placebo group. This striking difference suggests predicting therapeutic response and not placebo response. This study identified an optimal baseline *Pg*ACC glutamate cut-point of 99 in the memantine group and 97 in the placebo group (Fig. 2D).

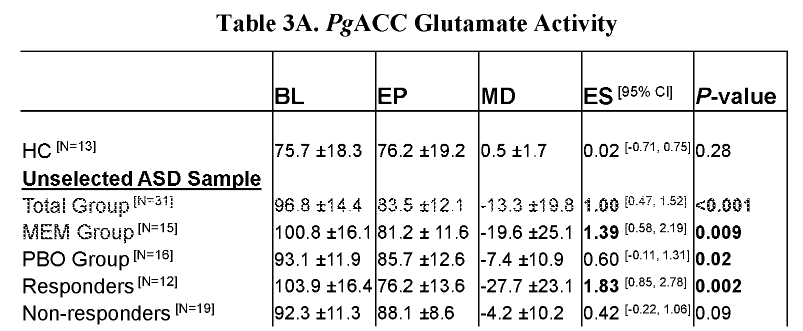

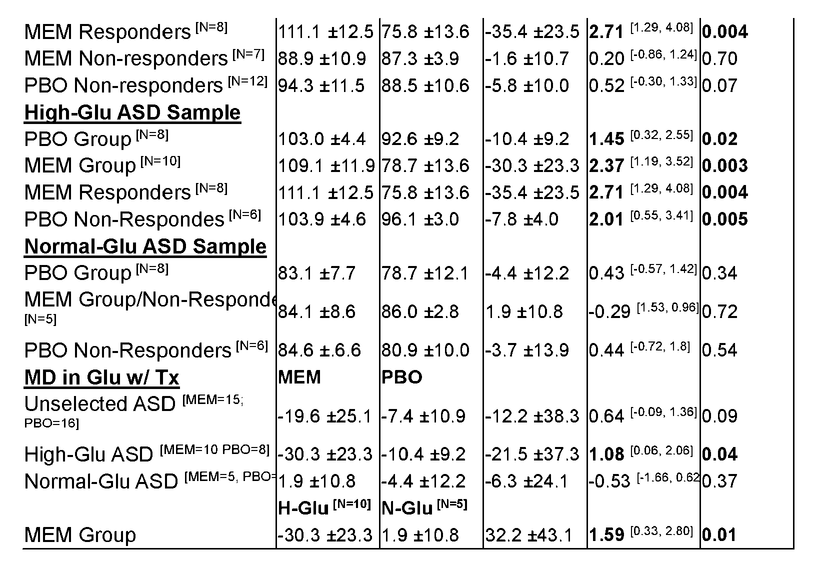

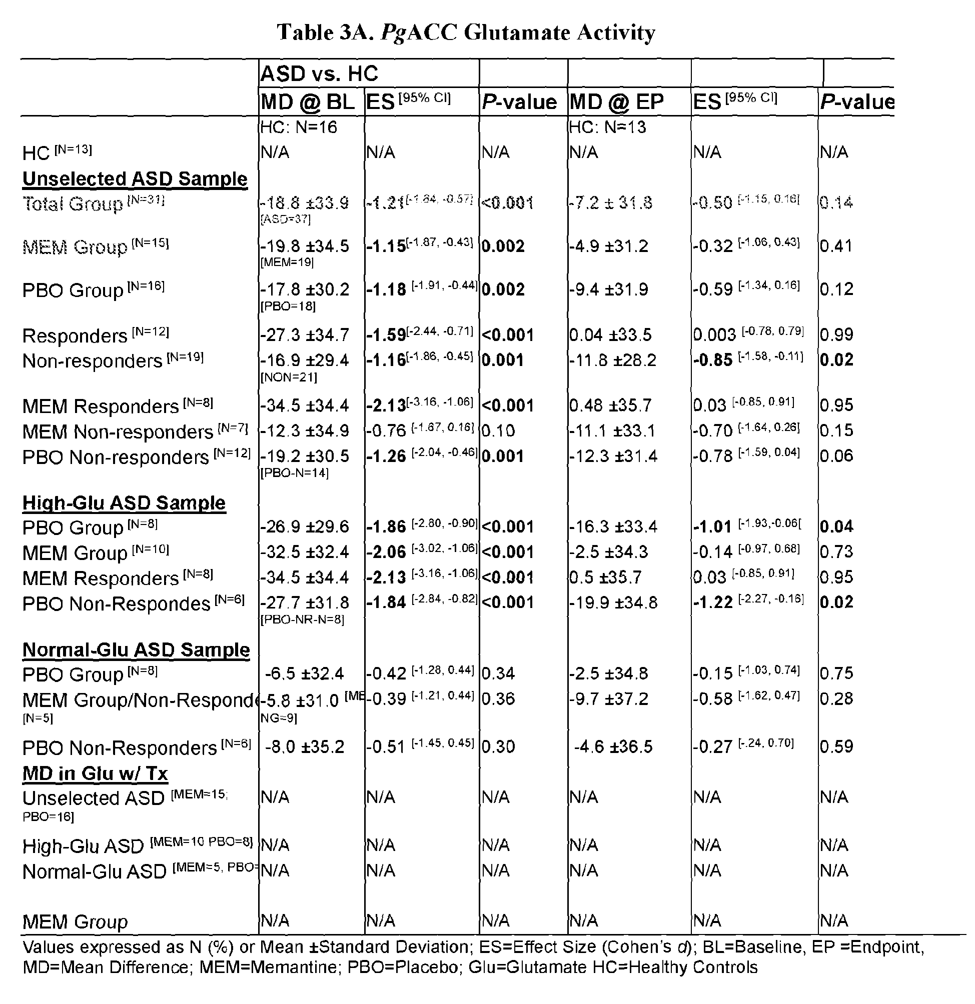

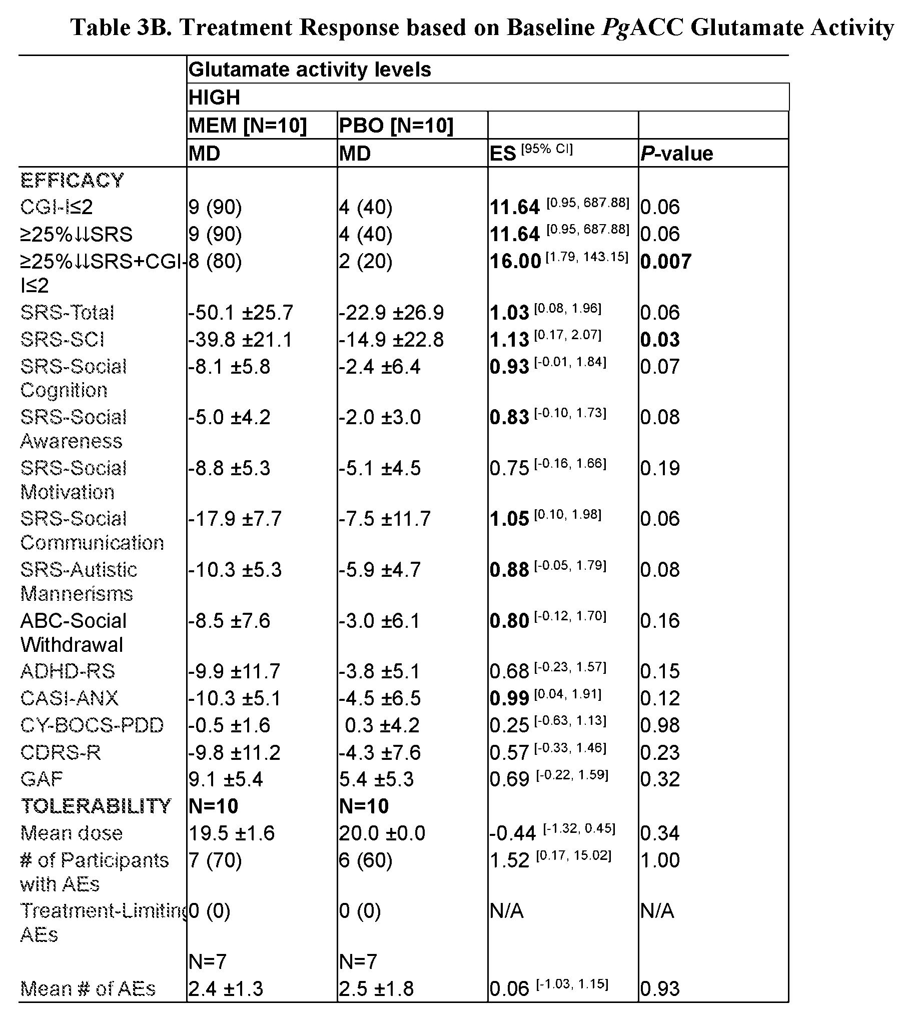

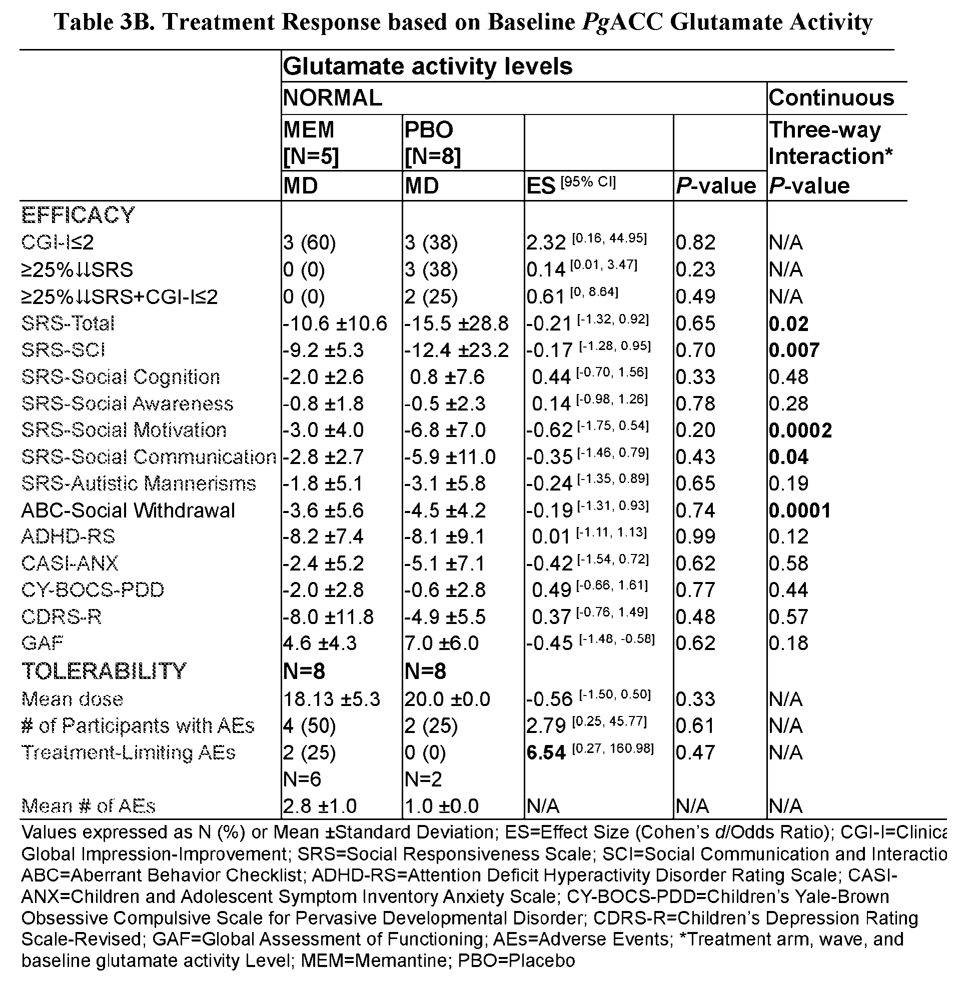

### References

Aman, M. G., Findling, R. L., Hardan, A. Y., Hendren, R. L., Melmed, R. D., Kehinde-Nelson, O., . . . Katz, E. (2017). Safety and Efficacy of Memantine in Children with Autism: Randomized, Placebo-Controlled Study and Open-Label Extension. J Child Adolesc Psychopharmacol, 27(5), 403-412. doi:10.1089/cap.2015.0146
Bauman, M., & Kemper, T. L. (1985). Histoanatomic observations of the brain in early infantile autism. Neurology, 35(6), 866-874.
Bejjani, A., O'Neill, J., Kim, J. A., Frew, A. J., Yee, V. W., Ly, R., ... Levitt, J. G. (2012). Elevated glutamatergic compounds in pregenual anterior cingulate in pediatric autism spectrum disorder demonstrated by 1H MRS and 1H MRSI. PLoS One, 7(7), e38786. doi:10.1371/journal.pone.0038786
Belsito, K. M., Law, P. A., Kirk, K. S., Landa, R. J., & Zimmerman, A. W. (2001). Lamotrigine therapy for autistic disorder: a randomized, double-blind, placebo-controlled trial. J Autism Dev Disord, 31(2), 175-181.
Bernardi, S., Anagnostou, E., Shen, J., Kolevzon, A., Buxbaum, J. D., Hollander, E., . . . Fan, J. (2011). In vivo 1H-magnetic resonance spectroscopy study of the attentional networks in autism. Brain Res, 1380, 198-205. doi:10.1016/j.brainres.2010.12.057 Blue, M. E., Naidu, S., & Johnston, M. V. (1999). Altered development of glutamate and GABA receptors in the basal ganglia of girls with Rett syndrome. Exp Neurol, 156(2), 345-352.
Blumberg, S. J., Bramlett, M. D., Kogan, M. D., Schieve, L. A., Jones, J. R., & Lu, M. C. (2013). Changes in prevalence of parent-reported autism spectrum disorder in school-aged U.S. children: 2007 to 2011-2012. Natl Health Stat Report(65), 1-11, 11 p following 11.
Bogner, W., Gruber, S., Trattnig, S., & Chmelik, M. (2012). High-resolution mapping of human brain metabolites by free induction decay (1)H MRSI at 7 T. NMR Biomed, 25(6), 873-882. doi:10.1002/nbm.1805
Campbell, M., Adams, P., Small, A. M., Curren, E. L., Overall, J. E., Anderson, L. T., .. . Perry, R. (1988). Efficacy and safety of fenfluramine in autistic children. Journal of the American Academy of Child and Adolescent Psychiatry, 27(4), 434-439.
Campbell, M., Anderson, L., Small, A., Adams, P., & Gonzalez, N. (1993). Naltrexone in autistic children: Behavioral symptoms and attentional learning. Journal of the American Academy of Child and Adolescent Psychiatry, 32(6), 1283-1291.
Carlsson, M. L. (1998). Hypothesis: is infantile autism a hypoglutamatergic disorder? Relevance of glutamate - serotonin interactions for pharmacotherapy. J Neural Transm, 105(4-5), 525-535.
Chez, M., Hing, P., Chin, K., Memon, S., & Kirschner, S. (2004). Memantine experience in children and adolescents with autism spectrum disorders. Ann Neurol, 56(S8).
Cochran et al. (2015). Relationship among Glutamine, y-Aminobutyric Acid, and Social Cognition in Autism Spectrum Disorders. J Child Adolesc Psychopharmacol. 2015 May;25(4):314-22.
Cohen, J. (1988). Statistical Power Analysis for the Behavioral Sciences (Second Edition ed.). Hillsdale, NJ: Erlbaum.
Constantino, J. N., Davis, S. A., Todd, R. D., Schindler, M. K., Gross, M. M., Brophy, S. L., ... Reich, W. (2003). Validation of a brief quantitative measure of autistic traits: comparison of the social responsiveness scale with the autism diagnostic interview-revised. J Autism Dev Disord, 33(4), 427-433.
Constantino, J. N., & Todd, R. D. (2003). Autistic traits in the general population: a twin study. Arch Gen Psychiatry, 60(5), 524-530. doi:10.1001/archpsyc.60.5.524 60/5/524 [pii]
Cotman, C. W., & Anderson, A. J. (1995). A potential role for apoptosis in neurodegeneration and Alzheimer's disease. Mol Neurobiol, 10(1), 19-45. doi:10.1007/BF02740836
D'Souza, D. C., Charney, D., & Krystal, J. (1995). Glycine Site Agonists of the NMDA Receptor: A Review. CNS Drug Rev, 1(2), 227-260. doi:10.1111/j.1527-3458.1995.tb00285.x
Dhamala, E., Abdelkefi, I., Nguyen, M., Hennessy, T. J., Nadeau, H., & Near, J. (2019). Validation of in vivo MRS measures of metabolite concentrations in the human brain. NMR Biomed, 32(3), e4058. doi:10.1002/nbm.4058
Di Martino, A., Ross, K., Uddin, L. Q., Sklar, A. B., Castellanos, F. X., & Milham, M. P. (2009). Functional brain correlates of social and nonsocial processes in autism spectrum disorders: an activation likelihood estimation meta-analysis. Biol Psychiatry, 65(1), 63-74. doi:S0006-3223(08)01157-8 [pii]10.1016/j.biopsych.2008.09.022
Di Martino, A., Scheres, A., Margulies, D. S., Kelly, A. M., Uddin, L. Q., Shehzad, Z., . .. Milham, M. P. (2008). Functional connectivity of human striatum: a resting state FMRI study. Cereb Cortex, 18(12), 2735-2747. doi:10.1093/cercor/bhn041
Ding, X. Q., Maudsley, A. A., Sabati, M., Sheriff, S., Dellani, P. R., & Lanfermann, H. (2015). Reproducibility and reliability of short-TE whole-brain MR spectroscopic imaging of human brain at 3T. Magn Reson Med, 73(3), 921-928. doi:10.1002/mrm.25208
DuPaul, G. J., Power, T. J., Anastopoulos, A., & Reed, R. (1998). The ADHD Rating Scale-IV Checklist, Norms and Clinical Interpretation. New York, NY: Guilford Press. Fatemi, S. H., Halt, A. R., Stary, J. M., Kanodia, R., Schulz, S. C., & Realmuto, G. R. (2002). Glutamic acid decarboxylase 65 and 67 kDa proteins are reduced in autistic parietal and cerebellar cortices. Biol Psychiatry, 52(8), 805-810.
Findling, R. L., McNamara, N. K., Stansbrey, R. J., Maxhimer, R., Periclou, A., Mann, A., & Graham, S. M. (2007). A pilot evaluation of the safety, tolerability, pharmacokinetics, and effectiveness of memantine in pediatric patients with attention-deficit/hyperactivity disorder combined type. J Child Adolesc Psychopharmacol, 17(1), 19-33. doi:10.1089/cap.2006.0044
Gadow, K. D., Sprafkin, J., Carlson, G. A., Schneider, J., Nolan, E. E., Mattison, R. E., & Rundberg-Rivera, V. (2002). A DSM-IV-referenced, adolescent self-report rating scale. J Am Acad Child Adolesc Psychiatry, 41(6), 671-679. doi:10.1097/00004583-200206000-00006
Gonenc, A., Govind, V., Sheriff, S., & Maudsley, A. A. (2010). Comparison of spectral fitting methods for overlapping J-coupled metabolite resonances. Magn Reson Med, 64(3), 623-628. doi:10.1002/mrm.22540
Goodman, W. K., Rasmussen, S. A., Price, L. H., Mazure, C., Heninger, G. R., & Charney, D. S. (1986 (Rev 89)). Yale-Brown obsessive compulsive scale (Y-BOCS). Gottschalk, M., Tropres, I., Lamalle, L., Grand, S., Le Bas, J. F., & Segebarth, C. (2016). Refined modelling of the short-T2 signal component and ensuing detection of glutamate and glutamine in short-TE, localised, (1) H MR spectra of human glioma measured at 3 T. NMR Biomed, 29(7), 943-951. doi:10.1002/nbm.3548
Govindaraju, V., Young, K., & Maudsley, A. A. (2000). Proton NMR chemical shifts and coupling constants for brain metabolites. NMR Biomed, 13(3), 129-153. doi:10.1002/1099-1492(200005)13:3<129::aid-nbm619>3.0.co;2-v
Hardan, A. Y., Fung, L. K., Libove, R. A., Obukhanych, T. V., Nair, S., Herzenberg, L. A., ... Tirouvanziam, R. (2012). A randomized controlled pilot trial of oral N-acetylcysteine in children with autism. Biol Psychiatry, 71(11), 956-961. doi:10.1016/j.biopsych.2012.01.014
Haznedar, M. M., Buchsbaum, M. S., Metyger, M., Solimando, A., Spiegel-Cohen, J., & Hollander, E. (1997). Anterior cingulate gyrus volume and glucose metabolism in autistic disorder. American journal psychiatry(August), 154:158.
Henry, M. E., Lauriat, T. L., Shanahan, M., Renshaw, P. F., & Jensen, J. E. (2011). Accuracy and stability of measuring GABA, glutamate, and glutamine by proton magnetic resonance spectroscopy: a phantom study at 4 Tesla. J Magn Reson, 208(2), 210-218. doi:10.1016/j.jmr.2010.11.003
Hollander, E., Anagnostou, E., Chaplin, W., Esposito, K., Haznedar, M. M., Licalzi, E., . .. Buchsbaum, M. (2005). Striatal volume on magnetic resonance imaging and repetitive behaviors in autism. Biol Psychiatry, 58(3), 226-232.
Hollander, E., Soorya, L., Chaplin, W., Anagnostou, E., Taylor, B. P., Ferretti, C. J., . . . Settipani, C. (2012). A double-blind placebo-controlled trial of fluoxetine for repetitive behaviors and global severity in adult autism spectrum disorders. Am J Psychiatry, 169(3), 292-299. doi:10.1176/appi.ajp.2011.10050764
Ito et al., 2017. A A Proton Magnetic Resonance Spectroscopic Study in Autism Spectrum Disorder Using a 3-Tesla Clinical Magnetic Resonance Imaging (MRI) System: The Anterior Cingulate Cortex and the Left Cerebellum. J Child Neurol. 2017 Jul;32(8):731-739
Jamain, S., Betancur, C., Quach, H., Philippe, A., Fellous, M., Giros, B., . . . Bourgeron, T. (2002). Linkage and association of the glutamate receptor 6 gene with autism. Mol Psychiatry, 7(3), 302-310.
Jensen, J. E., Licata, S. C., Ongur, D., Friedman, S. D., Prescot, A. P., Henry, M. E., & Renshaw, P. F. (2009). Quantification of J-resolved proton spectra in two-dimensions with LCModel using GAMMA-simulated basis sets at 4 Tesla. NMR Biomed, 22(7), 762-769. doi:10.1002/nbm.1390
Joshi, G., Biederman, J., Wozniak, J., Goldin, R. L., Crowley, D., Furtak, S., . . . Gonenc, A. (2013). Magnetic resonance spectroscopy study of the glutamatergic system in adolescent males with high-functioning autistic disorder: a pilot study at 4T. Eur Arch Psychiatry Clin Neurosci, 263(5), 379-384. doi:10.1007/s00406-012-0369-9
Joshi, G., Wozniak, J., Faraone, S. V., Fried, R., Chan, J., Furtak, S., . . . Biederman, J. (2016). A Prospective Open-Label Trial of Memantine Hydrochloride for the Treatment of Social Deficits in Intellectually Capable Adults With Autism Spectrum Disorder. J Clin Psychopharmacol, 36(3), 262-271. doi:10.1097/JCP.0000000000000499
Kemper, T. L., & Bauman, M. (1998). Neuropathology of infantile autism. J Neuropathol Exp Neurol, 57(7), 645-652.
King, B. H., Wright, D. M., Handen, B. L., Sikich, L., Zimmerman, A. W., McMahon, W., . . . Cook, E. H., Jr. (2001). Double-blind, placebo-controlled study of amantadine hydrochloride in the treatment of children with autistic disorder. J Am Acad Child Adolesc Psychiatry, 40(6), 658-665. doi:10.1097/00004583-200106000-00010 Lappalainen, R., & Riikonen, R. S. (1996). High levels of cerebrospinal fluid glutamate in Rett syndrome. Pediatr Neurol, 15(3), 213-216. doi:S0887899496002184 [pii]
Liu, X. (2012). Classification accuracy and cut point selection. Stat Med, 31(23), 2676-2686. doi:10.1002/sim.4509
McCracken, J. T., McGough, J., Shah, B., Cronin, P., Hong, D., Aman, M. G., . . . McMahon, D. (2002). Risperidone in children with autism and serious behavioral problems. N Engl J Med, 347(5), 314-321.
McDougle, C., Holmes, J., Carlson, D., Pelton, G., Cohen, D., & Price, L. (1998). A double-blind placebo-controlled study of risperidone in adults with austistic disorder and other pervasive development disorders. Archives of General Psychiatry, 55, 633-641.
McDougle, C., Naylor, S., Cohen, D., Volkmar, F., Heninger, G., & Price, L. (1996). A Double-blind, Placebo-Controlled Study of Fluvoxamine in Adults with Autistic Disorder. Archives of General Psychiatry, 53, 1001-1008.
McDougle, C. J. (2002). Current and emerging therapeutics of autistic disorder and related pervasive developmental disorders. In K. L. Davis, D. Charney, J. T. Coyle, & C. Nemeroff (Eds.), Neuropsychopharmacology: The Fifth Generation of Progress (pp. 565-576). Philadelphia, PA: Lippincott Williams & Wilkins.
Minshawi et al., 2016. A randomized, placebo-controlled trial of D-cycloserine for the enhancement of social skills training in autism spectrum disorders. Mol Autism. 2016 Jan 14;7:2.
Mohammadi, et al. (2013) Double-blind, placebo-controlled trial of risperidone plus amantadine in children with autism: a 10-week randomized study. Clin Neuropharmacol. Nov-Dec 2013;36(6):179-84
Montag et al. (2008). Prefrontal Cortex Glutamate Correlates with Mental Perspective-Taking. PLoS One. 2008;3(12):e3890
Moore, C. M., Frazier, J. A., Glod, C. A., Breeze, J. L., Dieterich, M., Finn, C. T., . . . Renshaw, P. F. (2007). Glutamine and glutamate activity levels in children and adolescents with bipolar disorder: a 4.0-T proton magnetic resonance spectroscopy study of the anterior cingulate cortex. J Am Acad Child Adolesc Psychiatry, 46(4), 524-534. doi:10.1097/chi.0b013e31802f5f2c00004583-200704000-00015 [pii]
Ohnishi, T., Matsuda, H., Hashimoto, T., Kunihiro, T., Nishikawa, M., Uema, T., & Sasaki, M. (2000). Abnormal regional cerebral blood flow in childhood autism. Brain, 123 ( Pt 9), 1838-1844.
Ongur, D., Jensen, J. E., Prescot, A. P., Stork, C., Lundy, M., Cohen, B. M., & Renshaw, P. F. (2008). Abnormal glutamatergic neurotransmission and neuronal-glial interactions in acute mania. Biol Psychiatry, 64(8), 718-726. doi:10.1016/j.biopsych.2008.05.014
Owley, T., Salt, J., Guter, S., Grieve, A., Walton, L., Ayuyao, N., . . . Cook, E. H. (2006). A Prospective, Open-Label Trial of Memantine in the Treatment of Cognitive, Behavioral, and Memory Dysfunction in Pervasive Developmental Disorders. J Child Adolesc Psychopharmacol, 16(5), 517-524.
Posey, D. J., Kem, D. L., Swiezy, N. B., Sweeten, T. L., Wiegand, R. E., & McDougle, C. J. (2004). A pilot study of D-cycloserine in subjects with autistic disorder. Am J Psychiatry, 161(11), 2115-2117. doi:10.1176/appi.ajp.161.11.2115
Poznanski, E., Mokros, H. B., Grossman, J., & Freeman, L. N. (1985). Diagnostic criteria in childhood depression. Am J Psychiatry, 142(10), 1168-1173. doi:10.1176/ajp.142.10.1168
Prescot, A. P., & Renshaw, P. F. (2013). Two-dimensional J-resolved proton MR spectroscopy and prior knowledge fitting (ProFit) in the frontal and parietal lobes of healthy volunteers: assessment of metabolite discrimination and general reproducibility. J Magn Reson Imaging, 37(3), 642-651. doi:10.1002/jmri.23848
Purcell, S., & Sham, P. (2002). Variance components models for gene-environment interaction in quantitative trait locus linkage analysis. Twin Res, 5(6), 572-576.
Ramoz, N., Reichert, J. G., Smith, C. J., Silverman, J. M., Bespalova, I. N., Davis, K. L., & Buxbaum, J. D. (2004). Linkage and Association of the Mitochondrial Aspartate/Glutamate Carrier SLC25A12 Gene With Autism. Am J Psychiatry, 161(4), 662-669.
Research Units on Pediatric Psychopharmacology Autism Network. (2005). Randomized, controlled, crossover trial of methylphenidate in pervasive developmental disorders with hyperactivity. Arch Gen Psychiatry, 62(11), 1266-1274. doi:62/11/1266 [pii]10. 1001/archpsyc.62.11.1266
Scahill, L., McDougle, C. J., Williams, S. K., Dimitropoulos, A., Aman, M. G., McCracken, J. T., . . . Research Units on Pediatric Psychopharmacology Autism, N. (2006). Children's Yale-Brown Obsessive Compulsive Scale modified for pervasive developmental disorders. J Am Acad Child Adolesc Psychiatry, 45(9), 1114-1123. doi:10.1097/01.chi.0000220854.79144.e7
Serajee, F. J., Zhong, H., Nabi, R., & Huq, A. H. (2003). The metabotropic glutamate receptor 8 gene at 7q31: partial duplication and possible association with autism. J Med Genet, 40(4), e42.
Shimmura, C., Suda, S., Tsuchiya, K. J., Hashimoto, K., Ohno, K., Matsuzaki, H., . . . Mori, N. (2011). Alteration of plasma glutamate and glutamine levels in children with high-functioning autism. PLoS One, 6(10), e25340. doi:10.1371/journal.pone.0025340 Shuang, M., Liu, J., Jia, M. X., Yang, J. Z., Wu, S. P., Gong, X. H., . . . Zhang, D. (2004). Family-based association study between autism and glutamate receptor 6 gene in Chinese Han trios. Am J Med Genet B Neuropsychiatr Genet, 131(1), 48-50.
Silk, T. J., Rinehart, N., Bradshaw, J. L., Tonge, B., Egan, G., O'Boyle M, W., & Cunnington, R. (2006). Visuospatial processing and the function of prefrontal-parietal networks in autism spectrum disorders: a functional MRI study. Am J Psychiatry, 163(8), 1440-1443.
Simms, M. L., Kemper, T. L., Timbie, C. M., Bauman, M. L., & Blatt, G. J. (2009). The anterior cingulate cortex in autism: heterogeneity of qualitative and quantitative cytoarchitectonic features suggests possible subgroups. Acta Neuropathol, 118(5), 673-684. doi:10.1007/s00401-009-0568-2
StataCorp. (2019). Stata Statistical Software: Release 16. College Station, TX: StatCorp LLC.
Urbano, M., Okwara, L., Manser, P., Hartmann, K., & Deutsch, S. I. (2015). A trial of d-cycloserine to treat the social deficit in older adolescents and young adults with autism spectrum disorders. J Neuropsychiatry Clin Neurosci, 27(2), 133-138. doi:10.1176/appi.neuropsych.13070155
Urbano, M., Okwara, L., Manser, P., Hartmann, K., Herndon, A., & Deutsch, S. I. (2014). A trial of D-cycloserine to treat stereotypies in older adolescents and young adults with autism spectrum disorder. Clin Neuropharmacol, 37(3), 69-72. doi: 10. 1097 /WNF . 0000000000000033
van Elst, L. T., Maier, S., Fangmeier, T., Endres, D., Mueller, G. T., Nickel, K., . . . Perlov, E. (2014). Magnetic resonance spectroscopy comparing adults with high functioning autism and above average IQ. Mol Psychiatry, 19(12), 1251. doi:10.1038/mp.2014.160
Wechsler, D. (2003). Wechsler Intelligence Scale for Children-Fourth Edition (WISC-IV). San Antonio, TX: The Psychological Corporation.
Wechsler, D. (2008). Wechsler Adult Intelligence Scale - Fourth Edition (WAIS-IV) (4th Ed ed.). San Antonio, TX: NCS Pearson, Inc.
Wiebenga, O. T., Klauser, A. M., Nagtegaal, G. J., Schoonheim, M. M., Barkhof, F., Geurts, J. J., & Pouwels, P. J. (2014). Longitudinal absolute metabolite quantification of white and gray matter regions in healthy controls using proton MR spectroscopic imaging. NMR Biomed, 27(3), 304-311. doi:10.1002/nbm.3063
Willemsen-Swinkels, S., Buitelaar, J. K., & van Engeland, H. (1996). The effects of chronic naltrexone treatment in young autisitc children: A double-blind plecebo-controlled crossover study. Society of Biological Psychiatry, 39, 1023-1031.
Williams, K., Wray, J. A., & Wheeler, D. M. (2012). Intravenous secretin for autism spectrum disorders (ASD). Cochrane Database Syst Rev, 4, CD003495. doi:10.1002/14651858.CD003495.pub3
Wink, L. K., Adams, R., Wang, Z., Klaunig, J. E., Plawecki, M. H., Posey, D. J., . . . Erickson, C. A. (2016). A randomized placebo-controlled pilot study of N-acetylcysteine in youth with autism spectrum disorder. Mol Autism, 7, 26. doi:10.1186/s13229-016-0088-6
Wozniak, J., Gonenc, A., Biederman, J., Moore, C., Joshi, G., Georgiopoulos, A., . . . Henin, A. (2012). A Magnetic Resonance Spectroscopy Study of the Anterior Cingulate Cortex In Youth with Emotional Dysregulation. Isr J Psychiatry Relat Sci, 49(1), 62-69. Research Units on Pediatric Psychopharmacology Autism Network: Risperidone in children with autism and serious behavioral problems. N Engl J Med. 2002;347: 314-321. PMID: 12151468.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.
The invention will now be defined by reference to the following clauses
1. A method of predicting response to treatment with a glutamate modulating (lowering) agent in a subject with autism spectrum disorder (ASD), the method comprising:
   (i) identifying a subject who has ASD;
   (ii) determining a level of glutamate in one or more selected brain regions of interest (ROIs) in the brain of the subject;
   (iii) comparing the level of glutamate in the ROI to a reference level; and
   (iv) on the basis of step (iii), identifying a subject who has a level of glutamate in the ROI above the reference level as likely to respond to treatment with a glutamate modulating agent.
2. A method of selecting a treatment comprising administration of a glutamate modulating (lowering) agent for a subject with autism spectrum disorder (ASD), the method comprising:
   (i) identifying a subject who has ASD;
   (ii) determining a level of glutamate in one or more selected brain regions of interest (ROIs) in the brain of the subject;
   (iii) comparing the level of glutamate in the ROI to a reference level; and
   (iv) on the basis of step (iii), selecting a treatment with a glutamate modulating agent for a subject who has a level of glutamate in the ROI above the reference level.
3. A method of treating a subject with autism spectrum disorder (ASD), the method comprising:
   (i) identifying a subject who has ASD;
   (ii) determining a level of glutamate in one or more selected brain regions of interest (ROIs) in the brain of the subject;
   (iii) comparing the level of glutamate in the ROI to a reference level; and
   (iv) on the basis of step (iii), administering a treatment comprising a therapeutically effective amount of a glutamate modulating agent to a subject who has a level of glutamate in the ROI above the reference level.
4. A method of treating a subject with autism spectrum disorder (ASD), the method comprising:
   (i) providing a subject identified as having ASD characterized by increased glutamate activity in one or more selected brain regions of interest (ROIs) in the brain of the subject; and
   (ii) administering to the subject a therapeutically effective amount of a glutamate modulating agent.
5. The method of clauses 1-4, wherein at least one ROI comprises all or part of the pregenual anterior cingulate cortex (PgACC).
6. The methods of clauses 1-4, wherein determining a level of glutamate in one or more selected ROIs comprises using magnetic resonance imaging (MRI), positron emission tomography (PET), or magnetic resonance spectroscopy (MRS), preferably proton Magnetic Resonance Spectroscopy (¹H MRS).
7. The method of clause 6, wherein determining a level of glutamate in the ROI comprises acquiring and/or analyzing a proton spectra using ¹H MRS.
8. The method of clause 7, wherein the proton spectra is acquired at 3 or 4 Tesla (3T or 4T) using a two-dimensional J-resolved (2D-JPRESS) ¹H MRS.
9. The method of clause 6, wherein determining a level of glutamate in the ROI comprises using a glutamate chemical exchange saturation transfer (GluCEST) imaging.
10. The method of clauses 1-9, wherein the glutamate modulating agent is memantine, a nitro-aminoadamantane compound, lamotrigine, amantadine, D-cycloserine, N-Acetylcysteine, an amino-adamantane derivative, or a pharmaceutically acceptable salt thereof.
11. The method of clauses 1-3, wherein the reference level represents a level in a cohort of subjects who have a level of glutamate in the ROI that is above the level of glutamate in the ROI of a cohort of healthy control subjects.
12. The method of clause 10, wherein the reference level represents a level of glutamate that is at least one standard deviation above the level of glutamate in the cohort of healthy subjects.
13. The method of any one of clauses 1-12, wherein the treatment comprises administration of the glutamate modulating agent at least once a day for at least a week, a month, three months, six months, or a year.
14. The method of any one of clauses 1-13, wherein the therapeutically effective amount is sufficient to result in an improvement in one or more impairments measured in the Social Responsiveness Scale-Second Edition (SRS-2) and/or a the Clinical Global Impression (CGI) severity scale.
15. The method of any one of clauses 1-14, wherein the subject has high-functioning ASD (HF-ASD) and/or is intellectually capable.
16. A method of monitoring response to treatment with a glutamate modulating (lowering) agent in a subject with autism spectrum disorder (ASD), the method comprising:
   (i) identifying a subject who has ASD;
   (ii) determining a baseline level of glutamate in one or more selected brain regions of interest (ROIs) in the brain of the subject;
   (iii) administering two or more doses of the glutamate modulating agent to the subject;
   (iv) determining a subsequent level of glutamate in the ROI;
   (v) comparing the baseline level of glutamate in the ROI to the subsequent level; and identifying a subject who has a subsequent level of glutamate in the ROI below the baseline level as responding to treatment with a glutamate modulating agent.
17. The method of clause 16, wherein at least one ROI comprises all or part of the pregenual anterior cingulate cortex (PgACC).
18. The methods of clause 16, wherein determining a level of glutamate in one or more selected ROIs comprises using magnetic resonance imaging (MRI), positron emission tomography (PET), or magnetic resonance spectroscopy (MRS), preferably proton Magnetic Resonance Spectroscopy (¹H MRS).
19. The method of clause 18, wherein determining a level of glutamate in the ROI comprises acquiring and/or analyzing a proton spectra using ¹H MRS.
20. The method of clause 19, wherein the proton spectra is acquired at 3 or 4 Tesla (3T or 4T) using a two-dimensional J-resolved (2D-JPRESS) ¹H MRS.
21. The method of clause 18, wherein determining a level of glutamate in the ROI comprises using a glutamate chemical exchange saturation transfer (GluCEST) imaging.
22. The method of clauses 15-20, wherein the glutamate modulating agent is memantine, a nitro-aminoadamantane compound, lamotrigine, amantadine, D-cycloserine, N-Acetylcysteine, or a pharmaceutically acceptable salt thereof.
23. The method of any one of clauses 1-22, wherein the subject is younger than 21 years old, 18 years old, or 15 years old.
24. The method of any one of clauses 1-23, wherein the glutamate modulating agent is memantine, or a pharmaceutically acceptable salt thereof.
25. The method of any one of clauses 1-23, wherein the glutamate modulating agent is a nitro-aminoadamantane compound, or a pharmaceutically acceptable salt thereof.
26. The method of clause 25, wherein the nitro-aminoadamantane compound is a compound of any one of formulas (I)-(V).
27. The method of clause 25, wherein the nitro-aminoadamantane compound is selected from: 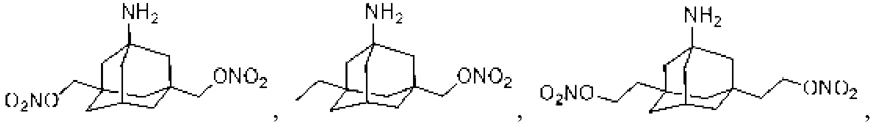 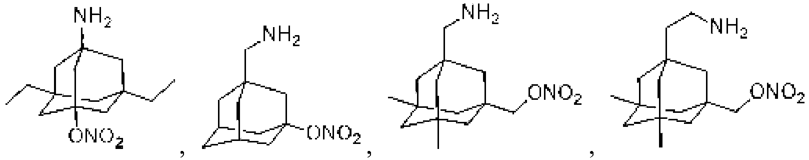 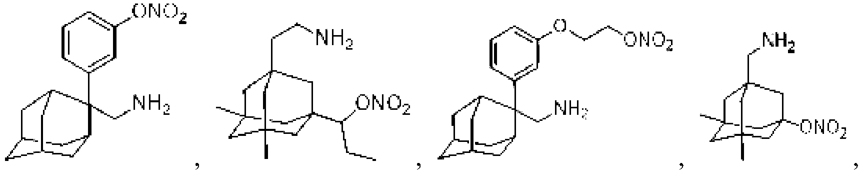 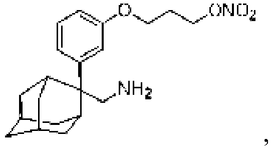 and pharmaceutically acceptable salts thereof.

## Claims

1. A glutamate modulating agent comprising nitro-aminoadamantane compound, memantine, lamotrigine, amantadine, D- cycloserine, or N-Acetylcysteine, an amino-adamantane derivative or a pharmaceutically acceptable salt thereof for use in a method of treating a subject with autism spectrum disorder (ASD), the method comprising:
a)
(i) identifying a subject who has ASD;
(ii) determining a level of glutamate in one or more selected brain regions of interest (ROIs) in the brain of the subject via non-invasive imaging methods;
(iii) comparing the level of glutamate in the ROI to a reference level; and
(iv) on the basis of step (iii), administering a treatment comprising a therapeutically effective amount of said glutamate modulating agent to a subject who has a level of glutamate in the ROI above the reference level; or
b) (i) providing a subject identified as having ASD **characterized by** increased glutamate activity in one or more selected brain regions of interest (ROIs) in the brain of the subject; and (ii) administering to the subject a therapeutically effective amount of a glutamate modulating agent to a subject who has a level of glutamate in the ROI above the reference level.

2. The glutamate modulating agent for the use of claim 1, wherein at least one ROI comprises all or part of the pregenual anterior cingulate cortex (PgACC).

3. The glutamate modulating agent for the use of claims 1 or 2, wherein determining a level of glutamate in one or more selected ROIs comprises using magnetic resonance imaging (MRI), positron emission tomography (PET), or magnetic resonance spectroscopy (MRS), preferably proton Magnetic Resonance Spectroscopy (¹H MRS); optionally, wherein determining a level of glutamate in the ROI comprises acquiring and/or analyzing a proton spectra using ¹H MRS, optionally, wherein the proton spectra is acquired at 3 or 4 Tesla (3T or 4T) using a two-dimensional J-resolved (2D-JPRESS) ¹H MRS; or, wherein determining a level of glutamate in the ROI comprises using a glutamate chemical exchange saturation transfer (GluCEST) imaging.

4. The glutamate modulating agent for the use of claims 1-3, wherein the reference level represents a level in a cohort of subjects who have a level of glutamate in the ROI that is above the level of glutamate in the ROI of a cohort of healthy control subjects, optionally, wherein the reference level represents a level of glutamate that is at least one standard deviation above the level of glutamate in the cohort of healthy subjects.

5. The glutamate modulating agent for the use of claims 1-4, wherein the treatment comprises administration of the glutamate modulating agent at least once a day for at least a week, a month, three months, six months, or a year.

6. The glutamate modulating agent for the use of claims 1-5, wherein the therapeutically effective amount is sufficient to result in an improvement in one or more impairments measured in the Social Responsiveness Scale-Second Edition (SRS-2) and/or a the Clinical Global Impression (CGI) severity scale.

7. The glutamate modulating agent for the use of claims 1-6, wherein the subject has high-functioning ASD (HF-ASD) and/or is intellectually capable.

8. The glutamate modulating agent for the use of claims 1-7, wherein the subject is younger than 21 years old, 18 years old, or 15 years old.

9. The glutamate modulating agent for the use of claims 1-8, wherein the nitro-aminoadamantane compound is a compound of any one of formulas (I)-(V), or, wherein the nitro-aminoadamantane compound is selected from: 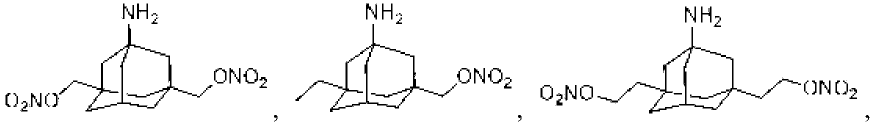 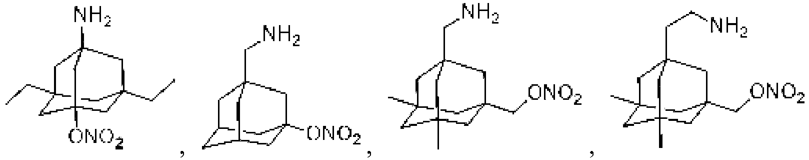 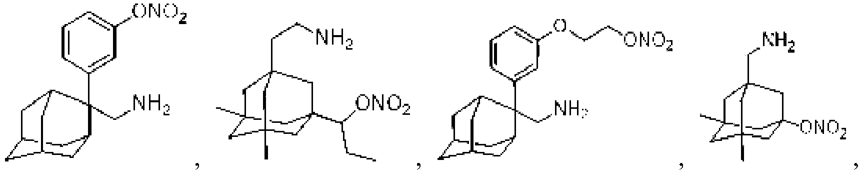 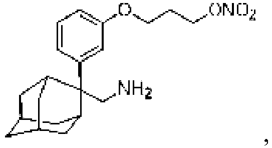 and pharmaceutically acceptable salts thereof.
